# EUROPEAN PATENT SPECIFICATION

(11) **EP 3 103 871 B1**
(45) Date of publication and mention of the grant of the patent: **29.07.2020**
(21) Application number: 16173137.7
(22) Date of filing: 16.09.2009
(51) Int. Cl.: C12Q 1/6804, C12Q 1/6806, C12Q 1/6809, C12Q 1/6888

(54) **PROCESSES FOR METHYLATION-BASED ENRICHMENT OF FETAL NUCLEIC ACID FROM A MATERNAL SAMPLE USEFUL FOR FETAL NUCLEIC ACID QUANTIFICATION**
VERFAHREN ZUR METHYLIERUNGSBASIERTEN ANREICHERUNG FETALER NUKLEINSÄURE AUS EINER MÜTTERLICHEN PROBE FÜR FETALE NUKLEINSÄURE QUANTIFIZIERUNG
PROCÉDÉS POUR L'ENRICHISSIMENT EN ACIDE NUCLEIC FÉTAL D'UN ÉCHANTILLON MATERNEL, BASÉ SUR LA MÉTHYLATION ET UTILISATION POUR LA QUANTIFICATION DE L'ACIDE NUCLEIQUE FÉTAL

(30) Priority: 16.09.2008 US 192264 P
(43) Date of publication of application: 14.12.2016
(62) Divisional of application: 09815148.3
(73) Proprietor: Sequenom, Inc., San Diego, CA 92121 (US); Sequenom Center For Molecular Medicine, Grand Rapids, MI 49503 (US)
(72) Inventor: EHRICH, Mathias, San Diego, CA 92109 (US); NYGREN, Anders, Olof, Herman, San Diego, CA 92122 (US)
(74) Representative: Vossius & Partner Patentanwälte Rechtsanwälte mbB

(56) References cited:
- WO-A1-2004/078999
- WO-A2-2006/056480
- WO-A2-2007/132166
- OLD ROBERT W ET AL: "Candidate epigenetic biomarkers for non-invasive prenatal diagnosis of Down syndrome", REPRODUCTIVE BIOMEDICINE ONLINE, REPRODUCTIVE HEALTHCARE LTD, GB, vol. 15, no. 2, 1 January 2007 (2007-01-01), pages 227-235, XP008144463, ISSN: 1472-6483

### SEQUENCE LISTING

<110> SEQUENOM, INC.
   SEQUENOM CENTER FOR MOLECULAR MEDICINE
<120> PROCESSES AND COMPOSITIONS FOR METHYLATION-BASED ENRICHMENT OF FETAL NUCLEIC ACID FROM A MATERNAL SAMPLE USEFUL FOR NON-INVASIVE PRENATAL DIAGNOSES
<130> T1450 EP/1
<150> US 61/192,264
   <151> 2008-09-16
<160> 179
<170> PatentIn version 3.5
<210> 1
   <211> 305
   <212> DNA
   <213> Homo sapiens
<400> 1
   cagcaggcgc gctcccggcg aatctgcctg aatcgccgtg aatgcggtgg ggtgcagggc      60
   aggggctggt tttctcagcc ggtcttggct tttctctttc tctcctgctc caccagcagc     120
   ccctccgcgg gtcccatggg ctccgcgctc agaacagccc ggaaccaggc gccgctcgcc     180
   gctcgctggg ggccacccgc ctctccccgg aacagcctcc cgcgggcctc ttggcctcgc     240
   actggcgccc tcacccacac atcgtccctt tatccgctca gacgctgcaa agggccttct     300
   gtctc                                                                 305
<210> 2
   <211> 336
   <212> DNA
   <213> Homo sapiens
<400> 2
   gctttggatt tatcctcatt ggctaaatcc ctcctgaaac atgaaactga aacaaagccc      60
   tgaaccccct caggctgaaa agacaaaccc cgcctgaggc cgggtcccgc tccccacctg     120
   gagggaccca attctgggcg ccttctggcg acggtccctg ctagggacgc tgcgctctcc     180
   gagtgcgagt tttcgccaaa ctgataaagc acgcagaacc gcaatcccca aactaacact     240
   gaacccggac ccgcgatccc caaactgaca agggacccgg aacagcgacc cccaaaccga     300
   cacgggactc gggaaccgct atctccaaag ggcagc                               336
<210> 3
   <211> 139
   <212> DNA
   <213> Homo sapiens
<400> 3
   tttccacaac agggagccag cattgaggcg cccagatggc atctgctgga aatcacgggc      60
   cgctggtgaa gcaccacgcc ttacccgacg tggggaggtg atcccccacc tcatcccacc     120
   cccttctgtc tgtctcctt                                                  139
<210> 4
   <211> 292
   <212> DNA
   <213> Homo sapiens
<400> 4
   gctggacaag gagcgctcac tgtagctctg ctgtggattg tgttggggcg aagagatggg      60
   taagaggtca aagtcgtagg attctggcga ccgcctacca agggattggg tccacagcac     120
   agaggtctga tcgcttcctt ctctgctctg ccacctccag acagcagctc taaccagctg     180
   cccagcagca agaggatgcg cacggctttc accagcacgc agctgctaga gctggagcgc     240
   gagttcgctt ctaatatgta cctgtcccgc ctacgtcgca tcgagatcgc ga             292
<210> 5
   <211> 190
   <212> DNA
   <213> Homo sapiens
<400> 5
   tgcctgacac tgaccccagg cgcagccagg aggggctttg tgcgggagag ggagggggac 60
   cccagcttgc ctggggtcca cgggactctc ttcttcctag ttcactttct tgctaaggcg 120
   aaggtcctga ggcaggacga gggctgaact gcgctgcaat cgtccccacc tccagcgaaa 180
   cccagttgac 190
<210> 6
   <211> 706
   <212> DNA
   <213> Homo sapiens
<400> 6
   tcggcggaga gacctcgagg agagtatggg gaaaggaatg aatgctgcgg agcgcccctc 60
   tgggctccac ccaagcctcg gaggcgggac ggtgggctcc gtcccgaccc cttaggcagc 120
   tggaccgata cctcctggat cagaccccac aggaagactc gcgtggggcc cgatatgtgt 180
   acttcaaact ctgagcggcc accctcagcc aactggccag tggatgcgaa tcgtgggccc 240
   tgaggggcga gggcgctcgg aactgcatgc ctgtgcacgg tgccgggctc tccagagtga 300
   gggggccgta aggagatctc caaggaagcc gaaaaaagca gccagttggg cttcgggaaa 360
   gacttttctg caaaggaagt gatctggtcc cagaactcca gggttgaccc cagtacctga 420
   cttctccggg agctgtcagc tctcctctgt tcttcgggct tggcgcgctc ctttcataat 480
   ggacagacac cagtggcctt caaaaggtct ggggtggggg aacggaggaa gtggccttgg 540
   gtgcagagga agagcagagc tcctgccaaa gctgaacgca gttagcccta cccaagtgcg 600
   cgctggctcg gcatatgcgc tccagagccg gcaggacagc ccggccctgc tcaccccgag 660
   gagaaatcca acagcgcagc ctcctgcacc tccttgcccc agagac 706
<210> 7
   <211> 325
   <212> DNA
   <213> Homo sapiens
<400> 7
   agatcccggt gcatttaaag gccggcgtga tctgcaccac gtacctatct cggattctca 60
   gtttcacttc gctggtgtct gccaccatct ttaccacatc ccggtagcta catttgtcta 120
   ccgcttgagc caccagcgtc tgaaacctgg accggatttt gcgcgccgag aggtagccgg 180
   aggcggtaat gaattccacc cagagggaca tgctcctctt gcgcccgtcg ctcaacttca 240
   gcaccgcgca gccgggcagt gagccatcgt ccacgaagtt gaacaccccc atttggttga 300
   gataaagcac cacttcaaat tcggt 325
<210> 8
   <211> 663
   <212> DNA
   <213> Homo sapiens
<400> 8
   actatgcctt gagggtcaaa acgtctggat ttcctgatcg atgctgtcgt cgctgtccac      60
   ggagctactg tcgccgtcag agcgggaagg cacgttcagg gagtagaagc gtgggcttgc     120
   agaaagggac ctgttgctgc cttacatggg ggccggcagg gtagtcttgg aaatgcccaa     180
   gattgcttcc gcgcgcgtca gttcagcgga cgtgtctgcc tggcacgagg accgttctac     240
   aaactcgttc ctggaagccg ggctcgctgg aggcggagct ttggtttcct tcgggagctt     300
   gtggggaatg gtcagcgtct aggcaccccg ggcaagggtc tgtggccttg gtggccactg     360
   gcttcctcta gctgggtgtt ttcctgtggg tctcgcgcaa ggcacttttt tgtggcgctg     420
   cttgtgctgt gtgcggggtc aggcgtcctc tctcctcccg gcgctgggcc ctctggggca     480
   ggtccccgtt ggcctccttg cgtgtttgcc gcagctagta cacctggatg gcctcctcag     540
   tgccgtcgtt gctgctggag tctgacgcct cgggcgcctg cgccgcactt gtgacttgct     600
   ttccccttct cagggcgcca gcgctcctct tgaccccgct tttattctgt ggtgcttctg     660
   aag                                                                   663
<210> 9
   <211> 1985
   <212> DNA
   <213> Homo sapiens
<400> 9
   gcaagtcggg tagctaccgg gtgctggaga actccgcacc gcacctgctg gacgtggacg      60
   cagacagcgg gctcctctac accaagcagc gcatcgaccg cgagtccctg tgccgccaca 120
   atgccaagtg ccagctgtcc ctcgaggtgt tcgccaacga caaggagatc tgcatgatca 180
   aggtagagat ccaggacatc aacgacaacg cgccctcctt ctcctcggac cagatcgaaa 240
   tggacatctc ggagaacgct gctccgggca cccgcttccc cctcaccagc gcacatgacc 300
   ccgacgccgg cgagaatggg ctccgcacct acctgctcac gcgcgacgat cacggcctct 360
   ttggactgga cgttaagtcc cgcggcgacg gcaccaagtt cccagaactg gtcatccaga 420
   aggctctgga ccgcgagcaa cagaatcacc atacgctcgt gctgactgcc ctggacggtg 480
   gcgagcctcc acgttccgcc accgtacaga tcaacgtgaa ggtgattgac tccaacgaca 540
   acagcccggt cttcgaggcg ccatcctact tggtggaact gcccgagaac gctccgctgg 600
   gtacagtggt catcgatctg aacgccaccg acgccgatga aggtcccaat ggtgaagtgc 660
   tctactcttt cagcagctac gtgcctgacc gcgtgcggga gctcttctcc atcgacccca 720
   agaccggcct aatccgtgtg aagggcaatc tggactatga ggaaaacggg atgctggaga 780
   ttgacgtgca ggcccgagac ctggggccta accctatccc agcccactgc aaagtcacgg 840
   tcaagctcat cgaccgcaac gacaatgcgc cgtccatcgg tttcgtctcc gtgcgccagg 900
   gggcgctgag cgaggccgcc cctcccggca ccgtcatcgc cctggtgcgg gtcactgacc 960
   gggactctgg caagaacgga cagctgcagt gtcgggtcct aggcggagga gggacgggcg 1020
   gcggcggggg cctgggcggg cccgggggtt ccgtcccctt caagcttgag gagaactacg 1080
   acaacttcta cacggtggtg actgaccgcc cgctggaccg cgagacacaa gacgagtaca 1140
   acgtgaccat cgtggcgcgg gacgggggct ctcctcccct caactccacc aagtcgttcg 1200
   cgatcaagat tctagacgag aacgacaacc cgcctcggtt caccaaaggg ctctacgtgc 1260
   ttcaggtgca cgagaacaac atcccgggag agtacctggg ctctgtgctc gcccaggatc 1320
   ccgacctggg ccagaacggc accgtatcct actctatcct gccctcgcac atcggcgacg 1380
   tgtctatcta cacctatgtg tctgtgaatc ccacgaacgg ggccatctac gccctgcgct 1440
   cctttaactt cgagcagacc aaggcttttg agttcaaggt gcttgctaag gactcggggg 1500
   cgcccgcgca cttggagagc aacgccacgg tgagggtgac agtgctagac gtgaatgaca 1560
   acgcgccagt gatcgtgctc cccacgctgc agaacgacac cgcggagctg caggtgccgc 1620
   gcaacgctgg cctgggctat ctggtgagca ctgtgcgcgc cctagacagc gacttcggcg 1680
   agagcgggcg tctcacctac gagatcgtgg acggcaacga cgaccacctg tttgagatcg 1740
   acccgtccag cggcgagatc cgcacgctgc accctttctg ggaggacgtg acgcccgtgg 1800
   tggagctggt ggtgaaggtg accgaccacg gcaagcctac cctgtccgca gtggccaagc 1860
   tcatcatccg ctcggtgagc ggatcccttc ccgagggggt accacgggtg aatggcgagc 1920
   agcaccactg ggacatgtcg ctgccgctca tcgtgactct gagcactatc tccatcatcc 1980
   tccta 1985
<210> 10
   <211> 213
   <212> DNA
   <213> Homo sapiens
<400> 10
   atgcgccctc tgcaccccta gagccagaag acgctaggtg ggctgcgcgc tctgccaggc 60
   gaaggctgga gcgcagacgg caaagccgcg cgtttcagcc gtggtcgggt ccgcaggacc 120
   tgggcgtggg gacaccacca ggcaggagca gaggcaggac tgggacgcca aaagctgaga 180
   atcctcgatg cccgcgcgag agccccgtgt tat 213
<210> 11
   <211> 1558
   <212> DNA
   <213> Homo sapiens
<400> 11
   ttctggaaac cgggccccac ttgcaggccc ggccaccttg ggttctggtg gccgaagccg 60
   gagctgtgtt tctcgcagac tcggggagct acattgtgcg taggcaattg tttagtttga 120
   aaggaggcac atttcaccac gcagccagcg ccctgcatgc aggagaagcc cccagggccc 180
   agggtcggct ggctttagag gccacttagg ttgttttaag cacatgtgaa agggcagaca 240
   gcaggggagc aggatatggg taagatcttc gggtctcaga acaggggctg cccttgggct 300
   gtcccggcgc cctgggctct gacactgaag ggtggaatgg aggaaggaat ggagaaagga 360
   cggtggaact ttcgcttccc ctctgggccg ccttcccagg gtcatgcctg agctgctttg 420
   atcccagtgt cgcgcatctt ggtccgctac ctcccaggcg atagctactg ggctcctcgc 480
   tggcctcact gggggccatc ccgggcagtg gcctgccctc cgaggcccgc gggacccagc 540
   ccagagctga ggttggagtt ctccgggcca cgttccgggt cgcttaggct cggagatttc 600
   ccggagaccg tcgtcctccc tttctgcttg gcactgcgga gctccctcgg cctctctcct 660
   cctctggtcc ctaaggcccg gagtggttgg cggtactggg gcccgtcgtc atctctgctt 720
   ctaaggcatt cagactgggc tccagctggg accggcagag gaggttctca aggaaactgg 780
   tgggaaatat agttttcttt cgtctggtcg tttaatttaa atgcaacttc ccttggggac 840
   attttcctgg acgttaacca gaccaccttg agatgtcgtt gatgacctag agacccagat 900
   gatgcgtccc aggaaagttc actgctgact attgtcactc ttggcgttat atctatagat 960
   atagacctat gtacatatct ccaccctgat ctctccgtgg acatgaaacc cacctacctt 1020
   gtgaaagccc tacgggtgac acatgactac tacgtctctg tcccaacagg ggctgggcct 1080
   cccctgccta atagttgcca ggagtttcgc agcccaagtg aataatgtct tatggctgaa 1140
   cgtggccaag gactcctgtg atttaggtcc caggaggagc agagacgtcc ccgccccgcc 1200
   tgggccctgc cgcattcaaa gctggaagaa ggcgctgatc agagaagggg cttccaggtc 1260
   ctgggttaga acaacaacaa acaaacgaaa ctccacaaca gacacgcctg cccatgaccc 1320
   cacgcaagga cataggaagt tctgtcgcct tcctgctccg cggatagccg cctgccgtct 1380
   gctgccacca gaacgcacgg acgctcgggg tggaggtagt caatgggcag caggggaccc 1440
   ccagccccca caagcgcggc tccgaggacc tggaagcggg tgcctgtcgc tctccgcagg 1500
   ctccgctctg cctccaggag caagatcccc aaaagggtct ggaagctgtg gagaaaac 1558
<210> 12
   <211> 1264
   <212> DNA
   <213> Homo sapiens
<400> 12
   ttttttaaac acttcttttc cttctcttcc tcgttttgat tgcaccgttt ccatctgggg        60
   gctagaggag caaggcagca gccttcccag ccagcccttg ttggcttgcc atcgtccatc       120
   tggcttataa aagtttgctg agcgcagtcc agagggctgc gctgctcgtc ccctcggctg       180
   gcagaagggg gtgacgctgg gcagcggcga ggagcgcgcc gctgcctctg gcgggctttc       240
   ggcttgaggg gcaaggtgaa gagcgcaccg gccgtggggt ttaccgagct ggatttgtat       300
   gttgcaccat gccttcttgg atcggggctg tgattcttcc cctcttgggg ctgctgctct       360
   ccctccccgc cggggcggat gtgaaggctc ggagctgcgg agaggtccgc caggcgtacg       420
   gtgccaaggg attcagcctg gcggacatcc cctaccagga gatcgcaggt aagcgcgggc       480
   gcgctgcagg ggcaggctgc agccctcggc tgccgcacgt cccactggcc gcccggcgtc       540
   cccttccttc cccctgttgc tgagttggtg ctcactttct gccaccgcta tgggactccg       600
   cgtctccgtg ttgggcggcg gatgctcctg cggcttcttc ggcgggggaa ggtgtgcgtc       660
   tccgccgcct cattgtgtgc acacgcggga gcaccctggc tcccgcctcc cgctgctctc       720
   gcgcccttct accccttagt tgatggctca ggcccggctg gccagggagc ccgggtcact      780
   ccggggcggc tgcaaggcgc agacggagag ccgagccggg cgctcactcc gcgttctggt       840
   tcgggcaaac ttggaagaac tgcgaccgca gtttgcccag cgccacagtc tgagtggcgc       900
   cttctccact cccgcccttg cgccggcagg ggcggtggag agacgcggag ggctccccca       960
   gcccctctct cccctatccg tccttcgggc gacagagcgc ccggcgctcg ggccgggggc      1020
   gggcaaggct gggagggacc ctcgccgggg acctggcctc tggacgccgg cgtttcaagg      1080
   ctggtttggg gacttcacgg gctgcctgtt tcagatgtgg ggcgggcttt cccgttaggg      1140
   ttcctcagtg cttccccagt tgctgttggc cactcagggc ccggggacac cctgccaccc      1200
   ggtctggagc cggcctcgtc tgccagcgaa cagccaactt tagcgggtgg ctcagctggg      1260
   gatt                                                                   1264
<210> 13
   <211> 761
   <212> DNA
   <213> Homo sapiens
<400> 13
   cactcagtgt gtgcatatga gagcggagag acagcgacct ggaggccatg ggtgggggcg        60
   ggtggtgaag ctgccgaagc ctacacatac acttagcttt gacacttctc gtaggttcca       120
   aagacgaaga cacggtggct tcagggagac aagtcgcaag ggcgactttt ccaagcggga       180
   gatggtgaag tctttggacg tgtagtgggt aggtgatgat ccccgcagcc gcctgtaggc       240
   ccgcagactt cagaaaacaa gggccttctg tgagcgctgt gtcctccccg gaatccgcgg       300
   cttaacacat tctttccagc tgcggggcca ggatctccac cccgcgcatc cgtggacaca       360
   cttagggtcg cctttgtttt gcgcagtgat tcaagttggg taacccttgc tcaacacttg       420
   ggaaatgggg agaatctccc ccacccgcaa cctcccgcac cccaggttcc caaaatctga       480
   atctgtatcc tagagtggag gcagcgtcta gaaagcaaag aaacggtgtc caaagacccc       540
   ggagagttga gtgagcgcag atccgtgacg cctgcggtac gctagggcat ccaggctagg       600
   gtgtgtgtgt gcgggtcggg gggcgcacag agaccgcgct ggtttaggtg gacccgcagt       660
   cccgcccgca tctggaacga gctgcttcgc agttccggct cccggcgccc cagagaagtt       720
   cggggagcgg tgagcctagc cgccgcgcgc tcatgtttat t                           761
<210> 14
   <211> 1198
   <212> DNA
   <213> Homo sapiens
<400> 14
   agtcactcca ggatcagagg ccgcgtcggt tctgcttggg gcatgggcag agggaggctg        60
   ctggggccaa gccccggctg gacgcgaggg aagaaactcg tcccaggacc cgcacgccca       120
   tacctggctg tcccagagct cttccctagg ccggcacctt cgctcttcct cttccccacc       180
   ccctagccct tttgtctctt tttcagacgg atgttttcag tctcaagtgg ttttattttc       240
   cgcacaaaac cctgagatca agggcagatc acagactgta ccggaggctc gggtttccct       300
   ggactctgtg ctgttctgcg tcccagggtt ggctaggaag gaaggcctgg gccggcgagg       360
   tgacgggtct cccgcccagg tcggcaggac ggggggaggt gtgtcccggt aggtccctgg       420
   tgagctcacc cgtggcatcg gggacccgcg ggaacccacc gggcgcccac tagagactcg       480
   ggtcctaccc tcccccacac tactccaccg aaatgatcgg aagggcgcgc taggcctgct        540
   tccaagggct cagtgataaa ggcctcaaaa tcacactcca tcaagacttg gttgaagctt       600
   tgggtaggtt tgttgttgtt gttgttgttg tttgtttgtt tgttttagca gacacgtcct 660
   ggaaagaggt cctcagaacc caaaggttca ataatgattt gtggatggat tgattatagt 720
   ctgatatcgc tctggttcca cagaaacccg gagctccttg gcccactgtt accccagcag 780
   acctaaatgg acggtttctg tttttcactg gcagctcaga actggaccgg aagaagttcc 840
   cctccacttc ccccctcccg acaccagatc attgctgggt ttttattttc gggggaaaaa 900
   caacaacaac aacaacaaaa aaaacactag gtccttccag actggatcag gtgatcgggc 960
   aaaaaccctc aggctagtcc ggctgggtgc ccgagcatga aaaggcctcc gtggccgttt 1020
   gaacagggtg ttgcaaatga gaacttttgt aagccataac cagggcatcc tgagggtctg 1080
   agttcacggt caaggctgtg ggctactagg tccagcgagt ccaggcctcg ccccgccccc 1140
   gagctgccac agccaagatc ttcggcaggg aattcgagac cagggtcctc ccactcct 1198
<210> 15
   <211> 377
   <212> DNA
   <213> Homo sapiens
<400> 15
   tttcgtgccg ctgttttcaa tgcgctaacg aggcacgtta ttcttagccg cgtccgggag 60
   gggatcacat tcctgcgcag ttgcgctgct ggcggaagtg acttgttttc taacgaccct 120
   cgtgacagcc agagaatgtc cgtttctcgg agcgcagcac agcctgtccc atcgagaagc 180
   ctcgggtgag gggcccggtg ggcgcccgga ggccgctgga gggctgtggg agggacggtg 240
   gctccccact cccgtggcga agggcaggca aaccagaagc ctcttttgag agccgtttgg 300
   gattgagacg agtaagccac agcgagtggt tagaagtagg ttaggaagaa ggggaggtaa 360
   gaaagccgag tagggtt 377
<210> 16
   <211> 256
   <212> DNA
   <213> Homo sapiens
<400> 16
   gttcggtgga caagggggca gcgcccacag caagccggaa agagggaggc gcggggccgc 60
   gcttggggcc tgccgctgca cgccagcctg ggcaaagagc tgccaccttc tgcgggcgaa 120
   gcgggtcggg acgcaggacg gcagcggggc tggaggcagc tacgtgggtc cacaccccca 180
   tgccctgcaa ggctccttgg ccctgcttct cctctgtctc ggcgggagag gagcagcctc 240
   ggttttacag aatttc 256
<210> 17
   <211> 189
   <212> DNA
   <213> Homo sapiens
<400> 17
   tgtgccattt agtgagaggt gttttgggca aagaatcaat ttaactgtga ctgaccgacg       60
   ggcttgactg tattaattct gctaccgaaa aaaaaaaaaa aaaaaaagca atgagccgca      120
   agccttggac tcgcagagct gccggtgccc gtccgagagc cccaccagcg cggctcacgc      180
   ctcagtctc                                                              189
<210> 18
   <211> 707
   <212> DNA
   <213> Homo sapiens
<400> 18
   agagtcccag ttctgcaggc cgctccaggg ctaggggtag agatggtggc aggtggtgcg       60
   tcaactctct agggaagagg aacttgcatt acaaagactt gtctttctga gctgaagtca      120
   aaacgggggc gtcaagcgcg ctccgtttgg cggcggtgga ggggccgcgc gcccgcgctg      180
   tcccagccgg agctgccctg gctggtgatt ggaggtttaa cgtccggaat tcaggcgctt      240
   ctgcagctca gatttgccgg ccaaggggcc tcagttgcaa cttttcaaaa tggtgtttct      300
   ggaaaataac aaattcagac tcaactggtg acagcttttg gctatagaga atgaaactgc      360
   ttccctttgg cggtggaact cttaaacttc gaagagtgaa agaatacaat gaaataaaat      420
   gccataagat cactggattt ttcagaaaaa ggaagacccc aaattactcc caaaatgagg      480
   ctttgtaaat tcttgttaaa aatctttaaa tctcgaattt ccccctacaa catctgatga      540
   gtgctttaag agcaaacgag caaatcccac ctcgagaatc aacaaaccca agctctggcc      600
   aaggctctcc ccgcgttttc ttctcgtgac ctggggaatg tcccgcccca tcgctcacct      660
   ggctcttgtc atctcgctca tcttgaagtg acccgtggac aatgctg                    707
<210> 19
   <211> 182
   <212> DNA
   <213> Homo sapiens
<400> 19
   agctgccctc tgtggccatg agcgggtgtc cagccccttc caaggctgca ccggggagac       60
   gctggttttc tgctcgctgt gaccgaacaa agcccctaag agtcagtgcg cggaacagaa      120
   gagccggacc ccgacgggcc gagtcccaac gtgaggcacc cggcagagaa aacacgttca      180
   cg                                                                     182
<210> 20
   <211> 179
   <212> DNA
   <213> Homo sapiens
<400> 20
   cctcggcagc accggcatgg ctggaggcca gtacggccag gtgtggcggg agggagcgcc 60
   gtctggcttg ggtcgtccat cctgacagga cgctgcaagg gcaggagccc cgcgccccgt 120
   gtcctgcgcc cccgctcgag gacaagcccc agccgccggt ctccgctggg ttccgacag 179
<210> 21
   <211> 369
   <212> DNA
   <213> Homo sapiens
<400> 21
   ctttaagagg ctgtgcaggc agacagacct ccaggcccgc taggggatcc gcgccatgga 60
   ggccgcccgg gactatgcag gagccctcat caggcgagtg ccccgcgtcc ccctgattgc 120
   cgtgcgcttc caatcgcctt gcgttcggtg gcctcatatt cccctgtgcg cctctagtac 180
   cgtaccccgc tcccttcagc cccctgctcc ccgcattctc ttgcgctccg cgaccccgcg 240
   cacacaccca tccgccccac tggtgcccaa gccgtccagc cgcgcccgcg ggcagagccc 300
   aatcccgtcc cgcgcctcct caccctcttg cagctgggca caggtaccag gtgtggctct 360
   tgcgaggtg 369
<210> 22
   <211> 176
   <212> DNA
   <213> Homo sapiens
<400> 22
   agacttgcag aactcgggcc ccctggagga gacctaaccg ccacggtctt ggggaggttc 60
   cggagggcct cggttgtctg cactcccaac accaagaaac ccctgagacg cgaagctgcc 120
   agcgtgctgc cctcagagca gggcgacgca aagccagcgg accccggggt ggcggg 176
<210> 23
   <211> 167
   <212> DNA
   <213> Homo sapiens
<400> 23
   tgctcggctg gggggctcgc tccgcacttt cggtgccaga aaatgcccag aggagcgggg 60
   cggccccaga gcctcctttc ggggcgcgag gcccggcgcg tgtgtacgga gtccagtccc 120
   cccagggagt ggggtgcccg caccttcccc tccgcgctcg gagccac 167
<210> 24
   <211> 1205
   <212> DNA
   <213> Homo sapiens
<400> 24
   tcttgcacac ctgcttgtag ttctgcaccg agatctggtc gttgaggaac tgcacgcaga 60
   gcttggtgac ctgggggatg tgcaggatct tgctgaccga cagcacctcc tccaccgtgt 120
   ccagggacag ggtcacgttg gccgtgtaga ggtactcgag caccaggcgc agcccgatgg 180
   acgagcagcc ctgcagcacc aggttgttga tggcccgggg gctggtcagc agcttgtcgt 240
   cgggggagga agaaggagtc ccgggctcct cctgcggcgg cggctgctgc tgctgtgacg 300
   gctgctgctg cggcggctgc tgctggtcct tgggggcccc caggccgtcc tggccgccga 360
   cccctccccc gagagggggg tggctggaga agagcgatcg gaagtactgc gagcaggagg 420
   ccagcacggc cttgtggcaa tggaactgct ggccctgggc cgtcagggtc acgtcgcaaa 480
   acagctgctt cctccacagc aggttgaggc cgtgcagcag gttgtcgctg tggctggggt 540
   cgaaggtgga ggtcctgtcc ccggatctgg acatggcgag ctgactcggt gcacctggct 600
   ttaaaccctc ctccaacctg gcagacaggg gtgggggatg ggagggaggg gagcagggtg 660
   gtggagcggg tggggtgtgg tcggggtggg gaagggtgtg gaggggaggg gagggcgaag 720
   aacaagaatc aaggctcagc ttgactccct cctggcgcgc tccggacccc gaccctagga 780
   ggaaagtccg aagacgctgg atccgtgagc gccaccagaa gggccctgtc tggggtcccg 840
   gcgccggttc tgcgccctgc ggctcctctc gccacctccc acacacttcg tccctcactt 900
   tcctaaaacc aaccacctca gctcggctgt tggcagcaac agcagtggca gcagcgacgg 960
   caaagtggcg gctgaggccg aggcacctcg tgggctcgtg tccatgccgg gccagatgaa 1020
   gggaaaggcc gggaagtggg gagccggggg tgccctgaaa gctcagaggc gaccgacggc 1080
   gaaggttcca ggtcaacttg tgcccgaagc tttgcttttc gcagttggcc cagtttgggg 1140
   gagggggtag gaacaggggc ccgaccagcg tgcggggtgt gcgaatctta gctctccaaa 1200
   agctg 1205
<210> 25
   <211> 44
   <212> DNA
   <213> Homo sapiens
<400> 25
   cctctgtgtt agtgccctcg ggaatttggt tgatggggtg tttg 44
<210> 26
   <211> 5002
   <212> DNA
   <213> Homo sapiens
<400> 26
   tgatgtcgca cctgaacggc ctgcaccacc cgggccacac tcagtctcac gggccggtgc 60
   tggcacccag tcgcgagcgg ccaccctcgt cctcatcggg ctcgcaggtg gccacgtcgg 120
   gccagctgga agaaatcaac accaaagagg tggcccagcg catcacagcg gagctgaagc 180
   gctacagtat cccccaggcg atctttgcgc agagggtgct gtgccggtct caggggactc 240
   tctccgacct gctccggaat ccaaaaccgt ggagtaaact caaatctggc agggagacct 300
   tccgcaggat gtggaagtgg cttcaggagc ccgagttcca gcgcatgtcc gccttacgcc 360
   tggcaggtaa ggccggggct agccaggggc caggctgctg ggaagagggc tccgggtccg 420
   gtgcttgtgg cccaagtctg cgcgccgagt cacttctctt gattctttcc ttctctttcc 480
   tatacacgtc ctctttcttc tcgtttttat ttcttcttcc attttctctt tctcttccgc      540
   tcttccccta ctttcccttc tcccttttct ttttctttct tactctctcc ttgtccctga      600
   gctttcattg accgaccccc ccccatttca ttcgccctcc cctcaatgtg ccaacctttg 660
   ccctatttcc gatcttccca ggtactggga ggcgggatgg gggtgtgcgt tttcctctag 720
   gagccctgtc tttccaagac ccacagaaac caggacctgc ccttattcaa aaccccatgc 780
   acttcaagtc tcttttagac aacacatttc aattttccgg gctgactagt ctccctgtgc 840
   agaggcagtt gagaggcttt gctctgcaga gggaaaagag ctctctactc tcccacccac 900
   catataggca aacttatttg gtcattggct gaaggcacag ccttgccccc gcggggaacc 960
   ggcggccagg atacaacagc gctcctggag cccatctctg gccttggcgt tggcgcaggg 1020
   actttctgac cgggcttgag gggctcgggc cagctccaat gtcactacct acagcgaggg 1080
   cagggtgtaa ggttgagaag gtcacattca ccgctttggg aggacgtggg agaagagact 1140
   gaggtggaaa gcgctttgcc ttgctcaccg gccgtccttg ccccggtccc agcgtttgct 1200
   gggatttgcc aggatttgcc ggggctccgg gagaccctga gcactcgcag gaagaggtgc 1260
   tgagaaatta aaaattcagg ttagttaatg catccctgcc gccggctgca ggctccgcct 1320
   ttgcattaag cgggcgctga ttgtgcgcgc ctggcgaccg cggggaggac tggcggcccg 1380
   cgggagggga cgggtagagg cgcgggttac attgttctgg agccggctcg gctctttgtg 1440
   cctcctctag cggccaagct gcgaggtaca gccctctatt gttctaggag cacagaaacc 1500
   tcctgtgtgg gcggcgggtg cgcgagctag agggaaagat gcagtagtta ctgcgactgg 1560
   cacgcagttg cgcgcttttg tgcgcacgga ccccgcgcgg tgtgcgtggc gactgcgctg 1620
   cccctaggag caagccacgg gcccagaggg gcaaaatgtc caggtccccc gctgggaagg 1680
   acacactata ccctatggca agccagggtg ggcgacttcc catggatcgg gtggaggggg 1740
   gtatctttca ggatcggcgg gcggtctagg ggaacaattc gtggtggcga tgatttgcat 1800
   agcgcgggtc ttgggatgcg cgcggttccg agccagcctc gcacagctcg cttccggagc 1860
   tgcgagctca ggtttccacc cccgatcccc cgggctttcc tcgcaccgct gagcccagct 1920
   tgtggggtgc actcgaccaa cgcccgacag ggctggggaa tgtgacaggc agcaggttca 1980
   cccgggcttg ggagggggga gtttccgctt tgacagcatt ttcctttgcc gtctgctggt 2040
   ggattcctat tcccagtcgg taatcgcccc gcagtgttga tctaagaagg taaagaaaac 2100
   taggtttccc tgcaaagagc ctcccccaaa tcggcggact ccggatactt tgagtggatt 2160
   tagaaattta tgtaatcttt ctcctttagt ttatttttca tcctctccta cagttttctc 2220
   tgatttgctg ttggttcggg gcaagataaa gcagccagta gagagcgata ataatagcgg 2280
   cgggaaatga actggagact ggctgacagt tcttaacatt ttgtcataga tccccccgaa 2340
   tgtcccaggc tgtctctggt gggttttagt acccgccggc ttcttgggca ccggggacca 2400
   gaaggaactt ggcagctggt cttaggggta cagttaaagg caggatgaca gctattctcc 2460
   tgctcatctc agagcgctgc cgccccctca tgccggtcgc gcaaagaaca cagcttttaa 2520
   aaaacacgtg ccttctgccc atataggtct gaaagtgatg aggaaagtaa tgcttcgcct 2580
   attagcgagt ttcagctttt aaaatgatcc caagcgttgc tgagatgaga aagcgtggca 2640
   tcccgggggt cctcagcccc acccgcgccc atggtgcaag tctgcaggga caggcccggg 2700
   acagcactgc ccacgctgct agattttccg cagaggatcg ctgaagctgc cttcgtggga 2760
   gacagaatgc ctcctccagc gagtggaaaa ggcctgctga ggaccccgct ttgctcgagc 2820
   attcaaatgt gtgtctgttt tattaccctg ggttgaaaag ggacaagagc tttagccttt 2880
   ttatctggcc attttatcag caactacaag tgtgttgagt ggttattatt acataggagg 2940
   cttttcagtt tggggtcagt agatcagtct cttcagacac tgatgcagaa gctgggactg 3000
   gtaagtaggt attatgtgct cggagcgcta ggggacagga gcaaatggag aagaaaagcg 3060
   gaggctttct ccgcccggag tatcgatcgg aatccccgcc ggtacgccgc agagggccct 3120
   cgccgttggg ccccgggggt ttaacaagcc cagccgctcc gcaggcggct cggccggact 3180
   ctcagaccgg tgcctggaag acaccgtccc tgcccccctc ccgccaaacc tgcctcttct 3240
   ctttctctca taggttatag gttccctttc tctctcattt tggccccgcc cccgggtcct 3300
   gccaaacagc caagcaggcc ggggtttagg gggctcagaa tgaagaggtc tgatttggcc 3360
   agcgccggca aagctcaccc ttaggcgagg tcacaacaga ggcaggtcct tcctgcccag 3420
   cctgccggtg tagtcacagc caagggtggc acttgaaagg aaaagggaga aaacttcgga 3480
   gaaatttaga ttgccccaac gttagatttc agagaaattg actccaaatg cacggattcg 3540
   ttcggaaagg gcggctaagt ggcaggtggt tgcaaccccg cccggtcggg ccttcgcaga 3600
   ggttccccaa gaccagccct tgcagggcgg ttttcagcaa cctgacaaga ggcggccaag 3660
   acaaatttct gcgggttcga gcacacactc tcgggcgttg ggccccagag acctctaaac 3720
   caagcacaaa caagaaggga gtgagagaac ccaggctaga acttgcacgg gcatcccact 3780
   gaggaaaagc gaggcctcgg tggcaggcat gttttcttcc gacgcccgaa aatcgagccg 3840
   agcgcccgac tacatttact gcagaggttt ccgcctccag tgagcccgga tcccccagcg 3900
   gcctgcccgg agctggtctc cagtccccgc cgtagtccga cgcacggccc tctcctggca 3960
   gcaagctccc agcggccagt ctgaagccaa ttctgttcag gcggccgagg gcccttagcc 4020
   aacccaccat gatgtcgcct gggccacctg atgcccgcag cggcgggaca cggcccgggc 4080
   agtgcgcagt ggctcctgct aggggcaccg cgtgcgtgct tgtctcccgc tgcgccgggg 4140
   acgtccttgg gtgacacggg ccgctgggca cctcccaagc cgaggaaacg gacccccttc 4200
   gcagagtctc gcgcccaccc cccaacctcc cacctcgttt ctcgctgcta gggctcccga 4260
   ctcagcccac ctctcctggc ggtttagtta gggatcagag ctggagaggc tgaacgcaac 4320
   ccgtgccagt acggaacaga cgatatgttt gcctgctagc tgcttggatg aataattgaa 4380
   aagttcgctg cagtctgtgc ttcgtcaagt cccgggtgcc gggagaacac cttcccaaca 4440
   cgcatcaggg tgggcgggag cgggcagagg aggcgggacc cgagggagga gagtgaaccc 4500
   gagcaggaga agcagcccag gcagccaggc gccctcgatg cgagaggctg ggcatttatt 4560
   tttattccag gctttccact gtgtggttat gtcactttct caaacaaatg tgtatatgga 4620
   gggagatcga tgctgataat gtttagaaga ttaaaagagc attaatgctg gcaacaataa 4680
   cgtaaacgtg tggacccaga tttcattgat ctggaacttg atccggcgcg tttccagtaa 4740
   gcccgacggc gcgctcttcc cagcagagcg ctcaccagcg ccacggcccc gcggttttcc 4800
   agcggtgccg cttcgccagc tctgcgcggg ttctcccgtc tgaccgcagc tcctcccccg 4860
   cgaggcccca gcccgcctta cttccccgag gttttctcct cctctcgcgg ggctctctgc 4920
   cctctgcacc ccctcccccg acctctgcac cacccgcccc tgtgcgcaca caccgctact 4980
   tgcgcttccg gcgatccgcc tg 5002
<210> 27
   <211> 150
   <212> DNA
   <213> Homo sapiens
<400> 27
   aaccggagat ctgcttggtg aactgagagg agtccttagg agagcgggga cgccaggggc 60
   cgggggacac ttcgctctcg ccctagggaa ggtggtcttg acgctttcta ttgaagtcaa 120
   acttgaaaat atcagctgcc gctggactat 150
<210> 28
   <211> 339
   <212> DNA
   <213> Homo sapiens
<400> 28
   cgtgagcaga acgcccgccc tggagcagtt aggaccgaag gtctccggag agtcgccggc      60
   ggtgccaggt aacgcagagg gctcgggtcg ggccccgctt ctggggcttg ggactccggg     120
   cgcgcggagc cagccctctg gggcgaaatc cccgggcggc gtgcgcggtc cctctccgcg     180
   ctgtgctctc ccagcaactc cctgccacct cgacgagcct accggccgct ccgagttcga     240
   cttcctcgga cttagtggga gaaggggttg gaaatgggct gccgggactg ggggagctgc    300
   tctctggaag cagggaagct ggggcgcacc ggggcaggt                            339
<210> 29
   <211> 1961
   <212> DNA
   <213> Homo sapiens
<400> 29
   tagaagagga agactcctct ggccccacta ggtatcatcc gcgctctccc gctttccacc 60
   tgcgccctcg cttgggccaa tctctgccgc acgtgtccat ccctgaactg cacgctatcc 120
   tccacccccg gggggttcct gcgcactgaa agaccgttct ccggcaggtt ttgggatccg 180
   gcgacggctg accgcgcgcc gcccccacgc ccggttccac gatgctgcaa tacagaaagt 240
   ttacgtcggc cccgacccgc gcgggactgc agggtccgcc ggagcgcggc gcagaggctt 300
   ttcctgcgcg ttcggccccg ggaaaggggc gggagggctg gctccgggag cgcacgggcg 360
   cggcggggag ggtactcact gtgaagcacg ctgcgcccat ggatcatgtc tgtgcgttac 420
   accagaggct ccgggctcca ctaattccat ttagagacgg gaagacttcc agtggcgggg 480
   ggaggacagg gtcgagaggt gttaaagacg caaagcaaga aggaaataaa ggggggccga 540
   gagggagacc gagaggaagg gggagctccg agcccacgct gcagccagat ccggatgagt 600
   ccgtcctccg ccccgggcgg gctctcgctc tcgctggccc tcagcgccgc gcagccagca 660
   gcatccccac cgtgacgctc gcatcacacc cgggcgccgg ccgccaccat ccgcgccgcc 720
   gccgtcagga ccctcctccc gggcatcgtc gccgccgcgg ggtcgggagg acgcggcgcg 780
   cgggaggcgg cggtcgcagg gcgagccccg ggacgccccg agccggggcc ggggccgggg 840
   agagggcgca gcgaggtggg ggccagtcca gaccgacggc agcgacggag cgggcggcgg 900
   cggcggcgcc ggcggcggcg gggtggctca gtccccagtc tcagacgcgc cgcgcagcag 960
   gtcggagcag cctccccggg aggatgtcca gcggcagcgc tcctcgctcc agcccttggg 1020
   gatcttccgc tgaggcattg aaggcaggaa gaaggggtcc gtcatcggct cgccgggctg 1080
   cgcgccacct ctgctatctt gcggaaagag gagcgggtgg gtgggcgtct gggaggcggg 1140
   ctggagggcg gtgcagggga gcggggcggc cggggggggg gccggggggc ggggaaggga 1200
   gggaggagaa aggagccgga agagggcaga gttaccaaat gggctcctta gtcatggctt 1260
   ggggctccac gaccctcctg gaagcccgga gcctgggtgg gatagcgagg ctgcgcgcgg 1320
   ccggcgcccc ggggctggtg cgcggcagaa tggggccgcg gcggcggcag caaggacatc 1380
   ccagccgcgc ggatctgggg gaggggcggg gagggggtga ggacccggct gggatccgcg 1440
   gctcggcccg ccagggcgca gagagaggat gcagccgcaa atcccgagcc ggatcctcgt 1500
   gccggacgga aggcgtggaa gcgggagggg ccttcgtgtg aaaatccctt gtggggtttg 1560
   gtgtttcact ttttaaaggt tagaccttgc gggctctctg cctcccaccc cttcttttcc 1620
   atccgcgtaa aggaactggg cgccccctct ccctccctcc ctggggcgca ggtttcgccg 1680
   cggactccgc gctcagcttg ggagacacgg caggggcgcg ccccagggaa aggcggccgt 1740
   aaaagtttcg cggttgagca ctgggcctga tgtccagtcc ccccaccaaa ttactcctgc 1800
   aaagacgcgg gcttcttgca attgagcccc ccacctcgag gtatttaaaa ccaccccaag 1860
   gcacacacgg acccccgttc ccccgcgcca cttcctccta caggctcgcg cggcgcgtta 1920
   aagtctggga gacacgagtt gcggggaaac agcaccggaa g 1961
<210> 30
   <211> 314
   <212> DNA
   <213> Homo sapiens
<400> 30
   aagaaacagc tcatttcgga gctgaggaca aggcgtggga agaagacgcg tttggtttca 60
   cccaggcggg tggcggcaaa gctgtgggat gcgcgctgca cactccttcc gtcatcccgt 120
   tcccaccttc cacacacacc tgcgggaggt cggacatgtc ctgattgcgt gttcatcacg 180
   atggcaaacc gaacatgagg agaacgccac tgacgctggg tgcgccggct ttcccagccc 240
   tcgtgcataa cggggaggga gatgcagaag ttttttccaa catcggtgca aaggggaagc 300
   tgaggttttc ctat 314
<210> 31
   <211> 584
   <212> DNA
   <213> Homo sapiens
<400> 31
   tctgtcagct gctgccatgg ggcagcggga aggccctgga gggtgcctgg gctgtgtctg 60
   gtcccggcca cgcgtccctg cagcgtctga gaccttgtgg aacacacttg acccggcgct 120
   gggacggggt cggcccacac gcaccgccag cccgcaggag tgaggtgcag gctgccgctg 180
   gctccttagg cctcgacagc tctcttgagg tcggccctcc tcccctcccg agagctcagc 240
   agccgcagac ccaggcagag agagcaaagg aggctgtggt ggcccccgac gggaacctgg 300
   gtggccgggg gacacaccga ggaactttcc gccccccgac gggctctccc accgaggctc 360
   aggtgctcgt gggcagcaag gggaagcccc atggccatgc cgcttccctt tcaccctcag 420
   cgacgcgccc tcctgtgccc gcggggaaca agacggctct cggcggccat gcaggcggcc 480
   tgtcccacga acacgatgga gacctcagac gccgtcccca ccctgtcact gtcaccatca 540
   cccatcctgt cccctcacgc ctccccacat cccatcatta ctac 584
<210> 32
   <211> 349
   <212> DNA
   <213> Homo sapiens
<400> 32
   gaagtagaat cacagtaaat gaggagttag ggaatttagg gtagagatta aagtaatgaa      60
   cagaggagga ggcctgagac agctgcagag agaccctgtg ttccctgtga ggtgaagcgt     120
   ctgctgtcaa agccggttgg cgctgagaag aggtaccggg ggcagcaccc gcctcctggg     180
   agagggatgg gcctgcgggc acctggggga accgcacgga cacagacgac actataaacg     240
   cgggcgagac atcagggacc gggaaacaga aggacgcgcg tttcgagcag ctgcccagtg     300
   ggccacaagc cccgccacgc cacagcctct tcccctcagc acgcagaga                 349
<210> 33
   <211> 3510
   <212> DNA
   <213> Homo sapiens
<400> 33
   tactccggcg acgggaggat gttgagggaa gcctgccagg tgaagaaggg gccagcagca 60
   gcacagagct tccgactttg ccttccaggc tctagactcg cgccatgcca agacgggccc 120
   ctcgactttc acccctgact cccaactcca gccactggac cgagcgcgca aagaacctga 180
   gaccgcttgc tctcaccgcc gcaagtcggt cgcaggacag acaccagtgg gcagcaacaa 240
   aaaaagaaac cgggttccgg gacacgtgcc ggcggctgga ctaacctcag cggctgcaac 300
   caaggagcgc gcacgttgcg cctgctggtg tttattagct acactggcag gcgcacaact 360
   ccgcgccccg actggtggcc ccacagcgcg caccacacat ggcctcgctg ctgttggcgg 420
   ggtaggcccg aaggaggcat ctacaaatgc ccgagccctt tctgatcccc acccccccgc 480
   tccctgcgtc gtccgagtga cagattctac taattgaacg gttatgggtc atccttgtaa 540
   ccgttggacg acataacacc acgcttcagt tcttcatgtt ttaaatacat atttaacgga 600
   tggctgcaga gccagctggg aaacacgcgg attgaaaaat aatgctccag aaggcacgag 660
   actggggcga aggcgagagc gggctgggct tctagcggag accgcagagg gagacatatc 720
   tcagaactag gggcaataac gtgggtttct ctttgtattt gtttattttg taactttgct 780
   acttgaagac caattattta ctatgctaat ttgtttgctt gtttttaaaa ccgtacttgc 840
   acagtaaaag ttccccaaca acggaagtaa cccgacgttc ctcacactcc ctaggagact 900
   gtgtgcgtgt gtgcccgcgc gtgcgctcac agtgtcaagt gctagcatcc gagatctgca 960
   gaaacaaatg tctgaattcg aaatgtatgg gtgtgagaaa ttcagctcgg ggaagagatt 1020
   agggactggg ggagacaggt ggctgcctgt actataagga accgccaacg ccagcatctg 1080
   tagtccaagc agggctgctc tgtaaaggct tagcaatttt ttctgtaggc ttgctgcaca 1140
   cggtctctgg cttttcccat ctgtaaaatg ggtgaatgca tccgtacctc agctacctcc 1200
   gtgaggtgct tctccagttc gggcttaatt cctcatcgtc aagagttttc aggtttcaga 1260
   gccagcctgc aatcggtaaa acatgtccca acgcggtcgc gagtggttcc atctcgctgt 1320
   ctggcccaca gcgtggagaa gccttgccca ggcctgaaac ttctctttgc agttccagaa 1380
   agcaggcgac tgggacggaa ggctctttgc taacctttta cagcggagcc ctgcttggac 1440
   tacagatgcc agcgttgccc ctgccccaag gcgtgtggtg atcacaaaga cgacactgaa 1500
   aatacttact atcatccggc tcccctgcta ataaatggag gggtgtttaa ctacaggcac 1560
   gaccctgccc ttgtgctagc gcggttaccg tgcggaaata actcgtccct gtacccacac 1620
   catcctcaac ctaaaggaga gttgtgaatt ctttcaaaac actcttctgg agtccgtccc 1680
   ctccctcctt gcccgccctc tacccctcaa gtccctgccc ccagctgggg gcgctaccgg 1740
   ctgccgtcgg agctgcagcc acggccatct cctagacgcg cgagtagagc accaagatag 1800
   tggggacttt gtgcctgggc atcgtttaca tttggggcgc caaatgccca cgtgttgatg 1860
   aaaccagtga gatgggaaca ggcggcggga aaccagacag aggaagagct agggaggaga 1920
   ccccagcccc ggatcctggg tcgccagggt tttccgcgcg catcccaaaa ggtgcggctg 1980
   cgtggggcat caggttagtt tgttagactc tgcagagtct ccaaaccatc ccatccccca 2040
   acctgactct gtggtggccg tattttttac agaaatttga ccacgttccc tttctccctt 2100
   ggtcccaagc gcgctcagcc ctccctccat cccccttgag ccgcccttct cctccccctc 2160
   gcctcctcgg gtccctcctc cagtccctcc ccaagaatct cccggccacg ggcgcccatt 2220
   ggttgtgcgc agggaggagg cgtgtgcccg gcctggcgag tttcattgag cggaattagc 2280
   ccggatgaca tcagcttccc agccccccgg cgggcccagc tcattggcga ggcagcccct 2340
   ccaggacacg cacattgttc cccgcccccg cccccgccac cgctgccgcc gtcgccgctg 2400
   ccaccgggct ataaaaaccg gccgagcccc taaaggtgcg gatgcttatt atagatcgac 2460
   gcgacaccag cgcccggtgc caggttctcc cctgaggctt ttcggagcga gctcctcaaa 2520
   tcgcatccag agtaagtgtc cccgccccac agcagccgca gcctagatcc cagggacaga 2580
   ctctcctcaa ctcggctgtg acccagaatg ctccgataca gggggtctgg atccctactc 2640
   tgcgggccat ttctccagag cgactttgct cttctgtcct ccccacactc accgctgcat 2700
   ctccctcacc aaaagcgaga agtcggagcg acaacagctc tttctgccca agccccagtc 2760
   agctggtgag ctccccgtgg tctccagatg cagcacatgg actctgggcc ccgcgccggc 2820
   tctgggtgca tgtgcgtgtg cgtgtgtttg ctgcgtggtg tcgatggaga taaggtggat 2880
   ccgtttgagg aaccaaatca ttagttctct atctagatct ccattctccc caaagaaagg 2940
   ccctcacttc ccactcgttt attccagccc gggggctcag ttttcccaca cctaactgaa 3000
   agcccgaagc ctctagaatg ccacccgcac cccgagggtc accaacgctc cctgaaataa 3060
   cctgttgcat gagagcagag gggagataga gagagcttaa ttataggtac ccgcgtgcag 3120
   ctaaaaggag ggccagagat agtagcgagg gggacgagga gccacgggcc acctgtgccg 3180
   ggaccccgcg ctgtggtact gcggtgcagg cgggagcagc ttttctgtct ctcactgact 3240
   cactctctct ctctctccct ctctctctct ctcattctct ctcttttctc ctcctctcct 3300
   ggaagttttc gggtccgagg gaaggaggac cctgcgaaag ctgcgacgac tatcttcccc 3360
   tggggccatg gactcggacg ccagcctggt gtccagccgc ccgtcgtcgc cagagcccga 3420
   tgaccttttt ctgccggccc ggagtaaggg cagcagcggc agcgccttca ctgggggcac 3480
   cgtgtcctcg tccaccccga gtgactgccc 3510
<210> 34
   <211> 333
   <212> DNA
   <213> Homo sapiens
<400> 34
   ttaattcgaa aatggcagac agagctgagc gctgccgttc ttttcaggat tgaaaatgtg 60
   ccagtgggcc aggggcgctg ggacccgcgg tgcggaagac tcggaacagg aagaaatagt 120
   ggcgcgctgg gtgggctgcc ccgccgccca cgccggttgc cgctggtgac agtggctgcc 180
   cggccaggca cctccgagca gcaggtctga gcgtttttgg cgtcccaagc gttccgggcc 240
   gcgtcttcca gagcctctgc tcccagcggg gtcgctgcgg cctggcccga aggatttgac 300
   tctttgctgg gaggcgcgct gctcagggtt ctg 333
<210> 35
   <211> 385
   <212> DNA
   <213> Homo sapiens
<400> 35
   ccggtcccca gtttggaaaa aggcgcaaga agcgggcttt tcagggaccc cggggagaac 60
   acgagggctc cgacgcggga gaaggattga agcgtgcaga ggcgccccaa attgcgacaa 120
   tttactggga tccttttgtg gggaaaggag gcttagaggc tcaagctata ggctgtccta 180
   gagcaactag gcgagaacct ggccccaaac tccctcctta cgccctggca caggttcccg 240
   gcgactggtg ttcccaaggg agccccctga gcctaccgcc cttgcagggg gtcgtgctgc 300
   ggcttctggg tcataaacgc cgaggtcggg ggtggcggag ctgtagaggc tgcccgcgca 360
   gaaagctcca ggatcccaat atgtg 385
<210> 36
   <211> 105
   <212> DNA
   <213> Homo sapiens
<400> 36
   gcgcaggtcc ccccagtccc cgagggagtg cgcccgacgg aaacgcccct agcccgcggg 60
   cctcgctttc ctctcccggg ttcctgggtc acttcccgct gtctc 105
<210> 37
   <211> 147
   <212> DNA
   <213> Homo sapiens
<400> 37
   ttccctcgcg gctttggaaa gggggtgcaa atgcaccctt ctgcgggccc gctacccgct 60
   gcaacacctg tgtttccttt ctgggcacct tctaggtttc tagatattgc tgtgaatacg 120
   gtcctccgct gtacagttga aaacaaa 147
<210> 38
   <211> 365
   <212> DNA
   <213> Homo sapiens
<400> 38
   tgggaattta ggtcgggcac tgccgatatg tcgccttcca caaggcgggc ccgggcctct 60
   gctgaccgtg caccggtcct ggggctgggt aattctgcag cagcagcgca gcccatgccg 120
   gggaatttgc gggcagagga gacagtgagg cccgcgttct gtgcgggaac tcccgagctc 180
   acagagccca agaccacacg gctgcatctg cttggctgac tgggccaggc ccacgcgtag 240
   taacccggac gtctctctct cacagtcccc ttgcgtctgg ccagggagct gccaggctgc 300
   accccgcggt ggggatcggg agaggggcag tgtcgcccat ccccggaagg ctgagcctgg 360
   tgcag 365
<210> 39
   <211> 418
   <212> DNA
   <213> Homo sapiens
<400> 39
   cggttttctc ctggaggact gtgttcagac agatactggt ttccttatcc gcaggtgtgc 60
   gcggcgctcg caagtggtca gcataacgcc gggcgaattc ggaaagcccg tgcgtccgtg 120
   gacgacccac ttggaaggag ttgggagaag tccttgttcc cacgcgcgga cgcttccctc 180
   cgtgtgtcct tcgagccaca aaaagcccag accctaaccc gctcctttct cccgccgcgt 240
   ccatgcagaa ctccgccgtt cctgggaggg gaagcccgcg aggcgtcggg agaggcacgt 300
   cctccgtgag caaagagctc ctccgagcgc gcggcgggga cgctgggccg acaggggacc 360
   gcgggggcag ggcggagagg acccgccctc gagtcggccc agccctaaca ctcaggac 418
<210> 40
   <211> 906
   <212> DNA
   <213> Homo sapiens
<400> 40
   agggaatcgg gctgaccagt cctaaggtcc cacgctcccc tgacctcagg gcccagagcc 60
   tcgcattacc ccgagcagtg cgttggttac tctccctgga aagccgcccc cgccggggca 120
   agtgggagtt gctgcactgc ggtctttgga ggcctaggtc gcccagagta ggcggagccc 180
   tgtatccctc ctggagccgg cctgcggtga ggtcggtacc cagtacttag ggagggagga 240
   cgcgcttggt gctcagggta ggctgggccg ctgctagctc ttgatttagt ctcatgtccg 300
   cctttgtgcc ggcctctccg atttgtgggt ccttccaaga aagagtcctc tagggcagct 360
   agggtcgtct cttgggtctg gcgaggcggc aggccttctt cggacctatc cccagaggtg 420
   taacggagac tttctccact gcagggcggc ctggggcggg catctgccag gcgagggagc 480
   tgccctgccg ccgagattgt ggggaaacgg cgtggaagac accccatcgg agggcaccca 540
   atctgcctct gcactcgatt ccatcctgca acccaggaga aaccatttcc gagttccagc 600
   cgcagaggca cccgcggagt tgccaaaaga gactcccgcg aggtcgctcg gaaccttgac 660
   cctgacacct ggacgcgagg tctttcagga ccagtctcgg ctcggtagcc tggtccccga 720
   ccaccgcgac caggagttcc ttcttccctt cctgctcacc agccggccgc cggcagcggc 780
   tccaggaagg agcaccaacc cgcgctgggg gcggaggttc aggcggcagg aatggagagg 840
   ctgatcctcc tctagccccg gcgcattcac ttaggtgcgg gagccctgag gttcagcctg 900
   actttc 906
<210> 41
   <211> 860
   <212> DNA
   <213> Homo sapiens
<400> 41
   cactacggat ctgcctggac tggttcagat gcgtcgttta aagggggggg ctggcactcc 60
   agagaggagg gggcgctgca ggttaattga tagccacgga agcacctagg cgccccatgc 120
   gcggagccgg agccgccagc tcagtctgac ccctgtcttt tctctcctct tccctctccc 180
   acccctcact ccgggaaagc gagggccgag gtaggggcag atagatcacc agacaggcgg 240
   agaaggacag gagtacagat ggagggacca ggacacagaa tgcaaaagac tggcaggtga 300
   gaagaaggga gaaacagagg gagagagaaa gggagaaaca gagcagaggc ggccgccggc 360
   ccggccgccc tgagtccgat ttccctcctt ccctgaccct tcagtttcac tgcaaatcca 420
   cagaagcagg tttgcgagct cgaatacctt tgctccactg ccacacgcag caccgggact 480
   gggcgtctgg agcttaagtc tgggggtctg agcctgggac cggcaaatcc gcgcagcgca 540
   tcgcgcccag tctcggagac tgcaaccacc gccaaggagt acgcgcggca ggaaacttct 600
   gcggcccaat ttcttcccca gctttggcat ctccgaaggc acgtacccgc cctcggcaca 660
   agctctctcg tcttccactt cgacctcgag gtggagaaag aggctggcaa gggctgtgcg 720
   cgtcgctggt gtggggaggg cagcaggctg cccctccccg cttctgcagc gagttttccc 780
   agccaggaaa agggagggag ctgtttcagg aatttcagtg ccttcaccta gcgactgaca 840
   caagtcgtgt gtataggaag 860
<210> 42
   <211> 452
   <212> DNA
   <213> Homo sapiens
<400> 42
   ggagcctgaa gtcagaaaag atggggcctc gttactcact ttctagccca gcccctggcc 60
   ctgggtcccg cagagccgtc atcgcaggct cctgcccagc ctctggggtc gggtgagcaa 120
   ggtgttctct tcggaagcgg gaagggctgc gggtcgggga cgtcccttgg ctgccacccc 180
   tgattctgca tccttttcgc tcgaatccct gcgctaggca tcctccccga tcccccaaaa 240
   gcccaagcac tgggtctggg ttgaggaagg gaacgggtgc ccaggccgga cagaggctga 300
   aaggaggcct caaggttcct ctttgctaca aagtggagaa gttgctctac tctggagggc 360
   agtggccttt tccaaacttt tccacttagg tccgtaagaa aagcaattca tacacgatca 420
   gcgctttcgg tgcgaggatg gaaagaaact tc 452
<210> 43
   <211> 1992
   <212> DNA
   <213> Homo sapiens
<400> 43
   ttttcctgtt acagagctga gcccactcat gtggtgccaa gtagcgacta tctctcggcc      60
   acctccaccc agagcaatgt gggcgccccc agcgggtggg agcgattgcc gagcggcgca     120
   agggcgttta acgcctaacc ccctcctcct gggttgccaa gccgctaggt cgccgtttcc     180
   aacgtggctg cgcgggactg aagtccgacg actcctcgtc ctcagtagga gacacacctc     240
   ccactgcccc cagccacgcg agctatgggc agaatcgggg caacggtaat atctggatgg     300
   ggcaggctcc cctgaggctg tgcttaagaa aaaaggaatc tggagtagcc tgaggggccc     360
   cacgaggggg cctcctttgc gatcgtctcc cagccttagg ccaaggctac ggaggcaggc     420
   ggccgagtgt tggcgcccag cccggccgag gactggatgg aggacgagaa gcagcctgcc     480
   tctgggcgac agctgcggac gcagcctcgc cgcctcgccg cctcagcctc ggtcccagcg     540
   tctctaaagc cgcgcccatt ttacagatgc agggcaggga gacaagaggc atctccgggg     600
   gccgagtaga atgatggcgc gggttctccc ggcgccctga tttcgaggct gcgcccgggg     660
   ccctacatgc aggcggggag gcctgggccg aaggcgtctg caaggagggg cgagtctgcc     720
   cggtccgggc agggagtgag gccacagtca gttctcccta ggaggccgcg cagcgggtag     780
   ggtatgggac tgggggacgc aacggggacc tggccgaatc agagccctca gcagagaacg 840
   ccgaaaactc tggggccggc cgctcgcttc ccgctagtgg gaatggtttc cggtcatccg 900
   ttcccagtcc agccccgggt agggagctct gatttgcaat gcacagcact tgcgaggttc 960
   gaatgccccc gcaatttgca gatggaaata ctaagcctag gccgggcgtg gtggctcaag 1020
   cctatcatct cagccctttg ggaggccaag ccgggaggat tgtttgagcc caagaattca 1080
   aaaccagcct gagcaacata gcgaccccgt ctctacaaaa taaaataaaa taaattatcc 1140
   gggcgtggtg gcacgcgcct gtggttccag ctactccgga ggctgaggtg ggaggatcgc 1200
   ttgagtccgg gaggtcgagg ctacagtgag ccgtgatcgc accactgcac tccagcctgg 1260
   gcgacagagt gagaccttgt ctcaaaaaag gaaaaaaaga aaaagaaagt aagcttcaaa 1320
   gaagctctga taatagttct gggtcgtgca gcggtggcgg ccccgcgctc tcgcccctaa 1380
   agcaagcgct ctttgtactg ggtggaggag ctttgagtag tgagggtgga gatgcagctt 1440
   cggggtggcg cagccaccct gacactaggc ccggggtcgc agtgggacag aagagtctgc 1500
   cgctctgact tgggctctga gttccaaggg cgcccggcac ttctagcctc ccaggcttgc 1560
   gcgctggcgc ctttgccatc cgtgccgaag tggggagacc tagccgcgac caccacgagc 1620
   gcagcggtga cacccagagg tcccaccggg cccctgggca gggtaacctt agcctgtccg 1680
   cttcggcagc tttgcgaaga gtggcgcgca gctagggctg aggctcttgc ggacctgcgg 1740
   tcgaagcagg cggctgagcc agttcgatcg ccaaggcctg ggctgccgac agtggtgcgc 1800
   gctctgttcc gccgcggccg ggccaggcgc tctggaatag cgatgggggg acacggcctc 1860
   caactttctg cagagaccat cgggcagctc cgggcctaag cagcgacctc accgaaggtt 1920
   cctgggaacc tttgccaaaa tcccagcctc tgcctcggtc cagctaaacc gtgtgtaaac 1980
   aagtgcacca ag 1992
<210> 44
   <211> 448
   <212> DNA
   <213> Homo sapiens
<400> 44
   ataaaggacc gggtaatttc gcggaatgcg gattttgaga caggcccaga cggcggcgga      60
   ttccctgtgt cccccaactg gggcgatctc gtgaacacac ctgcgtccca ccccgatcct     120
   aggttggggg gaaagggtat gggaaccctg agcccagagc gcgccccgct ctttcctttg     180
   ctccccggct tccctggcca gccccctccc ggctggtttc ctcgctcact cggcgcctgg     240
   cgtttcgggc gtctggagat caccgcgtgt ctggcacccc aacgtctagt ctccccgcag     300
   gttgaccgcg gcgcctggag ccgggaatag gggtggggag tccggagaac caaacccgag     360
   cctgaagttg ccattcgggt gactcccgag aaagcccggg agcattttgg ccaatgcggg     420
   tttttacctg aacttcagca tcttcacc                                        448
<210> 45
   <211> 395
   <212> DNA
   <213> Homo sapiens
<400> 45
   aattggaaaa ccctggtatt gtgcctgttt gggggaagaa aacgtcaata aaaattaatt      60
   gatgagttgg cagggcgggc ggtgcgggtt cgcggcgagg cgcagggtgt catggcaaat     120
   gttacggctc agattaagcg attgttaatt aaaaagcgac ggtaattaat actcgctacg     180
   ccatatgggc ccgtgaaaag gcacaaaagg tttctccgca tgtggggttc cccttctctt     240
   ttctccttcc acaaaagcac cccagcccgt gggtcccccc tttggcccca aggtaggtgg     300
   aactcgtcac ttccggccag ggaggggatg gggcggtctc cggcgagttc caagggcgtc     360
   cctcgttgcg cactcgcccg cccaggttct ttgaa                                395
<210> 46
   <211> 491
   <212> DNA
   <213> Homo sapiens
<400> 46
   gggaagcgat cgtctcctct gtcaactcgc gcctgggcac ttagcccctc ccgtttcagg 60
   gcgccgcctc cccggatggc aaacactata aagtggcggc gaataaggtt cctcctgctg 120
   ctctcggttt agtccaagat cagcgatatc acgcgtcccc cggagcatcg cgtgcaggag 180
   ccatggcgcg ggagctatac cacgaagagt tcgcccgggc gggcaagcag gcggggctgc 240
   aggtctggag gattgagaag ctggagctgg tgcccgtgcc ccagagcgct cacggcgact 300
   tctacgtcgg ggatgcctac ctggtgctgc acacggccaa gacgagccga ggcttcacct 360
   accacctgca cttctggctc ggtaagggac ggcgggcggc gggaccccga cgcaccaagg 420
   ccggcgaggg gagggcgtag gggtctgaga tttgcaggcg tgggagtaaa ggggaccgca 480
   aactgagcta g 491
<210> 47
   <211> 1284
   <212> DNA
   <213> Homo sapiens
<400> 47
   ctcaggggcg ggaagtggcg ggtgggagtc acccaagcgt gactgcccga ggcccctcct 60
   gccgcggcga ggaagctcca taaaagccct gtcgcgaccc gctctctgca ccccatccgc 120
   tggctctcac ccctcggaga cgctcgcccg acagcatagt acttgccgcc cagccacgcc 180
   cgcgcgccag ccaccgtgag tgctacgacc cgtctgtcta ggggtgggag cgaacggggc 240
   gcccgcgaac ttgctagaga cgcagcctcc cgctctgtgg agccctgggg ccctgggatg 300
   atcgcgctcc actccccagc ggactatgcc ggctccgcgc cccgacgcgg accagccctc 360
   ttggcggcta aattccactt gttcctctgc tcccctctga ttgtccacgg cccttctccc 420
   gggcccttcc cgctgggcgg ttcttctgag ttacctttta gcagatatgg agggagaacc 480
   cgggaccgct atcccaaggc agctggcggt ctccctgcgg gtcgccgcct tgaggcccag 540
   gaagcggtgc gcggtaggaa ggtttccccg gcagcgccat cgagtgagga atccctggag 600
   ctctagagcc ccgcgccctg ccacctccct ggattcttgg gctccaaatc tctttggagc 660
   aattctggcc cagggagcaa ttctctttcc ccttccccac cgcagtcgtc accccgaggt 720
   gatctctgct gtcagcgttg atcccctgaa gctaggcaga ccagaagtaa cagagaagaa 780
   acttttcttc ccagacaaga gtttgggcaa gaagggagaa aagtgaccca gcaggaagaa 840
   cttccaattc ggttttgaat gctaaactgg cggggccccc accttgcact ctcgccgcgc 900
   gcttcttggt ccctgagact tcgaacgaag ttgcgcgaag ttttcaggtg gagcagaggg 960
   gcaggtcccg accggacggc gcccggagcc cgcaaggtgg tgctagccac tcctgggttc 1020
   tctctgcggg actgggacga gagcggattg ggggtcgcgt gtggtagcag gaggaggagc 1080
   gcggggggca gaggagggag gtgctgcgcg tgggtgctct gaatccccaa gcccgtccgt 1140
   tgagccttct gtgcctgcag atgctaggta acaagcgact ggggctgtcc ggactgaccc 1200
   tcgccctgtc cctgctcgtg tgcctgggtg cgctggccga ggcgtacccc tccaagccgg 1260
   acaacccggg cgaggacgca ccag 1284
<210> 48
   <211> 554
   <212> DNA
   <213> Homo sapiens
<400> 48
   tggagaacct tgggctctgt ggcctcaaag gtaggggtga tttcgagggg ccggcacctc      60
   acagggcagg ttccaccgcg gaaacgcagt catcgcccag cgaccctgct cctggccctc     120
   agcctccccc caggtttctt tttctcttga atcaagccga ggtgcgccaa tggccttcct     180
   tgggtcggat ccgggggccc agggccagct tacctgcttt caccgagcag tggatatgtg     240
   ccttggactc gtagtacacc cagtcgaagc cggcctccac cgccaggcgg gccagcatgc     300
   cgtacttgct gcggtcgcgg tcagacgtgg tgatgtccac tgcgcggccc tcgtagtgca     360
   gagactcctc tgagtggtgg ccatcttcgt cccagccctc ggtcacccgc agtttcactc     420
   ctggccactg gttcatcacc gagatggcca aagcgttcaa cttgtcctta cacctctgcg     480
   aagacaaggg gacccccacc gacggacacg ttagcctggg caaccgccac ccctcccggc     540
   ccctccatca gcct                                                       554
<210> 49
   <211> 772
   <212> DNA
   <213> Homo sapiens
<400> 49
   tctcacgacc catccgttaa cccaccgttc ccaggagctc cgaggcgcag cggcgacaga      60
   ggttcgcccc ggcctgctag cattggcatt gcggttgact gagcttcgcc taacaggctt     120
   ggggagggtg ggctgggctg ggctgggctg ggctgggtgc tgcccggctg tccgcctttc     180
   gttttcctgg gaccgaggag tcttccgctc cgtatctgcc tagagtctga atccgacttt     240
   ctttcctttg ggcacgcgct cgccagtgga gcacttcttg ttctggcccc gggctgatct     300
   gcacgcggac ttgagcaggt gccaaggtgc cacgcagtcc cctcacggct ttcggggggt     360
   cttggagtcg ggtggggagg gagacttagg tgtggtaacc tgcgcaggtg ccaaagggca     420
   gaaggagcag ccttggatta tagtcacggt ctctccctct cttccctgcc atttttaggg     480
   ctttctctac gtgctgttgt ctcactgggt ttttgtcgga gccccacgcc ctccggcctc     540
   tgattcctgg aagaaagggt tggtcccctc agcaccccca gcatcccgga aaatggggag     600
   caaggctctg ccagcgccca tcccgctcca cccgtcgctg cagctcacca attactcctt     660
   cctgcaggcc gtgaacacct tcccggccac ggtggaccac ctgcagggcc tgtacggtct     720
   cagcgcggta cagaccatgc acatgaacca ctggacgctg ggtatcccca at             772
<210> 50
   <211> 1362
   <212> DNA
   <213> Homo sapiens
<400> 50
   tggtttcctt tcgcttctcg cctcccaaac acctccagca agtcggaggg cgcgaacgcg 60
   gagccagaaa cccttcccca aagtttctcc cgccaggtac ctaattgaat catccatagg 120
   atgacaaatc agccagggcc aagatttcca gacacttgag tgacttcccg gtccccgagg 180
   tgacttgtca gctccagtga gtaacttgga actgtcgctc ggggcaaggt gtgtgtctag 240
   gagagagccg gcggctcact cacgctttcc agagagcgac ccgggccgac ttcaaaatac 300
   acacagggtc atttataggg actggagccg cgcgcaggac aacgtctccg agactgagac 360
   attttccaaa cagtgctgac attttgtcgg gccccataaa aaatgtaaac gcgaggtgac 420
   gaacccggcg gggagggttc gtgtctggct gtgtctgcgt cctggcggcg tgggaggtta 480
   tagttccaga cctggcggct gcggatcgcc gggccggtac ccgcgaggag tgtaggtacc 540
   ctcagcccga ccacctcccg caatcatggg gacaccggct tggatgagac acaggcgtgg 600
   aaaacagcct tcgtgaaact ccacaaacac gtggaacttg aaaagacaac tacagccccg 660
   cgtgtgcgcg agagacctca cgtcacccca tcagttccca cttcgccaaa gtttcccttc 720
   agtggggact ccagagtggt gcgccccatg cccgtgcgtc ctgtaacgtg ccctgattgt 780
   gtacccctct gcccgctcta cttgaaatga aaacacaaaa actgttccga attagcgcaa 840
   ctttaaagcc ccgttatctg tcttctacac tgggcgctct taggccactg acagaaacat 900
   ggtttgaacc ctaattgttg ctatcagtct cagtcagcgc aggtctctca gtgacctgtg 960
   acgccgggag ttgaggtgcg cgtatcctta aacccgcgcg aacgccaccg gctcagcgta 1020
   gaaaactatt tgtaatccct agtttgcgtc tctgagcttt aactccccca cactctcaag 1080
   cgcccggttt ctcctcgtct ctcgcctgcg agcaaagttc ctatggcatc cacttaccag 1140
   gtaaccggga tttccacaac aaagcccggc gtgcgggtcc cttcccccgg ccggccagcg 1200
   cgagtgacag cgggcggccg gcgctggcga ggagtaactt ggggctccag cccttcagag 1260
   cgctccgcgg gctgtgcctc cttcggaaat gaaaaccccc atccaaacgg ggggacggag 1320
   cgcggaaacc cggcccaagt gccgtgtgtg cgcgcgcgtc tg 1362
<210> 51
   <211> 476
   <212> DNA
   <213> Homo sapiens
<400> 51
   gaaagccatc cttaccattc ccctcaccct ccgccctctg atcgcccacc cgccgaaagg        60
   gtttctaaaa atagcccagg gcttcaaggc cgcgcttctg tgaagtgtgg agcgagcggg       120
   cacgtagcgg tctctgccag gtggctggag ccctggaagc gagaaggcgc ttcctccctg       180
   catttccacc tcaccccacc cccggctcat ttttctaaga aaaagttttt gcggttccct       240
   ttgcctccta cccccgctgc cgcgcggggt ctgggtgcag acccctgcca ggttccgcag       300
   tgtgcagcgg cggctgctgc gctctcccag cctcggcgag ggttaaaggc gtccggagca       360
   ggcagagcgc cgcgcgccag tctattttta cttgcttccc ccgccgctcc gcgctccccc       420
   ttctcagcag ttgcacatgc cagctctgct gaaggcatca atgaaaacag cagtag           476
<210> 52
   <211> 300
   <212> DNA
   <213> Homo sapiens
<400> 52
   atcgaaaatg tcgacatctt gctaatggtc tgcaaacttc cgccaattat gactgacctc        60
   ccagactcgg ccccaggagg ctcgtattag gcagggaggc cgccgtaatt ctgggatcaa       120
   aagcgggaag gtgcgaactc ctctttgtct ctgcgtgccc ggcgcgcccc cctcccggtg       180
   ggtgataaac ccactctggc gccggccatg cgctgggtga ttaatttgcg aacaaacaaa       240
   agcggcctgg tggccactgc attcgggtta aacattggcc agcgtgttcc gaaggcttgt       300
<210> 53
   <211> 1246
   <212> DNA
   <213> Homo sapiens
<400> 53
   atcaacatcg tggctttggt cttttccatc atggtgagtg aatcacggcc agaggcagcc 60
   tgggaggaga gacccgggcg gctttgagcc cctgcagggg agtccgcgcg ctctctgcgg 120
   ctcccttcct cacggcccgg cccgcgctag gtgttctttg tcctcgcacc tcctcctcac 180
   ctttctcggg ctctcagagc tctccccgca atcatcagca cctcctctgc actcctcgtg 240
   gtactcagag ccctgatcaa gcttccccca ggctagcttt cctcttcttt ccagctccca 300
   gggtgcgttt cctctccaac ccggggaagt tcttccgtgg actttgctga ctcctctgac 360
   cttcctaggc acttgcccgg ggcttctcaa ccctcttttc tagagcccca gtgcgcgcca 420
   ccctagcgag cgcagtaagc tcataccccg agcatgcagg ctctacgttc ctttccctgc 480
   cgctccgggg gctcctgctc tccagcgccc aggactgtct ctatctcagc ctgtgctccc 540
   ttctctcttt gctgcgccca agggcaccgc ttccgccact ctccgggggg tccccaggcg 600
   attcctgatg ccccctcctt gatcccgttt ccgcgctttg gcacggcacg ctctgtccag 660
   gcaacagttt cctctcgctt cttcctacac ccaacttcct ctccttgcct ccctccggcg 720
   cccccttttt aacgcgcccg aggctggctc acacccacta cctctttagg cctttcttag 780
   gctccccgtg tgcccccctc accagcaaag tgggtgcgcc tctcttactc tttctaccca 840
   gcgcgtcgta gttcctcccc gtttgctgcg cactggccct aacctctctt ctcttggtgt 900
   cccccagagc tcccaggcgc ccctccaccg ctctgtcctg cgcccggggc tctcccggga 960
   atgaactagg ggattccacg caacgtgcgg ctccgcccgc cctctgcgct cagacctccc 1020
   gagctgcccg cctctctagg agtggccgct ggggcctcta gtccgccctt ccggagctca 1080
   gctccctagc cctcttcaac cctggtagga acacccgagc gaaccccacc aggagggcga 1140
   cgagcgcctg ctaggccctc gccttattga ctgcagcagc tggcccgggg gtggcggcgg 1200
   ggtgaggttc gtaccggcac tgtcccggga caacccttgc agttgc 1246
<210> 54
   <211> 984
   <212> DNA
   <213> Homo sapiens
<400> 54
   acaaataaaa caccctctag cttcccctag actttgttta actggccggg tctccagaag 60
   gaacgctggg gatgggatgg gtggagagag ggagcggctc aaggacttta gtgaggagca 120
   ggcgagaagg agcacgttca ggcgtcaaga ccgatttctc cccctgcttc gggagacttt 180
   tgaacgctcg gagaggcccg gcatctcacc actttacttg gccgtagggg cctccggcac 240
   ggcaggaatg agggaggggg tccgattgga cagtgacggt ttggggccgt tcggctatgt 300
   tcagggacca tatggtttgg ggacagcccc agtagttagt aggggacggg tgcgttcgcc 360
   cagtccccgg atgcgtaggg aggcccagtg gcaggcagct gtcccaagca gcgggtgcgc 420
   gtccctgcgc gctgtgtgtt cattttgcag agccagcctt cggggaggtg aaccagctgg 480
   gaggagtgtt cgtgaacggg aggccgctgc ccaacgccat ccggcttcgc atcgtggaac 540
   tggcccaact gggcatccga ccgtgtgaca tcagccgcca gctacgggtc tcgcacggct 600
   gcgtcagcaa gatcctggcg cgatacaacg agacgggctc gatcttgcca ggagccatcg      660
   ggggcagcaa gccccgggtc actaccccca ccgtggtgaa acacatccgg acctacaagc      720
   agagagaccc cggcatcttc gcctgggaga tccgggaccg cctgctggcg gacggcgtgt 780
   gcgacaagta caatgtgccc tccgtgagct ccatcagccg cattctgcgc aacaagatcg 840
   gcaacttggc ccagcagggt cattacgact catacaagca gcaccagccg acgccgcagc 900
   cagcgctgcc ctacaaccac atctactcgt accccagccc tatcacggcg gcggccgcca 960
   aggtgcccac gccacccggg gtgc 984
<210> 55
   <211> 545
   <212> DNA
   <213> Homo sapiens
<400> 55
   aggaggcgca acgcgctgcc agggcggctt tatcctgccg ccacagggcg gggaccagcc 60
   cggcagccgg gtgtccagcg ccgctcacgt gcctcgcctg gagcttagct ctcagactcc 120
   gaagagggcg actgagactt gggcctggga gttggcttcg gggtacccaa ggcgacgaca 180
   gctgagttgt accacgaagc tcaggccgag gcctcctccc ttgtctggcc ttcgaatcca 240
   tactggcagc ctctcctctc aggcactccg cgggccgggc cactaggccc cctgctcctg 300
   gagctgcgct atgatccggg tcttgagatg cgcgcgattc tctctgaacc ggtggagagg 360
   aggctctgcc ccgcgcggag cgaggacagc ggcgcccgag cttcccgcgc ctctccaggg 420
   cccaatggca agaacagcct ccgaagtgcg cggatgacag gaaaagatct tcagttcttc 480
   tgccgctaga gaagtgcggg atacaagcct ctattggatc cacaacctgg agtcctgcct 540
   tcgga 545
<210> 56
   <211> 1533
   <212> DNA
   <213> Homo sapiens
<400> 56
   atctgcgtgc ccttttctgg gcgagccctg ggagatccag ggagaactgg gcgctccaga 60
   tggtgtatgt ctgtaccttc acagcaaggc ttcccttgga tttgaggctt cctattttgt 120
   ctgggatcgg ggtttctcct tgtcccagtg gcagccccgc gttgcgggtt ccgggcgctg 180
   cgcggagccc aaggctgcat ggcagtgtgc agcgcccgcc agtcgggctg gtgggttgtg 240
   cactccgtcg gcagctgcag aaaggtggga gtgcaggtct tgcctttcct caccgggcgg 300
   ttggcttcca gcaccgaggc tgacctatcg tggcaagttt gcggcccccg cagatcccca 360
   gtggagaaag agggctcttc cgatgcgatc gagtgtgcgc ctccccgcaa agcaatgcag 420
   accctaaatc actcaaggcc tggagctcca gtctcaaagg tggcagaaaa ggccagacct 480
   aactcgagca cctactgcct tctgcttgcc ccgcagagcc ttcagggact gactgggacg 540
   cccctggtgg cgggcagtcc catccgccat gagaacgccg tgcagggcag cgcagtggag 600
   gtgcagacgt accagccgcc gtggaaggcg ctcagcgagt ttgccctcca gagcgacctg 660
   gaccaacccg ccttccaaca gctggtgagg ccctgcccta cccgccccga cctcgggact 720
   ctgcgggttg gggatttagc cacttagcct ggcagagagg ggagggggtg gccttgggct 780
   gaggggctgg gtacagccct aggcggtggg ggagggggaa cagtggcggg ctctgaaacc 840
   tcacctcggc ccattacgcg ccctaaacca ggtctccctg gattaaagtg ctcacaagag 900
   aggtcgcagg attaaccaac ccgctccccc gccctaatcc ccccctcgtg cgcctgggga 960
   cctggcctcc ttctccgcag ggcttgctct cagctggcgg ccggtcccca agggacactt 1020
   tccgactcgg agcacgcggc cctggagcac cagctcgcgt gcctcttcac ctgcctcttc 1080
   ccggtgtttc cgccgcccca ggtctccttc tccgagtccg gctccctagg caactcctcc 1140
   ggcagcgacg tgacctccct gtcctcgcag ctcccggaca cccccaacag tatggtgccg 1200
   agtcccgtgg agacgtgagg gggacccctc cctgccagcc cgcggacctc gcatgctccc 1260
   tgcatgagac tcacccatgc tcaggccatt ccagttccga aagctctctc gccttcgtaa 1320
   ttattctatt gttatttatg agagagtacc gagagacacg gtctggacag cccaaggcgc 1380
   caggatgcaa cctgctttca ccagactgca gacccctgct ccgaggactc ttagtttttc 1440
   aaaaccagaa tctgggactt accagggtta gctctgccct ctcctctcct ctctacgtgg 1500
   ccgccgctct gtctctccac gccccacctg tgt 1533
<210> 57
   <211> 702
   <212> DNA
   <213> Homo sapiens
<400> 57
   aggtctcttc agactgccca ttctccgggc ctcgctgaat gcgggggctc tatccacagc        60
   gcgcggggcc gagctcaggc aggctggggc gaagatctga ttctttcctt cccgccgcca       120
   aaccgaatta atcagtttct tcaacctgag ttactaagaa agaaaggtcc ttccaaataa       180
   aactgaaaat cactgcgaat gacaatacta tactacaagt tcgttttggg gccggtgggt       240
   gggatggagg agaaagggca cggataatcc cggagggccg cggagtgagg aggactatgg       300
   tcgcggtgga atctctgttc cgctggcaca tccgcgcagg tgcggctctg agtgctggct       360
   cggggttaca gacctcggca tccggctgca ggggcagaca gagacctcct ctgctagggc       420
   gtgcggtagg catcgtatgg agcccagaga ctgccgagag cactgcgcac tcaccaagtg       480
   ttaggggtgc ccgtgataga ccgccaggga aggggctggt tcggagggaa ttcccgctac       540
   cgggaaggtc ggaactcggg gtgatcaaac aaggaatgca tctcacctcc gtgggtgctt       600
   gtgctgcgca aggaattatt accggagcgg ttgcgatggc ctttgcccgg cgacccaaga       660
   agagtaagca aactaccgtc cacccagcgg atcaggtcca at                          702
<210> 58
   <211> 3180
   <212> DNA
   <213> Homo sapiens
<400> 58
   gatgtcctgt ttctagcagc ctccagagcc aagctaggcg agaggcgtag gaggcagaga 60
   gagcgggcgc gggaggccag ggtccgcctg ggggcctgag gggacttcgt ggggtcccgg 120
   gagtggccta gaaacaggga gctgggaggg ccgggaagag cttgaggctg agcgggggac 180
   gaacgggcag cgcaaagggg agatgaacgg aatggccgag gagccacgca ttcgccttgt 240
   gtccgcggac ccttgttccc gacaggcgac caagccaagg ccctccggac tgacgcggcc 300
   tgagcagcag cgagtgtgaa gtttggcacc tccggcggcg agacggcgcg ttctggcgcg 360
   cggctcctgc gtccggctgg tggagctgct gcgccctatg cggcctgccg agggcgccgc 420
   cgagggcccg cgagctccgt ggggtcgggg tggggggacc cgggagcgga cagcgcggcc 480
   cgaggggcag gggcaggggc gcgcctggcc tggggtgtgt ctgggccccg gctccgggct 540
   cttgaaggac cgcgagcagg aggcttgcgc aatcccttgg ctgagcgtcc acggagaaag 600
   aaaaagagca aaagcagagc gagagtggag cgagggatgg gggcgggcaa agagccatcc 660
   gggtctccac caccgccctg acacgcgacc cggctgtctg ttggggaccg cacgggggct 720
   cgggcgagca ggggagggag gagcctgcgc ggggctcgtg ttcgcccagg aatcccggag 780
   aagctcgaag acggtctggt gttgaacgca cacgtggact ccatttcatt accaccttgc 840
   agctcttgcg ccacggaggc tgctgctgcc cggcggctgc tacccaccga gacccacgtg 900
   gcccctcccc aggggtgtag gggtgacggt tgtcttctgg tgacagcaga ggtgttgggt 960
   ttgcgactga tctctaacga gcttgaggcg caaacctagg attccctgag tgttggggtg 1020
   cggcgggggg gcaagcaagg tgggacgacg cctgcctggt ttccctgact agttgcgggg 1080
   ggtgggggcc ggctctcagg ggccaccaga agctgggtgg gtgtacagga aatatttttt 1140
   ctcctgccgt gtttggcttt ttcctggcat ttttgcccag ggcgaagaac tgtcgcgcgg 1200
   ggcagctcca ccgcggaggg agaggggtcg cgaggctggc gcgggaagcg ctgtaggtgg 1260
   cagtcatccg tccacgccgc acaggccgtc tgcgccgtcg gaccatcggg aggtctgcag 1320
   caactttgtc ccggccagtc cccttgtccg ggaaggggct gagcttcccg acactctacc 1380
   ctccccctct tgaaaatccc ctggaaaatc tgtttgcaat gggtgtttcc gcggcgtcca 1440
   ggtctgggct gccgggggag gccgagcggc tgctgcagcc tccctgctgc caggggcgtc 1500
   ggactccgct tcgctcacta cgcccaggcc cctcaggggc ccacgctcag gacttcgggg 1560
   ccacacagca ggacccggtg ccccgacgac gagtttgcgc aggacccggg ctgggccagc 1620
   cgcggagctg gggaggaagg ggcgggggtc ggtgcagcgg atcttttctg ttgctgcctg 1680
   tgcggcggca ggaagcgtct tgaggctccc caagactacc tgaggggccg cccaagcact 1740
   tcagaagccc aaggagcccc cggccacccc cgctcctggc ctttttgcca acgactttga 1800
   aagtgaaatg cacaagcacc agcaattgac ttcccttccg tggttattta ttttgtcttt 1860
   gtggatggtg ggcagatggg gagagaggcc cctacctaac ctcggtggct ggtccctaga 1920
   ccacccctgc cagccggtgt ggggaggagc tcaggtccgc gggagagcga atgggcgcca 1980
   ggaggtggga cagaatcctg ggaaggtaca gcggacgccc tggaagctcc cctgatgccc 2040
   cagagggccc ttcctgggaa acctcccggg ggggtgcccc ataccatccc acccggctgt 2100
   cttggcccct cccagggagc cgcaggagaa actagcccta cacctgggat tcccagagcc 2160
   ttctgctggg gctcctgccc ccgacttcgg ataaccagct ccgcacaggt ccccgagaag 2220
   ggccgctggc ctgcttattt gatactgccc cctcccagac aggggctggt cgagcccctg 2280
   gttctgctgc cagactgaag ccttccagac gccacctcgg tttgggcccc cagggccctc 2340
   aggggcccca ggagaggaga gctgctatct agctcagcca caggctcgct cctggtgggg 2400
   gccaggctga aggagtggac cctggagagg tcgggaacct tttaacagcc gtgggctgga 2460
   gggtggctac taagtgttcg gtctgggaag aggcatgacc cgcaccatcc cggggaaata 2520
   aacgacttct taagggaatc ttctcgctga gcgggtgctc tgggccagga gattgccacc 2580
   gccagcccac ggaacccaga tttgggctct gccttgagcg ggccgcctgt ggcttcccgg 2640
   gtcgctcccc cgactcagaa agctctcaag ttggtatcgt tttcccggcc ctcggaggtg 2700
   gattgcagat caccgagagg ggatttacca gtaaccacta cagaatctac ccgggcttta 2760
   acaagcgctc atttctctcc cttgtcctta gaaaaacttc gcgctggcgt tgatcatatc 2820
   gtacttgtag cggcagctta ggggcagcgg aactggtggg gttgtgcgtg cagggggagg 2880
   ctgtgaggga gccctgcact ccgcccctcc acccttctgg aggagtggct ttgtttctaa 2940
   gggtgccccc ccaacccccg ggtccccact tcaatgtttc tgctctttgt cccaccgccc 3000
   gtgaaagctc ggctttcatt tggtcggcga agcctccgac gcccccgagt cccaccctag 3060
   cgggccgcgc ggcactgcag ccgggggttc ctgcggactg gcccgacagg gtgcgcggac 3120
   ggggacgcgg gccccgagca ccgcgacgcc agggtccttt ggcagggccc aagcacccct 3180
<210> 59
   <211> 1038
   <212> DNA
   <213> Homo sapiens
<400> 59
   tggcggccgg cgggcacagc cggctcattg ttctgcacta caaccactcg ggccggctgg 60
   ccgggcgcgg ggggccggag gatggcggcc tgggggccct gcgggggctg tcggtggccg 120
   ccagctgcct ggtggtgctg gagaacttgc tggtgctggc ggccatcacc agccacatgc 180
   ggtcgcgacg ctgggtctac tattgcctgg tgaacatcac gctgagtgac ctgctcacgg 240
   gcgcggccta cctggccaac gtgctgctgt cgggggcccg caccttccgt ctggcgcccg 300
   cccagtggtt cctacgggag ggcctgctct tcaccgccct ggccgcctcc accttcagcc 360
   tgctcttcac tgcaggggag cgctttgcca ccatggtgcg gccggtggcc gagagcgggg 420
   ccaccaagac cagccgcgtc tacggcttca tcggcctctg ctggctgctg gccgcgctgc 480
   tggggatgct gcctttgctg ggctggaact gcctgtgcgc ctttgaccgc tgctccagcc 540
   ttctgcccct ctactccaag cgctacatcc tcttctgcct ggtgatcttc gccggcgtcc 600
   tggccaccat catgggcctc tatggggcca tcttccgcct ggtgcaggcc agcgggcaga 660
   aggccccacg cccagcggcc cgccgcaagg cccgccgcct gctgaagacg gtgctgatga 720
   tcctgctggc cttcctggtg tgctggggcc cactcttcgg gctgctgctg gccgacgtct 780
   ttggctccaa cctctgggcc caggagtacc tgcggggcat ggactggatc ctggccctgg 840
   ccgtcctcaa ctcggcggtc aaccccatca tctactcctt ccgcagcagg gaggtgtgca 900
   gagccgtgct cagcttcctc tgctgcgggt gtctccggct gggcatgcga gggcccgggg 960
   actgcctggc ccgggccgtc gaggctcact ccggagcttc caccaccgac agctctctga 1020
   ggccaaggga cagctttc 1038
<210> 60
   <211> 374
   <212> DNA
   <213> Homo sapiens
<400> 60
   tagtaaggca ccgaggggtg gctcctctcc ctgcagcggc tgtcgcttac catcctgtag 60
   accgtgacct cctcacacag cgccaggacg aggatcgcgg tgagccagca ggtgactgcg 120
   atcctggagc tggtcgcagc aggccatcct gcacgcggtg gaggcgcccc ctgcaggccg 180
   cagcgcatcc ccagcttctg gacgcactgt gagcggttat gcagcagcac gctcatatga 240
   gatgccccgc agggtgctat gcaggcccac gtccccacaa agcccatggc aggcgcccgg 300
   gtgccggagc acgcacttgg ccccatggat ctctgtgccc agggctcagc caggcatctg 360
   gccgctaaag gttt 374
<210> 61
   <211> 246
   <212> DNA
   <213> Homo sapiens
<400> 61
   tctcatctga gcgctgtctt tcaccagagc tctgtaggac tgaggcagta gcgctggccc 60
   gcctgcgaga gcccgaccgt ggacgatgcg tcgcgccctt cccatcgcgg cctgggcggg 120
   cccgcctgcc ctcggctgag cccggtttcc ctaccccggg gcacctcccc tcgcccgcac 180
   ccggccccag tccctcccag gcttgcgggt agagcctgtc tttgcccaga aggccgtctc 240
   caagct 246
<210> 62
   <211> 222
   <212> DNA
   <213> Homo sapiens
<400> 62
   cagtccccga ggccctcccc ggtgactcta accagggatt tcagcgcgcg gcgcggggct 60
   gcccccaggc gtgacctcac ccgtgctctc tccctgcaga atctcctacg acccggcgag 120
   gtaccccagg tacctgcctg aagcctactg cctgtgccgg ggctgcctga ccgggctgtt 180
   cggcgaggag gacgtgcgct tccgcagcgc ccctgtctac at 222
<210> 63
   <211> 2209
   <212> DNA
   <213> Homo sapiens
<400> 63
   agagagacat tttccacgga ggccgagttg tggcgcttgg ggttgtgggc gaaggacggg        60
   gacacggggg tgaccgtcgt ggtggaggag aaggtctcgg aactgtggcg gcggcggccc       120
   ccctgcgggt ctgcgcggat gaccttggcg ccgcggtggg ggtccggggg ctggctggcc       180
   tgcaggaagg cctcgactcc cgacacctgc tccatgaggc tcagcctctt cacgcccgac       240
   gtcgggctgg ccacgcgggc agcttctggc ttcggggggg ccgcgatagg ttgcggcggg       300
   gtggcggcca caccaaaagc catctcggtg tagtcaccat tgtccccggt gtccgaggac       360
   aacgatgagg cggcgcccgg gccctgggcg gtggcaacgg ccgaggcggg gggcaggcgg       420
   tacagctccc ccggggccgg cggcggtggc ggcggctgca gagacgacga cggggacgcg       480
   gacggacgcg ggggcaacgg cggatacggg gaggaggcct cgggggacag gaggccgtcc        540
   aaggagccca cggggtggcc gctcggggcg cccggcttag gagacttggg ggagctgaag 600
   tcgaggttca tgtagtcgga gagcggagac cgctgccggc tgtcgctgct ggtgcccggg 660
   gtgcctgagc ccagcgacga ggccgggctg ctggcggaca agagcgagga ggacgaggcc 720
   gccgacgcca gcaggggagg cgcgggcggc gacaggcggg ccccgggctc gccaaagtcg 780
   atgttgatgt actcgccggg gctcttgggc tccggtggca gtgggtactc gtgcatgctg 840
   ggcaggctgg gcagcccctc cagggacagg cgcgtgggcc tcaccgcccg gccgcgctgg 900
   cccaagaagc cctccgggcg gccgccgcta ggccgcacgg gcgaaggcac tacagggtga 960
   gggggctgcg tggggccggc cccgaaggcg ctggccgcct ggctgggccc tggcgtggcc 1020
   tgaggctcca gacgctcctc ctccaggatg cgccccacgg gggagctcat gagcacgtac 1080
   tggtcgctgt ccccgccaca ggtgtagggg gccttgtagg agcggggcaa ggagctgtag 1140
   cagcagccgg gaacgcccct gagcggctcc ccgccggggt gcagggctgc ggagaagaag 1200
   tcgggcgggg tgcccgtggt gaccgcgtcg ctgggggaca cgttgaggta gtccccgttg 1260
   ggcagcagct tgccatctgc atgctccatg gacagcttgg aaccgcacca catgcgcatg 1320
   tacccactgt cctcggggga gctctcggcg ggcgagctgg ccttgtagcc gcccccgctc 1380
   gccgggaatg tcctgcccgc cgcagaggtg ggtgctggcc ccgcaggccc cgcagaaggc 1440
   acggcggcgg cggcggcggc ggcggccctg ggctgcaaga tctgcttggg ggcggacacg 1500
   ctggcggggc tcatgggcat gtagtcgtcg ctcctgcagc tgccgctccc actgcccgcg 1560
   agggccgcgc cgggcgtcat gggcatgtag ccgtcgtctg cccccaggtt gctgctggag 1620
   ctcctgtggg agccgatctc gatgtctccg tagtcctctg ggtaggggtg gtaggccacc 1680
   ttgggagagg acgcggggca ggacgggcag aggcggcccg cgctgcccga gaaggtggcc 1740
   cgcatcaggg tgtattcatc cagcgaggca gaggagggct ggggcaccgg ccgctgccgg 1800
   gctggcgtgg tcagggagta ggtcctcttg cgcagccctc ggtccaggtc ctgggccgcg 1860
   tcccccgaga cccggcggta ggagcggcca cagtggctca ggggcctgtc catggtcatg 1920
   tacccgtaga actcaccgcc gccgccgccg tctcgggccg ggggcgtctc cgcgatggac 1980
   tcgggcgtgt tgcttcggtg gctgcagaag gcgcgcaggt cgcctgggct ggagccgtac 2040
   tcgtccaggg acatgaagcc ggggtcgctg ggggagcccg aggcggaggc gctgccgctg 2100
   gagggccgct ggccggggcc gtggtgcagc ggatgcggca gaggcgggtg cgggccgggc 2160
   ggcggcgggt aggagcccga gccgtggccg ctgctggacg acagggagc 2209
<210> 64
   <211> 149
   <212> DNA
   <213> Homo sapiens
<400> 64
   taacctaaag aatgaagtca tgccccggcc tgcacccggg aaactgcaca cagcgaaaga      60
   tcgccactga gataaagagc tgaaagctat tccccaattc agctgtttca gccgtgcggt     120
   ctcacaatgg gctcacagac ggcagcatc                                       149
<210> 65
   <211> 832
   <212> DNA
   <213> Homo sapiens
<400> 65
   gtttccacaa tccacctcgt agctggggcg tgccgcttgc ctcggcttgt cccggcagaa      60
   cactcttacc tttaatggcg actgaaaagt tgccacgagt tcctgatcat tgtggtaggt     120
   gctgcgtgaa gctgagacgt gcgtgagcca catcccaggg ggctttgagc ccccaccgcg    180
   gcggcggctg aggggaggct tgtcgtactc gcacaggagg acacagggct gcagtgttca     240
   ctccagggcc tcttatcatt gggatctgag gaattttccg agaggaagtg cgaattaaca     300
   atgatgaaag gtttgtgagt gagtgacagg cacgttctat tgagcactgc atggggcatt     360
   atgtgccacc agagacgggg gcagaggtca agagccctcg agggctggga gagttcggag     420
   gatagaagtc atcagagcac aatgaagcca gaccctgcag ccgccttccc cttcgggggc     480
   ttccttagaa tgcagcattg cggggactga gctgtcccag gtgaaggggg gccgtcacgg     540
   tgtgtggacg cccctcggct cagccctcta agagactcgg cagccaggat gggctcaagg     600
   catgagccct caaaggaggt taggaaggag cgagggagaa aagatatgct tgtgtgacgt     660
   cctggccgaa gtgagaacaa ttgtatcaga taatgagtca tgtcccattg aggggtgccg     720
   acaaggactc gggaggaggc cacggagccc tgtactgagg agacgcccac agggagcctc     780
   gggggcccag cgtcccggga tcactggatg gtaaagccgc cctgcctggc gt             832
<210> 66
   <211> 256
   <212> DNA
   <213> Homo sapiens
<400> 66
   tccagctgca gcgagggcgg ccaggccccc ttctccgacc tgcaggggta gcgcggcctc      60
   ggcgccggag acccgcgcgc tgtctggggc tgcggtggcg tggggagggc gcggcccccg     120
   gacgccccga ggaaggggca cctcaccgcc cccacccaga gcgcctggcc gtgcgggctg     180
   cagaggaccc ctccggggca gaggcaggtt ccacggaaga ccccggcccg ctggggcttc     240
   cccggagact ccagag                                                     256
<210> 67
   <211> 184
   <212> DNA
   <213> Homo sapiens
<400> 67
   acttactgct tccaaaagcg ctgggcacag ccttatatga ctgaccccgc ccccgagtcc     60
   caggccgccc catgcaaccg cccaaccgcc caaccgccac tccaaaggtc accaaccact    120
   gctccaggcc acgggctgcc tctccccacg gctctagggc ccttcccctc caccgcaggc    180
   tgac                                                                 184
<210> 68
   <211> 118
   <212> DNA
   <213> Homo sapiens
<400> 68
   tgccacaccc aggtaccgcc cgcccgcgcg agagccgggc aggtgggccg cggatgctcc     60
   cagaggccgg cccagcagag cgatggactt ggacaggcta agatggaagt gacctgag      118
<210> 69
   <211> 1534
   <212> DNA
   <213> Homo sapiens
<400> 69
   tcgccagcgc agcgctggtc catgcaggtg ccacccgagg tgagcgcgga ggcaggcgac      60
   gcggcagtgc tgccctgcac cttcacgcac ccgcaccgcc actacgacgg gccgctgacg     120
   gccatctggc gcgcgggcga gccctatgcg ggcccgcagg tgttccgctg cgctgcggcg     180
   cggggcagcg agctctgcca gacggcgctg agcctgcacg gccgcttccg gctgctgggc     240
   aacccgcgcc gcaacgacct ctcgctgcgc gtcgagcgcc tcgccctggc tgacgaccgc     300
   cgctacttct gccgcgtcga gttcgccggc gacgtccatg accgctacga gagccgccac     360
   ggcgtccggc tgcacgtgac aggcgaggcg gcgtgggagc gggtccccgg cctcccttcc     420
   cgccctcccg cctgccccgc cccaagggct acgtgggtgc caggcgctgt gctgagccag     480
   gaagggcaac gagacccagc cctctcctct accccaggga tctcacacct gggggtagtt     540
   taggaccacc tgggagcttg acacaaatgc agaatccagg tcccaggaag ggctgaggtg     600
   ggcccgggaa taggcattgc cgtgactctc gtagagtgac tgtccccagt ggctctcaga     660
   cgaagaggcg agaaagacaa gtgaatggca atcctaaata tgccaagagg tgcaatgtgg     720
   tgtgtgctac cagcccggaa agacactcgc agcccctcta cccaggggtg cacagacagc     780
   ccaccaagta gtgcctagca ctttgccaga ccctgatata caaagatgcc tgaaccaggg     840
   tcccgtccct agagcagtgg ctctccactc tagcccccac cctgctctgc gacaataatg     900
   gccacttagc atttgctagg gagccgggac ctagtccaag cacccacaag catgaatttg     960
   ccaaatcttt tcagcaacct cttaaggcaa ctgctatcat gatcctcact ttacacatgg    1020
   agaagcagaa gcagagatga tagaatcttt cgcccaaggc cacatctgta ttgggacggg    1080
   ggcagcctgg cacccaagtg cccattcctc ccttctgacc agcccccacc cctccggctc    1140
   tggcgtccaa agggctaagg ggaggggtgc ccttgtgaca gtcacccgcc ttctcccctg    1200
   cagccgcgcc gcggatcgtc aacatctcgg tgctgcccag tccggctcac gccttccgcg    1260
   cgctctgcac tgccgaaggg gagccgccgc ccgccctcgc ctggtccggc ccggccctgg    1320
   gcaacagctt ggcagccgtg cggagcccgc gtgagggtca cggccaccta gtgaccgccg    1380
   aactgcccgc actgacccat gacggccgct acacgtgtac ggccgccaac agcctgggcc    1440
   gctccgaggc cagcgtctac ctgttccgct tccatggcgc cagcggggcc tcgacggtcg    1500
   ccctcctgct cggcgctctc ggcttcaagg cgct                                1534
<210> 70
   <211> 269
   <212> DNA
   <213> Homo sapiens
<400> 70
   atgaacttca agggcgacat catcgtggtc tacgtcagcc agacctcgca ggagggcgcg 60
   gcggcggctg cggagcccat gggccgcccg gtgcaggagg agaccctggc gcgccgagac 120
   tccttcgcgg ggaacggccc gcgcttcccg gacccgtgcg gcggccccga ggggctgcgg 180
   gagccggaga aggcctcgag gccggtgcag gagcaaggcg gggccaaggc ttgagcgccc 240
   cccatggctg ggagcccgaa gctcggagc 269
<210> 71
   <211> 282
   <212> DNA
   <213> Homo sapiens
<400> 71
   tcagtgttat gtggggagcg ctagatcgtg cacacagtag gcgtcaggaa gtgttttccc 60
   cagtaattta ttctccatgg tactttgcta aagtcatgaa ataactcaga ttttgttttc 120
   caaggaagga gaaaggccca gaatttaaga gcaggcagac acacaaccgg gcacccccag 180
   accctggccc ttccagcagt caggaattga cttgccttcc aaagccccag cccggagctt 240
   gaggaacgga ctttcctgcg cagggggatc ggggcgcact cg 282
<210> 72
   <211> 142
   <212> DNA
   <213> Homo sapiens
<400> 72
   gtggaaacac aacctgcctt ccattgtctg cgcctccaaa acacaccccc cgcgcatccg 60
   tgaagctgtg tgtttctgtg ttactacagg ggccggctgt ggaaatccca cgctccagac 120
   cgcgtgccgg gcaggcccag cc 142
<210> 73
   <211> 741
   <212> DNA
   <213> Homo sapiens
<400> 73
   tccacacctc gggcagtcac taggaaaagg gtcgccaact gaaaggcctg caggaaccag        60
   gatgatacct gcgtcagtcc cgcggctgct gcgagtgcgc gctctcctgc cagggggacc      120
   tcagaccctc ctttacagca caccgagggc cctgcagaca cgcgagcggg ccttcagttt       180
   gcaaaccctg aaagcgggcg cggtccacca ggacgatctg gcagggctct gggtgaggag       240
   gccgcgtctt tatttggggt cctcgggcag ccacgttgca gctctggggg aagactgctt       300
   aaggaacccg ctctgaactg cgcgctggtg tcctctccgg ccctcgcttc cccgaccccg       360
   cacaggctaa cgggagacgc gcaggcccac cccaccggct ggagaccccg gcacggcccg       420
   catccgccag gattgaagca gctggcttgg acgcgcgcag ttttcctttg gcgacattgc       480
   agcgtcggtg cggccacaat ccgtccactg gttgtgggaa cggttggagg tcccccaaga       540
   aggagacacg cagagctctc cagaaccgcc tacatgcgca tggggcccaa acagcctccc       600
   aaggagcacc caggtccatg cacccgagcc caaaatcaca gacccgctac gggcttttgc       660
   acatcagctc caaacacctg agtccacgtg cacaggctct cgcacagggg actcacgcac       720
   ctgagttcgc gctcacagat c                                                 741
<210> 74
   <211> 4498
   <212> DNA
   <213> Homo sapiens
<400> 74
   ctgccctcgc ggatctcccc cggcctcgcc ggcctccgcc tgtcctccca ccaccctctc        60
   cgggccagta ccttgaaagc gatgggcagg gtcttgttgc agcgccagtg cgtaggcagc       120
   acggagcaga ggaagttggg gctgtcggtg cgcaccagct cgcccgggtg gtcggccagc       180
   acctccacca tgctgcggtc gccgctcctc agcttgccgg ccagggcagc gccggcgtcc       240
   ggggcgccca gcggcaacgc ctcgctcatc ttgcctgggc tcagcgcggt ggaaggcggc       300
   gtgaagcggc ggctcgtgct ggcatctacg gggatacgca tcacaacaag ccgattgagt       360
   taggaccctg caaacagctc ctaccagacg gcgacagggg cgcggatctt cagcaagcag       420
   ctcccgggag accaacatac acgttcaggg gcctttatta ctgcgggggg tggggggggg       480
   cgggggtggt taggggagga gggagactaa gttactaaca gtccaggagg ggaaaacgtt       540
   ctggttctgc ggatcggcct ctgacccagg atgggctcct agcaaccgat tgcttagtgc       600
   attaaaaagt ggagactatc ttccacgaat cttgcttgca gaggttaagt tctgtctttg       660
   gctgttagaa aagttcctga aggcaaaatt ctcatacact tcctaaaata tttatgcgaa 720
   gagtaaaacg atcagcaaac acattatttg gaagttccag tagttaatgc ctgtcagttt 780
   tttgcaggtg agttttgtct aaagtcccaa cagaacacaa ttatctcccg taacaaggcc 840
   acttttatca tgcaaaactg gcttcagtcc cgaaaagcaa gagctgagac ttccaaaggt 900
   agtgctacta atgtatgtgc acgtatatat aaatatatac atatgctcta cttcataaaa 960
   tatttacaat acaatctgtg gagaatttaa acacaacaga aatccattaa tgtacgctgc 1020
   agattttttt aagtagcctt gaaaatcagc ttcagtagtt ggagcagtgc tgagctagaa 1080
   gtacttgtca tgttctctgt tctctcaatg aattctgtca aaacgctcag tgcagaaaat 1140
   tcagcgtttc agagatcttc agctaatctt aaaacaacaa tcataagaag gcccagtcga 1200
   tgacactcag ggttctacag ctctcccaca tctgtgaact cgggtttggg gatgttggtt 1260
   aagtttgtgg ctggtcctct ggtttgttgg gagttgagca gccgcagagt cacacacatg 1320
   caaacacgca ctcttcggaa ggcagccact gtctacatca gctgggtgac tcagccctga 1380
   ctcgggcagc agcgagacga tactcctcca ccgtcgccca gcacccgccg gttagctgct 1440
   ccgaggcacg aacacccacg agcgccgcgt aaccgcagca ggtggagcgg gccttgaggg 1500
   agggctccgc ggcgcagatc gaaacagatc gggcggctcg ggttacacac gcacgcacat 1560
   cctgccacgc acactgccac gcacacgcaa cttcacggct cgcctcggac cacagagcac 1620
   tttctccccc tgttgtaaaa ggaaaacaat tggggaaaag ttcgcagcca ggaaagaagt 1680
   tgaaaacatc cagccaagaa gccagttaat tcaaaaggaa gaaaggggaa aaacaaaaaa 1740
   aaacaacaaa aaaaggaagg tccaacgcag gccaaggaga agcagcagag gttgacttcc 1800
   ttctggcgtc cctaggagcc ccggaaagaa gtgcctggcg gcgcagggcc gggcagcgtg 1860
   gtgccctggc tgggtccggc cgcggggcgc ccgtcccgcc cgcgcccgct ggctctatga 1920
   atgagagtgc ctggaaatga acgtgctttt actgtaagcc cggccggagg aattccattc 1980
   cctcagctcg tttgcatagg ggcggccggc ggccaatcac aggcctttcc ggtatcagcc 2040
   agggcgcggc tcgccgccgc cggctcctgg aattggcccg cgcgcccccg ccgccgcgcc 2100
   gcgcgctact gtacgcagcc cgggcgggga gtcggaggcc acccccgcgc cccgcatcca 2160
   agcctgcatg ctggcccggg gccccgcccg cgtgcggacc cctttccgca gccacacgca 2220
   ggcttgtgcg gctccgcgag tggccacggt ccggagacct ggaaaaagaa agcaggcccc 2280
   gccggcccga ggaggacccg gccggcgcgc cgcacccgga gaggcccggc cccgcgagcc 2340
   gctgcaggca ggcgcagtgg ccgccacgag gctcccgaac cgggctgcag cccgcggacg 2400
   gccccagatc ctgcgcggcc gcccagggcc aggcctccgc ttccagggcg ggggtgcgat 2460
   ttggccgcgg ggcccggggg agccactccg cgctcctgca ccgtccggct ggcagctgcg 2520
   gcgaagcggc gctgattcct tgcatgaggc cggacggcgt ccgcgcgtgc cgtttgctct 2580
   cagcgtcttc ccttgggtcg gtttctgtaa tgggtgtttt ttaccgctgc gcccgggccg 2640
   cggctcgatc cctccgcgcg tctcacttgc tgcgtgcgtc agcggccagc gaagagtttc 2700
   ctagtcagga aagaccccaa gaacgcgcgg ctggaaggaa agttgaaagc agccacgcgg 2760
   cttgctcccg ggccttgtag cgccggcacc cgcagcagcc ggacagcctg cccgggcccc 2820
   gcgtctcccc tccggctccc cggaagcggc ccccgctcct ctccccgccc ccgtgcgctc 2880
   gagcggcccc aggtgcggaa cccaccccgg cttcgcgtgc gggcggccgc ttccccctgc 2940
   gccggtcccc gcggtgctgc gggcattttc gcggagctcg gagggccccg cccccggtcc 3000
   ggcgtgcgct gccaactccg accccgcccg gcggggctcc ctcccagcgg aggctgctcc 3060
   cgtcaccatg agtccctcca cgccctccct gccgggccct gcacctcccg gggcctctca 3120
   tccaccccgg ggctgcaacc cagtccccgg atcccggccc cgttccaccg cgggctgctt 3180
   tgtggtcccc gcggagcccc tcaattaagc tccccggcgc gggggtccct cgccgacctc 3240
   acggggcccc tgacgcccgc tcctccctcc cccagggcta gggtgctgtg gccgctgccg 3300
   cgcagggact gtccccgggc gttgccgcgg gcccggacgc aggagggggc cggggttgac 3360
   tggcgtggag gcctttcccg ggcgggcccg gactgcgcgg agctgtcggg acgcgccgcg 3420
   ggctctggcg gacgccaggg ggcagcagcc gccctccctg gacgccgcgc gcagtccccg 3480
   gagctcccgg aacgcccccg acggcgcggg gctgtgcggc ccgcctcgtg gccttcgggt 3540
   cgcccgggaa gaactagcgt tcgaggataa aagacaggaa gccgccccag agcccacttg 3600
   agctggaacg gccaaggcgc gtttccgagg ttccaatata gagtcgcagc cggccaggtg 3660
   gggactctcg gaccaggcct ccccgctgtg cggcccggtc ggggtctctt cccgaagccc 3720
   ctgttcctgg ggcttgactc gggccgctct tggctatctg tgcttcagga gcccgggctt 3780
   ccggggggct aaggcgggcg gcccgcggcc tcaaccctct ccgcctccgc tccccctggg 3840
   cactgccagc acccgagttc agttttgttt taatggacct ggggtctcgg aaagaaaact 3900
   tactacattt ttcttttaaa atgatttttt taagcctaat tccagttgta aatccccccc 3960
   tccccccgcc caaacgtcca ctttctaact ctgtccctga gaagagtgca tcgcgcgcgc 4020
   ccgcccgccc gcaggggccg cagcgccttt gcctgcgggt tcggacgcgg cccgctctag 4080
   aggcaagttc tgggcaaggg aaaccttttc gcctggtctc caatgcattt ccccgagatc 4140
   ccacccaggg ctcctggggc cacccccacg tgcatccccc ggaacccccg agatgcggga 4200
   gggagcacga gggtgtggcg gctccaaaag taggcttttg actccagggg aaatagcaga 4260
   ctcgggtgat ttgcccctcg gaaaggtcca gggaggctcc tctgggtctc gggccgcttg 4320
   cctaaaaccc taaaccccgc gacgggggct gcgagtcgga ctcgggctgc ggtctcccag 4380
   gagggagtca agttccttta tcgagtaagg aaagttggtc ccagccttgc atgcaccgag 4440
   tttagccgtc agaggcagcg tcgtgggagc tgctcagcta ggagtttcaa ccgataaa 4498
<210> 75
   <211> 453
   <212> DNA
   <213> Homo sapiens
<400> 75
   ttcggaagtg agagttctct gagtcccgca cagagcgagt ctctgtcccc agcccccaag 60
   gcagctgccc tggtgggtga gtcaggccag gcccggagac ttcccgagag cgagggaggg 120
   acagcagcgc ctccatcaca gggaagtgtc cctgcgggag gccctggccc tgattgggcg 180
   ccggggcgga gcggcctttg ctctttgcgt ggtcgcgggg gtataacagc ggcgcgcgtg 240
   gctcgcagac cggggagacg ggcgggcgca cagccggcgc ggaggcccca cagccccgcc 300
   gggacccgag gccaagcgag gggctgccag tgtcccggga cccaccgcgt ccgccccagc 360
   cccgggtccc cgcgcccacc ccatggcgac ggacgcggcg ctacgccggc ttctgaggct 420
   gcaccgcacg gagatcgcgg tggccgtgga cag 453
<210> 76
   <211> 560
   <212> DNA
   <213> Homo sapiens
<400> 76
   acgcacactg ggggtgtgat ggaaaggggg acgcgatgga taggggtggg cgcacactgg 60
   gggacgcgac ggggaggggt gagcacacac tgggggtgtg atggagaggg cgacgcaata 120
   gggaggggtg ggcgcacacc agggacgcga tgatggggac gggtgggcgc acaccaggtg 180
   gcatgatggg gaggagtggg tacacaccat ggggggcgtg atggggaggc gtgggcgtac 240
   accggggggc gcgatgggga ggggtgggcg cacaccgggg gacgcgatgg aggcggtggg 300
   tgcacacggg gcgcgatggg tgggagtagg tgcacactga gggcacgatt ggggagacac 360
   gaaggagagg ggtgggcgca cactggggga cgcgatggcc gggacacgat gcggagaagt 420
   gggtgaatac cggggtcgcg atgggcgccc tggaaggacg gcagtgctgc tcacaggggc 480
   caggcccctc agagcgcgcc ccttgggggt aaccccagac gcttgttccc gagccgactc 540
   cgtgcactcg acacaggatc 560
<210> 77
   <211> 157
   <212> DNA
   <213> Homo sapiens
<400> 77
   ccacagggtg gggtgcgccc acctgccctg tccatgtggc cttgggcctg cgggggagag 60
   ggaatcagga cccacagggc gagccccctc cgtagcccgc ggcaccgact ggatctcagt 120
   gaacacccgt cagcccatcc agaggctaga aggggga 157
<210> 78
   <211> 114
   <212> DNA
   <213> Homo sapiens
<400> 78
   ttgaggtctc tgtgcatgct tgtgcgtacc ctggactttg ccgtgagggg tggccagtgc 60
   tctgggtgcc tttgccagac aactggtctg ccgggccgag cattcatgct ggtc 114
<210> 79
   <211> 104
   <212> DNA
   <213> Homo sapiens
<400> 79
   tgacgcgccc ctctccccgc agctccacct ggttgcgctc aacagccccc tgtcaggcgg 60
   catgcggggc atccgcgggg ccgacttcca gtgcttccag cagg 104
<210> 80
   <211> 659
   <212> DNA
   <213> Homo sapiens
<400> 80
   aacacactgt ctcgcactag gtgctcgcgg aagagcgcgg cgtcgatgct gcggctcagg 60
   ttgatgggcg atggcggccg cagatccagc tcgctcagcg atggcgccgg tcccacaccg 120
   ttgcgggaca gtcccgggcc accctggggt ccgcgaccca acgacgcagc cgagccccag 180
   gcgcctgaac tgggcgtggc cagctgccca ctctccgccg ggttgcggat gaggctcttg 240
   ctgatgtcca agctgcctgc accaacgttg ctgggccctg catagcagtt attgggtcgc 300
   tccggcacct cgctctttcc tgacggcgcc gggcacgcca gacgcatcag cttagcccag 360
   caagcgtgct ccgtgggcgg cctgggtctc gcggcagcca ccgcggccaa cgccagggcg 420
   agcgcccatg tcagctccag gaggcgcagc cagaagtgga caccccacca ggcccacgag 480
   aagcggccca cgcggcctgg gcccgggtac agccagagcg cagccgccag ctgcaagccg 540
   ctagccagca gccccagcgc gcccgccaca gccaacagcc gagggcccgg gctggcatcc 600
   cagccccgtg ggccgtccag caggcggcga cggcacaggc agagcgtgcc cagagccac 659
<210> 81
   <211> 813
   <212> DNA
   <213> Homo sapiens
<400> 81
   gtctgcacga agcccgcggc ggcctgcagg gggcccagcg actcgtccag ggaaccggtg      60
   cgcaggagca gccgggggcg cggcgcgccg gccgcccttg ggggactctg gggccggggg     120
   cgcagctcga tctgacgctt gggcactgtc cggggcctgg cgggcgcggc gccctcctcc     180
   agagccacct ccacacactc gaactgcgct ggggcggcag gacttggccc acggggccgc     240
   agctctaggt aggtggccca gcgggagcca ccatcgggga cctgggactg gcgtgggacc     300
   gcggcgggag acgctggccc cggcggcaag gggctgatga aggccggctc cgtgaactgt     360
   tgttgcgcct cgcgatcgtc tgcgccggag cagccgaaca ggggtccgac gccgaagatg     420
   acttccatct cccccgacgg cagcgtgcgc agctggggct ggggtggccg tgggccggaa     480
   cctgggcctc gcgggaaacc cgagccgggc ccgtgccgct ggcggctatt ctgggcgctg     540
   acggacaggc gaggctgcgc gcccgccccc cgcccaggag ccacccaggg ccaattcgct     600
   gggcctttcg cgtccggccc aacgtccggg ggctccggag aacctggagc cgtgtagtag     660
   gagcctgacg aaccggagga gtcctggcgc cgcgcggggg ccgtgggcag ctgcctcggg     720
   atcccaggca gggctggcgg ggcgagcgcg gtcagcatgg tggggccgga cgccgtgcac     780
   tatctccctc gcattcgcct ccgctggtgg cgc                                  813
<210> 82
   <211> 440
   <212> DNA
   <213> Homo sapiens
<400> 82
   ctggagagaa ctatacgggc tgtgggagtc accgggcgac tatcaccggg cctcctttcc      60
   acatcctcct ccgggaaggg accccgttcc gggcctcgac cggcgcagac tgggctgacc     120
   cactttcttg ggcccactga gtcacctcga aacctccagg ccggtagcgg ggaggagagg     180
   aggagcaggc gggggtgcca aggtgtgggc tgcgccctgg ttagggggcg agcccggctt     240
   gtttatgagg aggagcgcgg aggaggatcc agacacacag gcttgcgcgc ccagactcgc     300
   ccggccagcg gctggcggcc tccgacgtca ccaaaccggt tgggtgagag ggcagagagc     360
   agggggaagg gccgcagtcc cgcccgcgcc ccccggcacg caccgtacat cttgccctcg     420
   tctgacagga tgatcttccg                                                 440
<210> 83
   <211> 252
   <212> DNA
   <213> Homo sapiens
<400> 83
   gagtgcggag tgaaggggtg cactgggcac tcagcgcggc ccttgggagg cagggccgcc      60
   ccagcctgcc ctcctgtctg ggaaggccgt ccagaagcag gagccccggg gaaaacaact     120
   ggctggacgg ggcggccttc agtgtctctc ccagcctgag agtcgcttcc caccacctgg     180
   gcacgaacct gctctgcgat ctccggcaag ttcctgcgcc tcctgtcggt aaaatgcaga     240
   tcgtggcgtc tt                                                         252
<210> 84
   <211> 1539
   <212> DNA
   <213> Homo sapiens
<400> 84
   tcttctttcc gcccctaggg ggcacaagcg ggcatgtcca agcgcctagg agcccgtacc 60
   gctggggacc tccccttccg cgaaccccga gcgggtagac ccagagcaat ccgagtgtgg 120
   aaacaatgga gagggggcgt gttgagctgg ggtctccatg cctcgttggg gagagggagg 180
   tgagtttgtg tcttctggaa ggcgtggggg ctgtgccctc gtgggggtag gaagtgctcc 240
   cgtggggcgg ggtgcggatc ggagaggtga gtgggtgcgt ctgtccagcg gtccgcccgg 300
   tgtggtcgtg cccggcccgc gtggggatgg gggtgtctct cccgctgggc aactatacca 360
   gcgcaaccgg ggcgtcggcg cggcccacgc tagcggcgct gctccggcgg cgggggctgg 420
   gcgtggcggt gatgctgggc gtggtggccg cgctgggcgt ggtggccgcg ctgccgccct 480
   cacccgggca gccgtgctgg agaaggatgt cggcgcacag ctggcttcca gcctggcggg 540
   cgtagaacag cgccgtgcgg ccctgggcgt cacgggccgc cacgtccgcg ccgtactaga 600
   gggcggaaac ggccgcgtga ccgcgcgtcc ccagggcgcc cacacccggc gccgcctccc 660
   ccacatggcc aagcctactt ccggggtccc tctgggaatt tcgggctttc ccgcgccagg 720
   cgttttccga gatgaagcct caaagacccc ctttcctccc cccagctcac gtacccacag 780
   cagcagttgc gtgatgacga cgtgggcgag ctcggccgcc aggtggagtg gggagcgcag 840
   ctgtgggtcc tctacgctgg tgtcgagcgg cccgtgtcgc gcatgggcca aaagcaggag 900
   aacggtagcc acgtcctggg cctgcacggc ggcccacagc tggcggccca gcggctcctc 960
   cgaggtgctc agcggcgcca ggaacagtag ctgctcgtac ttggcgcgaa tccacgactc 1020
   gcgctcctcc ctgcaagacc agggatcaac ggaaaaggct ctagggaccc ccagccagga 1080
   cttctgcccc tacccacggg accgtctcag gttcgcacac cctcagcaac cctccccccg 1140
   ctctgttccc tcacgcttac cgcgaagagt cccgcgaggg cttggcacgg cctcgcgtgt 1200
   cgctttccca cacgcggttg gccgtgtcgt tgccaatagc cgtcagcacc agggtcagct 1260
   cccgtggcca gtcgtccaag tccagcgagc gaacgcggga caggtgtgtg cccaggttgc 1320
   ggtggatgcc agaacactcg atgcagatga gggcgcccag gttcaagctg gcccacgtgg 1380
   ggtctgcgga aggagcgtag aggtcggctc ccagccgggc agcacaggca ccccggcatt 1440
   cactacactc cctagcccct ccgctgcctc ctggcactca ctgggggccc cgcagtccac 1500
   gcagattgaa ttccccttgg cgttccggat cgcctggat 1539
<210> 85
   <211> 2648
   <212> DNA
   <213> Homo sapiens
<400> 85
   agccaggtcc agcccccgcg cctgacaccg gccggacgtt cccggggcgc cgcagctgcg 60
   gcgggaactc tgggatccgg agccatctgc tcccacccgc tccggagcca aaccccgggg 120
   gccgcctccg ctcccggacc cgcctcctct cccgggagtg tgagccgaac caagagtctc 180
   ctgcctatct cctccagtag gaaaatagta ataataatag acaccctgcc cccgtaaaaa 240
   acactacctt ccccgtaccg cctcccaagt ctcccggggt acggattgcc tttgcagcag 300
   ttccgcccca cctgactcac tccagggtca gccccgggtg ggtttcaatg cggctctggg 360
   gagggggtgg gcagtggggg aagtgaggct tcctatccgc cccctctcac ttcacattta 420
   aatattctgc acgttccagc ccccgcggac tcgcgtaccg cccaatccgc cttcaccgca 480
   cgaaaaacat cactagcctg ctctcagccc aggggacgac tagtccctgg cgagaagctg 540
   cctgcaaggt cactgtcatg ccacctgccc caagtgctca ggggaaactg aggcttcctc 600
   atccccttca ccttcaacgt cgctctaaac acggcaaagc cccgtttcca tgctcccaga 660
   gttcagctga ggctggaagt ggggtcctgg gcttctctgg gagcaatttt ctagtcactc 720
   tgatcaagga cgttactttc ccagaaagct ctgaggctga gtccctctga aatcaagtcc 780
   tttctcctgt cgcacaatgt agctactcgc cccgcttcag gactcctatt ctttgcccca 840
   atccttgaca gaggggtgag cttggttcat ccgcccaccc cagagaaaag cttccctagt 900
   ttcctggacc tcgctcctcc accccaagct gagcattcca ggtacccttc cctccctgtt 960
   ctcaagccct gactcaactc actaggggaa gcgcggagct cggcgcccag cagctccctg 1020
   gacccgctgc cagaagacag gctggggggt ccgggaaggg gcccggagcc aggaggccct 1080
   cctgtgctct tggtgaagat gccgctgata aacttgagca tcttgcggtc acgagtggat 1140
   gctcggcccc cctcccggcc ccgtttcagc cccggagctg gaggctccag agtgattgga 1200
   ggtgcaggcc cggggggctg cgcggaagca gcggtgacag cagtggctgg actcggagtt 1260
   ggtgggaggg ttagcggagg aggagagccg gcaggcggtc ccggatgcaa gtcactgttg 1320
   tccaaggtct tactcttgcc tttccgaggg gacaacttcc ctcgggctcc agccccagcc 1380
   ccgaccccac cagaggtcga agctgtagag ccccctcccc cggcggcggc ggcggtggcg 1440
   gcggcagaga ccgaagctcc agtcccggcg ctgctctttg accccttgac cctgggcttg 1500
   ccctcgcttt cgggccatga caggcggcta cccgcgccct tgcccccgcc ggctttggct 1560
   ccactcgtgg tcacggtctt gcaaggcttg ggagccggcg gaggaggcgc caccttgagc 1620
   ctccggctgc cggtgccagg gtgcggagag gatgagccag ggatgccgcc gcccgcccgg 1680
   ccttcgggct ccgggccgcc ccagctcggg ctgctgagca gggggcgccg ggaggaggtg 1740
   ggggcgcccc caggcttggg gtcggggctc agtcccccgg agagcggggg tcccggaggg 1800
   acggcccaga gggagaggcg gcggccggga gcgggggaga ctgggcgggc cggactggcc 1860
   ggagccgggg acagggctgg gggctccgcg cccccggtgc ccgcgctgct cgtgctgatc 1920
   cacagcgcat cctgccggtg gaagagacgt tcgtgccgct tcttgcccgg ctcctccgcg 1980
   cctcgggggc tgccaggatc cccagtctcg gagcctctgg caccggcggc gccggccgcg 2040
   gccgcagacg gagaaggcgg cggcggaggc accgactcga gcttaaccag ggtcagcgag 2100
   atgaggtagg tcgttgtccg gcgctgaagc gcgcccgcgc cccggctcat ggggcccgga 2160
   gacccccgag ctggggaggg gaggggactc ccccggactg cctcaggggg gcccggccat 2220
   ggggccgccc tgctcgctgc ccccagcccc cggaccccgc tgagcccccg gcccggctcc 2280
   gctgtcgccg ccgcctccgc cgcctccgct tgcgcccccc tcccatcaca tggggcgccc 2340
   cctccccatg ctccccgccc tgcgccccca ccctcttgga gccccgggac cttggtgctg 2400
   ctccagggag gcgcgccgga ccgtccaccc cggcctgggt gggggcgctg agatgggtgg 2460
   gggagggcgg ggaggacagt agtgggggca aatgggggag agagaggaaa agggagcaga 2520
   aaaggggacc ggaggctagg ggaaacgaac ctgtgcgggg gaggcagggg cggggaattg 2580
   ggactcaagg gacaggggcc gcggatgcgg tcggaaagag ggtctagagg agggtgggaa 2640
   gctagtgg 2648
<210> 86
   <211> 452
   <212> DNA
   <213> Homo sapiens
<400> 86
   aggagcgcaa ggcttgcagg gcatgctggg agagcgcagg gaacgctggg agagcgcggg 60
   aaatactggg attggctccc gagggctgtg aggagggcac gaggggacac tccgatgaag 120
   gcagggcacg cggggcgagc cgggagcgtc tcctgagggc agcgaggagg gagctgaggc 180
   acgcgggctc tcaatcgacg ccccacagag accaagaggc ctggccttgg ggggcagctg 240
   cttgaaggag gcagagcgga agcgagggag actgctggag gccctgccgc ccacccgccc 300
   tttcctcccc ctgaggagac gcctgacgca tctgcagtgc aggaggccgt gggcgttaga 360
   agtgttgctt ttccagtttg taagaccatt ttcctgattc tcttccccac ggttgcggag 420
   gagcaggtca gggccgccat gagggcagga tc 452
<210> 87
   <211> 232
   <212> DNA
   <213> Homo sapiens
<400> 87
   tcgaccgcta ctattatgaa aacagcgacc agcccattga cttaaccaag tccaagaaca 60
   agccgctggt gtccagcgtg gctgattcgg tggcatcacc tctgcgggag agcgcactca 120
   tggacatctc cgacatggtg aaaaacctca caggccgcct gacgcccaag tcctccacgc 180
   cctccacagt ttcagagaag tccgatgctg atggcagcag ctttgaggag gc 232
<210> 88
   <211> 221
   <212> DNA
   <213> Homo sapiens
<400> 88
   tgtgccgtcg cacacagacg ccctcaacgt cggagagctg tgagcggggc cgtgctcttg 60
   ggatgggagc ccccgggaga gctgcccgcc aacaccactc cgacgtgatc catgctggac 120
   ataaagtgct cttccctccg ctagtcatcg gccgagcggg cccctcgctc ctgggtgtaa 180
   gttctttctg tgcgtccttc tcccatctcc gtgcagttca g 221
<210> 89
   <211> 477
   <212> DNA
   <213> Homo sapiens
<400> 89
   ccatgcgccg ctgcgcgcgc gagttcgggc tgctgctgct gttcctctgc gtggccatgg 60
   cgctcttcgc gccactggtg cacctggccg agcgcgagct gggcgcgcgc cgcgacttct 120
   ccagcgtgcc cgccagctat tggtgggccg tcatctccat gaccaccgtg ggctacggcg 180
   acatggtccc gcgcagcctg cccgggcagg tggtggcgct cagcagcatc ctcagcggca 240
   tcctgctcat ggccttcccg gtcacctcca tcttccacac cttttcgcgc tcctactccg 300
   agctcaagga gcagcagcag cgcgcggcca gccccgagcc ggccctgcag gaggacagca 360
   cgcactcggc cacagccacc gaggacagct cgcagggccc cgacagcgcg ggcctggccg 420
   acgactccgc ggatgcgctg tgggtgcggg cagggcgctg acgcctgcgc cgcccac 477
<210> 90
   <211> 88
   <212> DNA
   <213> Homo sapiens
<400> 90
   gattgacagt ttctccttcc ccagactggc caatcacagg caggaagatg aaggttctgt 60
   gggctgcgtt gctggtcaca ttcctggc 88
<210> 91
   <211> 30
   <212> DNA
   <213> Artificial Sequence
<220>
   <223> Description of Artificial Sequence: Synthetic primer
<400> 91
   acgttggatg ttgacagttt ctccttcccc 30
<210> 92
   <211> 30
   <212> DNA
   <213> Artificial Sequence
<220>
   <223> Description of Artificial Sequence: Synthetic primer
<400> 92
   acgttggatg gaatgtgacc agcaacgcag 30
<210> 93
   <211> 18
   <212> DNA
   <213> Artificial Sequence
<220>
   <223> Description of Artificial Sequence: Synthetic primer
<400> 93
   gcaggaagat gaaggtty 18
<210> 94
   <211> 88
   <212> DNA
   <213> Artificial Sequence
<220>
   <223> Description of Artificial Sequence: Synthetic oligonucleotide
<400> 94
   gattgacagt ttctccttcc ccagactggc caatcacagg caggaagatg aaggttttgt 60
   gggctgcgtt gctggtcaca ttcctggc 88
<210> 95
   <211> 90
   <212> DNA
   <213> Homo sapiens
<400> 95
   gagttttgga tagtaaaata agtttcgaac tctggcacct ttcaattttg tcgcactctc 60
   cttgtttttg acaatgcaat catatgcttc 90
<210> 96
   <211> 31
   <212> DNA
   <213> Artificial Sequence
<220>
   <223> Description of Artificial Sequence: Synthetic primer
<400> 96
   acgtggatag taaaataagt ttcgaactct g 31
<210> 97
   <211> 27
   <212> DNA
   <213> Artificial Sequence
<220>
   <223> Description of Artificial Sequence: Synthetic primer
<400> 97
   gaagcatatg attgcattgt caaaaac 27
<210> 98
   <211> 20
   <212> DNA
   <213> Artificial Sequence
<220>
   <223> Description of Artificial Sequence: Synthetic primer
<400> 98
   atttcaattt tgtcgcacty 20
<210> 99
   <211> 90
   <212> DNA
   <213> Artificial Sequence
<220>
   <223> Description of Artificial Sequence: Synthetic oligonucleotide
<400> 99
   gagttttgga tagtaaaata agtttcgaac tctggcacct ttcaattttg tcgcactttc 60
   cttgtttttg acaatgcaat catatgcttc 90
<210> 100
   <211> 97
   <212> DNA
   <213> Homo sapiens
<400> 100
   gaactcctct ttgtctctgc gtgcccggcg cgcccccctc ccggtgggtg ataaacccac 60
   tctggcgccg gccatgcgct gggtgattaa tttgcga 97
<210> 101
   <211> 29
   <212> DNA
   <213> Artificial Sequence
<220>
   <223> Description of Artificial Sequence: Synthetic primer
<400> 101
   acgttggatg tctttgtctc tgcgtgccc 29
<210> 102
   <211> 30
   <212> DNA
   <213> Artificial Sequence
<220>
   <223> Description of Artificial Sequence: Synthetic primer
<400> 102
   acgttggatg ttaatcaccc agcgcatggc 30
<210> 103
   <211> 22
   <212> DNA
   <213> Artificial Sequence
<220>
   <223> Description of Artificial Sequence: Synthetic primer
<400> 103
   cccctcccgg tgggtgataa ay 22
<210> 104
   <211> 97
   <212> DNA
   <213> Artificial Sequence
<220>
   <223> Description of Artificial Sequence: Synthetic oligonucleotide
<400> 104
   gaactcctct ttgtctctgc gtgcccggcg cgcccccctc ccggtgggtg ataaatccac 60
   tctggcgccg gccatgcgct gggtgattaa tttgcga 97
<210> 105
   <211> 86
   <212> DNA
   <213> Homo sapiens
<400> 105
   ccattggccg tccgccgtgg cagtgcgggc gggagcgcag ggagagaacc acagctggaa 60
   tccgattccc accccaaaac ccagga 86
<210> 106
   <211> 29
   <212> DNA
   <213> Artificial Sequence
<220>
   <223> Description of Artificial Sequence: Synthetic primer
<400> 106
   acgttggatg ccattggccg tccgccgtg 29
<210> 107
   <211> 31
   <212> DNA
   <213> Artificial Sequence
<220>
   <223> Description of Artificial Sequence: Synthetic primer
<400> 107
   acgttggatg tcctgggttt tggggtggga a 31
<210> 108
   <211> 19
   <212> DNA
   <213> Artificial Sequence
<220>
   <223> Description of Artificial Sequence: Synthetic primer
<400> 108
   ttccagctgt ggttctctc 19
<210> 109
   <211> 86
   <212> DNA
   <213> Artificial Sequence
<220>
   <223> Description of Artificial Sequence: Synthetic oligonucleotide
<400> 109
   ccattggccg tccgccgtgg cagtgcgggc gggagcgcag agagagaacc acagctggaa 60
   tccgattccc accccaaaac ccagga 86
<210> 110
   <211> 31
   <212> DNA
   <213> Artificial Sequence
<220>
   <223> Description of Artificial Sequence: Synthetic primer
<400> 110
   acgttggatg acatggtcgg ccccacggaa t 31
<210> 111
   <211> 30
   <212> DNA
   <213> Artificial Sequence
<220>
   <223> Description of Artificial Sequence: Synthetic primer
<400> 111
   acgttggatg acccattggc cgtccgccgt 30
<210> 112
   <211> 31
   <212> DNA
   <213> Artificial Sequence
<220>
   <223> Description of Artificial Sequence: Synthetic primer
<400> 112
   acgttggatg gaactcctct ttgtctctgc g 31
<210> 113
   <211> 31
   <212> DNA
   <213> Artificial Sequence
<220>
   <223> Description of Artificial Sequence: Synthetic primer
<400> 113
   acgttggatg cgcagcaacg ggaccgctac a 31
<210> 114
   <211> 29
   <212> DNA
   <213> Artificial Sequence
<220>
   <223> Description of Artificial Sequence: Synthetic primer
<400> 114
   acgttgcgta gcaacctgtt acatattaa 29
<210> 115
   <211> 31
   <212> DNA
   <213> Artificial Sequence
<220>
   <223> Description of Artificial Sequence: Synthetic primer
<400> 115
   acgttggatg catagaggcc catgatggtg g 31
<210> 116
   <211> 33
   <212> DNA
   <213> Artificial Sequence
<220>
   <223> Description of Artificial Sequence: Synthetic primer
<400> 116
   acgttggatg gtgtggtcag ctcttccctt cat 33
<210> 117
   <211> 32
   <212> DNA
   <213> Artificial Sequence
<220>
   <223> Description of Artificial Sequence: Synthetic primer
<400> 117
   acgttggatg ctccttccta gtgtgagaac cg 32
<210> 118
   <211> 31
   <212> DNA
   <213> Artificial Sequence
<220>
   <223> Description of Artificial Sequence: Synthetic primer
<400> 118
   acgttggatg ttttggggtg ggaatcggat t 31
<210> 119
   <211> 29
   <212> DNA
   <213> Artificial Sequence
<220>
   <223> Description of Artificial Sequence: Synthetic primer
<400> 119
   acgttggatg tggcatggcc ggcgccaga 29
<210> 120
   <211> 32
   <212> DNA
   <213> Artificial Sequence
<220>
   <223> Description of Artificial Sequence: Synthetic primer
<400> 120
   acgttggcat ctaggtaggt ctttgtagcc aa 32
<210> 121
   <211> 32
   <212> DNA
   <213> Artificial Sequence
<220>
   <223> Description of Artificial Sequence: Synthetic primer
<400> 121
   acgttggatc tgagcaaagg caatcaacac cc 32
<210> 122
   <211> 32
   <212> DNA
   <213> Artificial Sequence
<220>
   <223> Description of Artificial Sequence: Synthetic primer
<400> 122
   acgttggatg accttctgcc cctctactcc aa 32
<210> 123
   <211> 33
   <212> DNA
   <213> Artificial Sequence
<220>
   <223> Description of Artificial Sequence: Synthetic primer
<400> 123
   acgttggccc acatgtaatg tgttgaaaaa gca 33
<210> 124
   <211> 18
   <212> DNA
   <213> Artificial Sequence
<220>
   <223> Description of Artificial Sequence: Synthetic primer
<400> 124
   caggttccgg ggcttggg 18
<210> 125
   <211> 19
   <212> DNA
   <213> Artificial Sequence
<220>
   <223> Description of Artificial Sequence: Synthetic primer
<400> 125
   cgcagggaga gaaccacag 19
<210> 126
   <211> 21
   <212> DNA
   <213> Artificial Sequence
<220>
   <223> Description of Artificial Sequence: Synthetic primer
<400> 126
   cccctcccgg tgggtgataa a 21
<210> 127
   <211> 21
   <212> DNA
   <213> Artificial Sequence
<220>
   <223> Description of Artificial Sequence: Synthetic primer
<400> 127
   aaagctgtag gacaatcggg t 21
<210> 128
   <211> 22
   <212> DNA
   <213> Artificial Sequence
<220>
   <223> Description of Artificial Sequence: Synthetic primer
<400> 128
   catttttcta catcctttgt tt 22
<210> 129
   <211> 23
   <212> DNA
   <213> Artificial Sequence
<220>
   <223> Description of Artificial Sequence: Synthetic primer
<400> 129
   agaagatcac caggcagaag agg 23
<210> 130
   <211> 25
   <212> DNA
   <213> Artificial Sequence
<220>
   <223> Description of Artificial Sequence: Synthetic primer
<400> 130
   acttggagaa caaaggacac cgtta 25
<210> 131
   <211> 81
   <212> DNA
   <213> Artificial Sequence
<220>
   <223> Description of Artificial Sequence: Synthetic oligonucleotide
<400> 131
   ggtcggcccc acggaatccc ggctctgtgt gcgcccaggt tccggggctt gggtgttgcc 60
   ggttctcaca ctaggaagga g 81
<210> 132
   <211> 79
   <212> DNA
   <213> Artificial Sequence
<220>
   <223> Description of Artificial Sequence: Synthetic oligonucleotide
<400> 132
   ccattggccg tccgccgtgg cagtgcgggc gggagcgcag agagagaacc acagctggaa 60
   tccgattccc accccaaaa 79
<210> 133
   <211> 77
   <212> DNA
   <213> Artificial Sequence
<220>
   <223> Description of Artificial Sequence: Synthetic oligonucleotide
<400> 133
   gaactcctct ttgtctctgc gtgcccggcg cgcccccctc ccggtgggtg ataaatccac 60
   tctggcgccg gccatgc 77
<210> 134
   <211> 81
   <212> DNA
   <213> Artificial Sequence
<220>
   <223> Description of Artificial Sequence: Synthetic oligonucleotide
<400> 134
   gcagcaacgg gaccgctaca gccactggac aaagccgtag gacaatcggg taacattggc 60
   tacaaagacc tacctagatg c 81
<210> 135
   <211> 95
   <212> DNA
   <213> Artificial Sequence
<220>
   <223> Description of Artificial Sequence: Synthetic oligonucleotide
<400> 135
   gcgtagcaac ctgttacata ttaaagtttt attatactac atttttctac atcctttgtt 60
   tcagagtgtt gattgccttt gctcagtatc ttcag 95
<210> 136
   <211> 86
   <212> DNA
   <213> Artificial Sequence
<220>
   <223> Description of Artificial Sequence: Synthetic oligonucleotide
<400> 136
   ccttctgccc ctctactcca agcgctacac cctcttctgc ctggtgatct ttgccggcgt 60
   cctggccacc atcatgggcc tctatg 86
<210> 137
   <211> 83
   <212> DNA
   <213> Artificial Sequence
<220>
   <223> Description of Artificial Sequence: Synthetic oligonucleotide
<400> 137
   gtgtggtcag ctcttccctt catcacatac ttggagaaca aaggacaccg ttatccatgc 60
   tttttcaaca cattacatgt ggg 83
<210> 138
   <211> 30
   <212> DNA
   <213> Artificial Sequence
<220>
   <223> Description of Artificial Sequence: Synthetic primer
<400> 138
   acgttggatg ttctgcccct ctactccaag 30
<210> 139
   <211> 30
   <212> DNA
   <213> Artificial Sequence
<220>
   <223> Description of Artificial Sequence: Synthetic primer
<400> 139
   acgttggatg tcagctcttc ccttcatcac 30
<210> 140
   <211> 30
   <212> DNA
   <213> Artificial Sequence
<220>
   <223> Description of Artificial Sequence: Synthetic primer
<400> 140
   acgttggatg ttgacagttt ctccttcccc 30
<210> 141
   <211> 28
   <212> DNA
   <213> Artificial Sequence
<220>
   <223> Description of Artificial Sequence: Synthetic primer
<400> 141
   acgttggatg cggtcggccc cacggaat 28
<210> 142
   <211> 31
   <212> DNA
   <213> Artificial Sequence
<220>
   <223> Description of Artificial Sequence: Synthetic primer
<400> 142
   acgtggatag taaaataagt ttcgaactct g 31
<210> 143
   <211> 30
   <212> DNA
   <213> Artificial Sequence
<220>
   <223> Description of Artificial Sequence: Synthetic primer
<400> 143
   acgttggatg cacagctcac cgcagcaacg 30
<210> 144
   <211> 29
   <212> DNA
   <213> Artificial Sequence
<220>
   <223> Description of Artificial Sequence: Synthetic primer
<400> 144
   acgttggatg tctttgtctc tgcgtgccc 29
<210> 145
   <211> 29
   <212> DNA
   <213> Artificial Sequence
<220>
   <223> Description of Artificial Sequence: Synthetic primer
<400> 145
   acgttggatg gactgagccc cagaactcg 29
<210> 146
   <211> 29
   <212> DNA
   <213> Artificial Sequence
<220>
   <223> Description of Artificial Sequence: Synthetic primer
<400> 146
   acgttggatg aagccaagtt tccctccgc 29
<210> 147
   <211> 30
   <212> DNA
   <213> Artificial Sequence
<220>
   <223> Description of Artificial Sequence: Synthetic primer
<400> 147
   acgttagcgt agcaacctgt tacatattaa 30
<210> 148
   <211> 30
   <212> DNA
   <213> Artificial Sequence
<220>
   <223> Description of Artificial Sequence: Synthetic primer
<400> 148
   acgttggatg catagaggcc catgatggtg 30
<210> 149
   <211> 31
   <212> DNA
   <213> Artificial Sequence
<220>
   <223> Description of Artificial Sequence: Synthetic primer
<400> 149
   acgttggatg cctacctccc acatgtaatg t 31
<210> 150
   <211> 30
   <212> DNA
   <213> Artificial Sequence
<220>
   <223> Description of Artificial Sequence: Synthetic primer
<400> 150
   acgttggatg gaatgtgacc agcaacgcag 30
<210> 151
   <211> 32
   <212> DNA
   <213> Artificial Sequence
<220>
   <223> Description of Artificial Sequence: Synthetic primer
<400> 151
   acgttggatg ctccttccta gtgtgagaac cg 32
<210> 152
   <211> 27
   <212> DNA
   <213> Artificial Sequence
<220>
   <223> Description of Artificial Sequence: Synthetic primer
<400> 152
   gaagcatatg attgcattgt caaaaac 27
<210> 153
   <211> 32
   <212> DNA
   <213> Artificial Sequence
<220>
   <223> Description of Artificial Sequence: Synthetic primer
<400> 153
   acgttggatg ctaggtaggt ctttgtagcc aa 32
<210> 154
   <211> 30
   <212> DNA
   <213> Artificial Sequence
<220>
   <223> Description of Artificial Sequence: Synthetic primer
<400> 154
   acgttggatg ttaatcaccc agcgcatggc 30
<210> 155
   <211> 30
   <212> DNA
   <213> Artificial Sequence
<220>
   <223> Description of Artificial Sequence: Synthetic primer
<400> 155
   acgttggatg gtgggtttgt gctttccacg 30
<210> 156
   <211> 30
   <212> DNA
   <213> Artificial Sequence
<220>
   <223> Description of Artificial Sequence: Synthetic primer
<400> 156
   acgttggatg cttttgcttt cccagccagg 30
<210> 157
   <211> 30
   <212> DNA
   <213> Artificial Sequence
<220>
   <223> Description of Artificial Sequence: Synthetic primer
<400> 157
   acgttggatg ctgagcaaag gcaatcaaca 30
<210> 158
   <211> 17
   <212> DNA
   <213> Artificial Sequence
<220>
   <223> Description of Artificial Sequence: Synthetic primer
<400> 158
   ttctgcctgg tgatctt 17
<210> 159
   <211> 17
   <212> DNA
   <213> Artificial Sequence
<220>
   <223> Description of Artificial Sequence: Synthetic primer
<400> 159
   aacaaaggac accgtta 17
<210> 160
   <211> 17
   <212> DNA
   <213> Artificial Sequence
<220>
   <223> Description of Artificial Sequence: Synthetic primer
<400> 160
   gcaggaagat gaaggtt 17
<210> 161
   <211> 18
   <212> DNA
   <213> Artificial Sequence
<220>
   <223> Description of Artificial Sequence: Synthetic primer
<400> 161
   aaggttccgg ggcttggg 18
<210> 162
   <211> 19
   <212> DNA
   <213> Artificial Sequence
<220>
   <223> Description of Artificial Sequence: Synthetic primer
<400> 162
   atttcaattt tgtcgcact 19
<210> 163
   <211> 19
   <212> DNA
   <213> Artificial Sequence
<220>
   <223> Description of Artificial Sequence: Synthetic primer
<400> 163
   agctgtagga caatcgggt 19
<210> 164
   <211> 20
   <212> DNA
   <213> Artificial Sequence
<220>
   <223> Description of Artificial Sequence: Synthetic primer
<400> 164
   ccctcccggt gggtgataaa 20
<210> 165
   <211> 20
   <212> DNA
   <213> Artificial Sequence
<220>
   <223> Description of Artificial Sequence: Synthetic primer
<400> 165
   agggccgggg tctgcgcgtg 20
<210> 166
   <211> 21
   <212> DNA
   <213> Artificial Sequence
<220>
   <223> Description of Artificial Sequence: Synthetic primer
<400> 166
   gaggcactgc ccggacaaac c 21
<210> 167
   <211> 22
   <212> DNA
   <213> Artificial Sequence
<220>
   <223> Description of Artificial Sequence: Synthetic primer
<400> 167
   catttttcta catcctttgt tt 22
<210> 168
   <211> 86
   <212> DNA
   <213> Artificial Sequence
<220>
   <223> Description of Artificial Sequence: Synthetic oligonucleotide
<400> 168
   ccttctgccc ctctactcca agcgctacac cctcttctgc ctggtgatct ttgccggcgt 60
   cctggccacc atcatgggcc tctatg 86
<210> 169
   <211> 90
   <212> DNA
   <213> Artificial Sequence
<220>
   <223> Description of Artificial Sequence: Synthetic oligonucleotide
<400> 169
   gtgtggtcag ctcttccctt catcacatac ttggagaaca aaggacaccg ttatccatgc 60
   tttttcaaca cattacatgt gggaggtagg 90
<210> 170
   <211> 88
   <212> DNA
   <213> Artificial Sequence
<220>
   <223> Description of Artificial Sequence: Synthetic oligonucleotide
<400> 170
   gattgacagt ttctccttcc ccagactggc caatcacagg caggaagatg aaggttttgt 60
   gggctgcgtt gctggtcaca ttcctggc 88
<210> 171
   <211> 98
   <212> DNA
   <213> Artificial Sequence
<220>
   <223> Description of Artificial Sequence: Synthetic oligonucleotide
<400> 171
   aaaaccagag attcgcggtc ggccccacgg aatcccggct ctgtgtgcgc ccaggttccg 60
   gggcttgggt gttgccggtt ctcacactag gaaggagc 98
<210> 172
   <211> 90
   <212> DNA
   <213> Artificial Sequence
<220>
   <223> Description of Artificial Sequence: Synthetic oligonucleotide
<400> 172
   gagttttgga tagtaaaata agtttcgaac tctggcacct ttcaattttg tcgcactttc 60
   cttgtttttg acaatgcaat catatgcttc 90
<210> 173
   <211> 95
   <212> DNA
   <213> Artificial Sequence
<220>
   <223> Description of Artificial Sequence: Synthetic oligonucleotide
<400> 173
   gcagccagct caccgcagca acgggaccgc tacagccact ggacaaagct gtaggacaat 60
   cgggtgacat tggctacaaa gacctaccta gatgc 95
<210> 174
   <211> 97
   <212> DNA
   <213> Artificial Sequence
<220>
   <223> Description of Artificial Sequence: Synthetic oligonucleotide
<400> 174
   gaactcctct ttgtctctgc gtgcccggcg cgcccccctc ccggtgggtg ataaatccac 60
   tctggcgccg gccatgcgct gggtgattaa tttgcga 97
<210> 175
   <211> 86
   <212> DNA
   <213> Artificial Sequence
<220>
   <223> Description of Artificial Sequence: Synthetic oligonucleotide
<400> 175
   gtgggtttgt gctttccacg cgtgcacaca cacgcgcaga ccccggccct tgccccgcct 60
   acctccccga gttctggggc tcagtc 86
<210> 176
   <211> 89
   <212> DNA
   <213> Artificial Sequence
<220>
   <223> Description of Artificial Sequence: Synthetic oligonucleotide
<400> 176
   gcgccagctt ttgctttccc agccagggcg cggtgaggtt tgtccgggca gtgcctcgag 60
   caactgggaa ggccaaggcg gagggaaac 89
<210> 177
   <211> 96
   <212> DNA
   <213> Artificial Sequence
<220>
   <223> Description of Artificial Sequence: Synthetic oligonucleotide
<400> 177
   gcgtagcaac ctgttacata ttaaagtttt attatactac atttttctac atcctttgtt 60
   ttagggtgtt gattgccttt gctcagtatc ttcagc 96
<210> 178
   <211> 10
   <212> DNA
   <213> Artificial Sequence
<220>
   <223> Description of Artificial Sequence: Synthetic oligonucleotide
<400> 178
   cgcaaccact 10
<210> 179
   <211> 10
   <212> DNA
   <213> Artificial Sequence
<220>
   <223> Description of Artificial Sequence: Synthetic oligonucleotide
<400> 179
   cgcgaccact 10

## Description

### FIELD

The present invention is defined by the claims. Provided herein are biomarkers. The biomarkers provided may be useful for noninvasive detection of fetal genetic traits. Certain fetal genetic traits include but are not limited to presence or absence of fetal nucleic acid.

### BACKGROUND

Non-invasive prenatal testing is becoming a field of rapidly growing interest. Early detection of pregnancy-related conditions, including complications during pregnancy and genetic defects of the fetus is of crucial importance, as it allows early medical intervention necessary for the safety of both the mother and the fetus. Prenatal diagnosis has been conducted using cells isolated from the fetus through procedures such as chorionic villus sampling (CVS) or amniocentesis. However, these conventional methods are invasive and present an appreciable risk to both the mother and the fetus. The National Health Service currently cites a miscarriage rate of between 1 and 2 per cent following the invasive amniocentesis and chorionic villus sampling (CVS) tests.

An alternative to these invasive approaches has been developed for prenatal screening, e.g., to detecting fetal abnormalities, following the discovery that circulating cell-free fetal nucleic acid can be detected in maternal plasma and serum (Lo et al., Lancet 350:485-487, 1997; and U.S. Patent 6,258,540). Circulating cell free fetal nucleic acid (cffNA) has several advantages making it more applicable for non-invasive prenatal testing. For example, cell free nucleic acid is present at higher levels than fetal cells and at concentrations sufficient for genetic analysis. Also, cffNA is cleared from the maternal bloodstream within hours after delivery, preventing contamination from previous pregnancies.

Examples of prenatal tests performed by detecting fetal DNA in maternal plasma or serum include fetal rhesus D (RhD) genotyping (Lo et al., N. Engl. J. Med. 339:1734-1738, 1998), fetal sex determination (Costa et al., N. Engl. J. Med. 346:1502, 2002), and diagnosis of several fetal disorders (Amicucci et al., Clin. Chem. 46:301-302, 2000; Saito et al., Lancet 356:1170, 2000; and Chiu et al., Lancet 360:998-1000, 2002). In addition, quantitative abnormalities of fetal DNA in maternal plasma/serum have been reported in preeclampsia (Lo et al., Clin. Chem. 45:184-188, 1999 and Zhong et al., Am. J. Obstet. Gynecol. 184:414-419, 2001), fetal trisomy 21 (Lo et al., Clin. Chem. 45:1747-1751, 1999 and Zhong et al., Prenat. Diagn. 20:795-798, 2000) and hyperemesis gravidarum (Sekizawa et al., Clin. Chem. 47:2164-2165, 2001).

WO/2007132166A2 describes that in a pregnant woman certain genes (such as *RASSF1A, APC, CASP8, RARB, SCGB3A1, DAB21P, PTPN6, THY1, TMEFF2,* and *PYCARD*) originated from a fetus are highly methylated, whereas the same genes of maternal origin are unmethylated. These genes can be measured directly for diagnosis of certain pregnancy-related conditions. Old (2007), Reproductive Biomedicine online, 15(2):227-235 describes three candidate epigenetic chromosome 21-specific biomarkers for the non-invasive prenatal diagnosis of trisomy 21. WO/2004078999A1 provides methods of quantifying fetal specific nucleic acids in, for example, a maternal plasma or serum sample using subtractive hybridization based procedures.

### SUMMARY

The technology described herein provides *inter alia* human epigenetic biomarkers that are useful for the noninvasive detection of fetal genetic traits, including, but not limited to, the presence or absence of fetal nucleic acid, the absolute or relative amount of fetal nucleic acid, fetal sex, and fetal chromosomal abnormalities such as aneuploidy. The human epigenetic biomarkers of the technology represent genomic DNA that display differential CpG methylation patterns between the fetus and mother. The compositions and processes of the technology allow for the detection and quantification of fetal nucleic acid in a maternal sample based on the methylation status of the nucleic acid in said sample. More specifically, the amount of fetal nucleic acid from a maternal sample can be determined relative to the total amount of nucleic acid present, thereby providing the percentage of fetal nucleic acid in the sample. Further, the amount of fetal nucleic acid can be determined in a sequence-specific (or locus-specific) manner and with sufficient sensitivity to allow for accurate chromosomal dosage analysis (for example, to detect the presence or absence of a fetal aneuploidy).

The present invention is defined by the claims. In more detail, the invention pertains to a method for determining the amount of fetal nucleic acid in a sample comprising: a) contacting nucleic acid present in a sample from a pregnant female, which sample comprises differentially methylated fetal nucleic acid and maternal nucleic acid, with an a methyl-CpG binding protein (MBD) or fragment thereof being capable of specifically binding methylated nucleotides; thereby producing bound nucleic acid; b) isolating the bound nucleic acid of (a) by contact with solutions of increasing salt concentration, thereby enriching the fetal nucleic acid; and c) determining the amount of fetal nucleic acid by analyzing the amount of fetal nucleic acid of (b) at a plurality of loci selected from loci of SEQ ID NOs:1-59.

Also described herein is a method for enriching fetal nucleic acids from a maternal biological sample, based on differential methylation between fetal and maternal nucleic acid comprising the steps of: (a) binding a target nucleic acid, from a sample, and a control nucleic acid, from the sample, to a methylation-specific binding protein; and (b) eluting the bound nucleic acid based on methylation status, wherein differentially methylated nucleic acids elute at least partly into separate fractions. The nucleic acid sequence may include one or more of the polynucleotide sequences of SEQ ID NOs: 1-89. SEQ ID NOs: 1-89 are provided in Table 4. The technology includes the sequences of SEQ ID NOs: 1-89, and variations thereto. A control nucleic acid may not be included in step (a).

Also described herein is a method for enriching fetal nucleic acid from a maternal sample, which comprises the following steps: (a) obtaining a biological sample from a woman; (b) separating fetal and maternal nucleic acid based on the methylation status of a CpG-containing genomic sequence in the sample, wherein the genomic sequence from the fetus and the genomic sequence from the woman are differentially methylated, thereby distinguishing the genomic sequence from the woman and the genomic sequence from the fetus in the sample. The genomic sequence may be at least 15 nucleotides in length, comprising at least one cytosine, further wherein the region has (1) a genomic locus selected from Table 1; and (2) a DNA sequence of no more than 10 kb upstream and/or downstream from the locus. Obtaining a biological sample from a woman may not limit the scope of the technology. This obtaining can refer to actually collecting a sample from a woman (e.g., a blood draw) or to receiving a sample from elsewhere (e.g., from a clinic or hospital) and performing steps of a method.

Also described herein is a method for enriching fetal nucleic acid from a maternal sample, which comprises the following steps: (a) obtaining a biological sample from the woman; (b) digesting or removing maternal nucleic acid based on the methylation status of a CpG-containing genomic sequence in the sample, wherein the genomic sequence from the fetus and the genomic sequence from the woman are differentially methylated, thereby enriching for the genomic sequence from the fetus in the sample. Maternal nucleic acid may be digested using one or more methylation sensitive restriction enzymes that selectively digest or cleave maternal nucleic acid based on its methylation status. The genomic sequence may be at least 15 nucleotides in length, comprising at least one cytosine, further wherein the region consists of (1) a genomic locus selected from Table 1; and (2) a DNA sequence of no more than 10 kb upstream and/or downstream from the locus.

Also described herein is a method for preparing nucleic acid having a nucleotide sequence of a fetal nucleic acid, which comprises the following steps: (a) providing a sample from a pregnant female; (b) separating fetal nucleic acid from maternal nucleic acid from the sample of the pregnant female according to a different methylation state between the fetal nucleic acid and the maternal nucleic acid counterpart, wherein the nucleotide sequence of the fetal nucleic acid comprises one or more CpG sites from one or more of the polynucleotide sequences of SEQ ID NOs: 1-89 within a polynucleotide sequence from a gene or locus that contains one of the polynucleotide sequences of SEQ ID NOs: 1-89; and (c) preparing nucleic acid comprising a nucleotide sequence of the fetal nucleic acid by an amplification process in which fetal nucleic acid separated in part (b) is utilized as a template. Furthermore described herein is a method for preparing nucleic acid having a nucleotide sequence of a fetal nucleic acid, which comprises the following steps: (a) providing a sample from a pregnant female; (b) digesting or removing maternal nucleic acid from the sample of the pregnant female according to a different methylation state between the fetal nucleic acid and the maternal nucleic acid counterpart, wherein the nucleotide sequence of the fetal nucleic acid comprises one or more CpG sites from one or more of the polynucleotide sequences of SEQ ID NOs: 1-89 within a polynucleotide sequence from a gene that contains one of the polynucleotide sequences of SEQ ID NOs: 1-89; and (c) preparing nucleic acid comprising a nucleotide sequence of the fetal nucleic acid. The preparing process of step (c) may be a hybridization process, a capture process, or an amplification process in which fetal nucleic acid separated in part (b) is utilized as a template. Also, in case the maternal nucleic acid is digested, the maternal nucleic acid may be digested using one or more methylation sensitive restriction enzymes that selectively digest or cleave maternal nucleic acid based on its methylation status. The polynucleotide sequences of SEQ ID NOs: 1-89 may be within a polynucleotide sequence from a CpG island that contains one of the polynucleotide sequences of SEQ ID NOs: 1-89. The polynucleotide sequences of SEQ ID NOs: 1-89 are further characterized in Tables 1-3 herein, including the identification of CpG islands that overlap with the polynucleotide sequences provided in SEQ ID NOs: 1-89. The nucleic acid prepared by part (c) may be in solution. The method may further comprise quantifying the fetal nucleic acid from the amplification process of step (c).

Also described herein is a method for enriching fetal nucleic acid from a sample from a pregnant female with respect to maternal nucleic acid, which comprises the following steps: (a) providing a sample from a pregnant female; and (b) separating or capturing fetal nucleic acid from maternal nucleic acid from the sample of the pregnant female according to a different methylation state between the fetal nucleic acid and the maternal nucleic acid, wherein the nucleotide sequence of the fetal nucleic acid comprises one or more CpG sites from one or more of the polynucleotide sequences of SEQ ID NOs: 1-89 within a polynucleotide sequence from a gene that contains one of the polynucleotide sequences of SEQ ID NOs: 1-89The polynucleotide sequences of SEQ ID NOs: 1-89 may be within a polynucleotide sequence from a CpG island that contains one of the polynucleotide sequences of SEQ ID NOs: 1-89. The polynucleotide sequences of SEQ ID NOs: 1-89 are characterized in Table 1 herein. The nucleic acid separated by part (b) may be in solution. The method may further comprise amplifying and/or quantifying the fetal nucleic acid from the separation process of step (b).

Also described herein is a composition comprising an isolated nucleic acid from a fetus of a pregnant female, wherein the nucleotide sequence of the nucleic acid comprises one or more of the polynucleotide sequences of SEQ ID NOs: 1-89. The nucleotide sequence may consist essentially of a nucleotide sequence of a gene, or portion thereof. The nucleotide sequence may also consist essentially of a nucleotide sequence of a CpG island, or portion thereof. The polynucleotide sequences of SEQ ID NOs: 1-89 are further characterized in Table 1. The nucleic acid may bein solution. The nucleic acid from the fetus may be enriched relative to maternal nucleic acid. The composition may further comprise an agent that binds to methylated nucleotides. For example, the agent may be a methyl-CpG binding protein (MBD) or fragment thereof.

Also described herein is a composition comprising an isolated nucleic acid from a fetus of a pregnant female, wherein the nucleotide sequence of the nucleic acid comprises one or more CpG sites from one or more of the polynucleotide sequences of SEQ ID NOs: 1-89 within a polynucleotide sequence from a gene, or portion thereof, that contains one of the polynucleotide sequences of SEQ ID NOs: 1-89. The nucleotide sequence of the nucleic acid may comprise one or more CpG sites from one or more of the polynucleotide sequences of SEQ ID NOs: 1-89 within a polynucleotide sequence from a CpG island, or portion thereof, that contains one of the polynucleotide sequences of SEQ ID NOs: 1-89. The polynucleotide sequences of SEQ ID NOs: 1-89 are further characterized in Table 1. The nucleic acid may be in solution. The nucleic acid from the fetus may be enriched relative to maternal nucleic acid. Hyper- and hypomethylated nucleic acid sequences of the technology are identified in Table 1. The composition may further comprise an agent that binds to methylated nucleotides. For example, the agent may be a methyl-CpG binding protein (MBD) or fragment thereof.

A nucleotide sequence of the technology may include three or more of the CpG sites. The nucleotide sequence may include five or more of the CpG sites. The nucleotide sequence may be from a gene region that comprises a PRC2 domain (see Table 3). The nucleotide sequence may be from a gene region involved with development. For example, SOX14 - which is an epigenetic marker of the present technology (See Table 1) - is a member of the SOX (SRY-related HMG-box) family of transcription factors involved in the regulation of embryonic development and in the determination of cell fate.

The genomic sequence from the woman may be methylated and the genomic sequence from the fetus may be unmethylatedThe genomic sequence from the woman may be unmethylated and the genomic sequence from the fetus may be methylated. The genomic sequence from the fetus may be hypermethylated relative to the genomic sequence from the mother. Fetal genomic sequences found to be hypermethylated relative to maternal genomic sequence are provided in SEQ ID NOs: 1-59. Alternatively, the genomic sequence from the fetus may be hypomethylated relative to the genomic sequence from the mother. Fetal genomic sequences found to be hypomethylated relative to maternal genomic sequence are provided in SEQ ID NOs: 60-85. Methylation sensitive restriction enzymes of the technology may be sensitive to hypo- or hypermethylated nucleic acid.

The fetal nucleic acid may be extracellular nucleic acid. Generally the extracellular fetal nucleic acid may be about 500, 400, 300, 250, 200 or 150 (or any number there between) nucleotide bases or less. The digested maternal nucleic acid may be less than about 90, 100, 110, 120, 130, 140 or 150 base pairs. The fetal nucleic acid may be selectively amplified, captured or separated from or relative to the digested maternal nucleic acid based on size. For example, PCR primers may be designed to amplify nucleic acid greater than about 75, 80, 85, 90, 95, 100, 105, 110, 115 or 120 (or any number there between) base pairs thereby amplifying fetal nucleic acid and not digested maternal nucleic acid. The nucleic acid may be subjected to fragmentation prior to certain methods of the technology. Examples of methods of fragmenting nucleic acid, include but are not limited to sonication and restriction enzyme digestion. The fetal nucleic acid may be derived from the placenta. The fetal nucleic acid may be apoptotic.

The present technology may provide a method in which the sample is a member selected from the following: maternal whole blood, maternal plasma or serum, amniotic fluid, a chorionic villus sample, fetal nucleated cells or fetal cellular remnants isolated from maternal blood, maternal urine, maternal saliva, washings of the female reproductive tract and a sample obtained by celocentesis or lung lavage. The biological sample may be maternal blood. The biological sample may be a chorionic villus sample. The maternal sample may be enriched for fetal nucleic acid prior to certain methods of the present technology. Examples of fetal enrichment methods are provided in PCT Publication Nos. WO/2007140,417A2, WO2009/032781A2 and US Publication No. 20050164241.

Nucleated and anucleated cell populations may be removed from the sample prior to practicing certain methods of the technology (e.g., substantially all nucleated and anucleated cell populations are removed). The sample may be collected, stored or transported in a manner known to the person of ordinary skill in the art to minimize degradation or the quality of fetal nucleic acid present in the sample.

The sample can be from any animal, including but not limited to human, non-human, mammal, reptile, cattle, cat, dog, goat, swine, pig, monkey, ape, gorilla, bull, cow, bear, horse, sheep, poultry, mouse, rat, fish, dolphin, whale, and shark, or any animal or organism that may have a detectable pregnancy-associated disorder or chromosomal abnormality.

The sample may be treated with a reagent that differentially modifies methylated and unmethylated DNA. For example, the reagent may comprise bisulfite; or the reagent may comprise one or more enzymes that preferentially cleave methylated DNA; or the reagent may comprise one or more enzymes that preferentially cleave unmethylated DNA. Examples of methylation sensitive restriction enzymes include, but are not limited to, Hhal and Hpall.

The fetal nucleic acid may be separated from the maternal nucleic acid by an agent that specifically binds to methylated nucleotides in the fetal nucleic acid. The fetal nucleic acid may be separated or removed from the maternal nucleic acid by an agent that specifically binds to methylated nucleotides in the maternal nucleic acid counterpart. The agent that binds to methylated nucleotides may be a methyl-CpG binding protein (MBD) or fragment thereof.

Also described herein is a method for determining the amount or copy number of fetal DNA in a maternal sample that comprises differentially methylated maternal and fetal DNA. The method is performed by a) distinguishing between the maternal and fetal DNA based on differential methylation status; and b) quantifying the fetal DNA of step a). The method may comprise a) digesting the maternal DNA in a maternal sample using one or more methylation sensitive restriction enzymes thereby enriching the fetal DNA; and b) determining the amount of fetal DNA from step a). The amount of fetal DNA can be used *inter alia* to confirm the presence or absence of fetal nucleic acid, determine fetal sex, diagnose fetal disease or be used in conjunction with other fetal diagnostic methods to improve sensitivity or specificity. The method for determining the amount of fetal DNA does not require the use of a polymorphic sequence. For example, an allelic ratio is not used to quantify the fetal DNA in step b). The method for determining the amount of fetal DNA may not require the treatment of DNA with bisulfite to convert cytosine residues to uracil. Bisulfite is known to degrade DNA, thereby, further reducing the already limited fetal nucleic acid present in maternal samples. Determining the amount of fetal DNA in step b) may be done by introducing one or more competitors at known concentrations. Alternatively, determining the amount of fetal DNA in step b) may be done by RT-PCR, primer extension; sequencing or counting. The amount of nucleic acid may be determined using BEAMing technology as described in US Patent Publication No. US20070065823. The restriction efficiency may be determined and the efficiency rate may be used to further determine the amount of fetal DNA. Exemplary differentially methylated nucleic acids are provided in SEQ ID NOs: 1-89.

Also described herein is a method for determining the concentration of fetal DNA in a maternal sample, wherein the maternal sample comprises differentially methylated maternal and fetal DNA, comprising a) determining the total amount of DNA present in the maternal sample; b) selectively digesting the maternal DNA in a maternal sample using one or more methylation sensitive restriction enzymes thereby enriching the fetal DNA; c) determining the amount of fetal. DNA from step b); and d) comparing the amount of fetal DNA from step c) to the total amount of DNA from step a), thereby determining the concentration of fetal DNA in the maternal sample. The concentration of fetal DNA can be used *inter alia* in conjunction with other fetal diagnostic methods to improve sensitivity or specificity. The method for determining the amount of fetal DNA may not require the use of a polymorphic sequence. For example, an allelic ratio is not used to quantify the fetal DNA in step b). The method for determining the amount of fetal DNA may not require the treatment of DNA with bisulfite to convert cytosine residues to uracil. Determining the amount of fetal DNA in step b) may be done by introducing one or more competitors at known concentrations. Alternatively, determining the amount of fetal DNA in step b) may be done by RT-PCR, sequencing or counting. The restriction efficiency may be determined and may be used to further determine the amount of total DNA and fetal DNA. Exemplary differentially methylated nucleic acids are provided in SEQ ID NOs: 1-89.

Also described herein is a method for determining the presence or absence of a fetal aneuploidy using fetal DNA from a maternal sample, wherein the maternal sample comprises differentially methylated maternal and fetal DNA, comprising a) selectively digesting the maternal DNA in a maternal sample using one or more methylation sensitive restriction enzymes thereby enriching the fetal DNA; b) determining the amount of fetal DNA from a target chromosome; c) determining the amount of fetal DNA from a reference chromosome; and d) comparing the amount of fetal DNA from step b) to step c), wherein a biologically or statistically significant difference between the amount of target and reference fetal DNA is indicative of the presence of a fetal aneuploidy. The method for determining the amount of fetal DNA may not require the use of a polymorphic sequence. For example, an allelic ratio is not used to quantify the fetal DNA in step b). The method for determining the amount of fetal DNA may not require the treatment of DNA with bisulfite to convert cytosine residues to uracil. Determining the amount of fetal DNA in steps b) and c) may be done by introducing one or more competitors at known concentrations. Determining the amount of fetal DNA in steps b) and c) may be done by RT-PCR, sequencing or counting. The amount of fetal DNA from a target chromosome determined in step b) may be compared to a standard control, for example, the amount of fetal DNA from a target chromosome from euploid pregnancies. The restriction efficiency may be determined and may be used to further determine the amount of fetal DNA from a target chromosome and from a reference chromosome. Exemplary differentially methylated nucleic acids are provided in SEQ ID NOs: 1-89.

Also described herein is a method for detecting the presence or absence of a chromosomal abnormality by analyzing the amount or copy number of target nucleic acid and control nucleic acid from a sample of differentially methylated nucleic acids comprising the steps of: (a) enriching a target nucleic acid, from a sample, and a control nucleic acid, from the sample, based on its methylation state; (b) performing a copy number analysis of the enriched target nucleic acid in at least one of the fractions; (c) performing a copy number analysis of the enriched control nucleic acid in at least one of the fractions; (d) comparing the copy number from step (b) with the copy number from step (c); and (e) determining if a chromosomal abnormality exists based on the comparison in step (d), wherein the target nucleic acid and control nucleic acid have the same or substantially the same methylation status. Also described herein is a method for detecting the presence or absence of a chromosomal abnormality by analyzing the amount or copy number of target nucleic acid and control nucleic acid from a sample of differentially methylated nucleic acids comprising the steps of: (a) binding a target nucleic acid, from a sample, and a control nucleic acid, from the sample, to a binding agent; (b) eluting the bound nucleic acid based on methylation status, wherein differentially methylated nucleic acids elute at least partly into separate fractions; (c) performing a copy number analysis of the eluted target nucleic acid in at least one of the fractions; (d) performing a copy number analysis of the eluted control nucleic acid in at least one of the fractions; (e) comparing the copy number from step (c) with the copy number from step (d); and (f) determining if a chromosomal abnormality exists based on the comparison in step (e), wherein the target nucleic acid and control nucleic acid have the same or substantially the same methylation status. Differentially methylated nucleic acids are provided in SEQ ID NOs: 1-89.

Also described herein is a method for detecting the presence or absence of a chromosomal abnormality by analyzing the allelic ratio of target nucleic acid and control nucleic acid from a sample of differentially methylated nucleic acids comprising the steps of: (a) binding a target nucleic acid, from a sample, and a control nucleic acid, from the sample, to a binding agent; (b) eluting the bound nucleic acid based on methylation status, wherein differentially methylated nucleic acids elute at least partly into separate fractions; (c) performing an allelic ratio analysis of the eluted target nucleic acid in at least one of the fractions; (d) performing an allelic ratio analysis of the eluted control nucleic acid in at least one of the fractions; (e) comparing the allelic ratio from step c with the allelic ratio from step d; and (f) determining if a chromosomal abnormality exists based on the comparison in step (e), wherein the target nucleic acid and control nucleic acid have the same or substantially the same methylation status. Differentially methylated nucleic acids are provided in SEQ ID NOs: 1-89, and SNPs within the differentially methylated nucleic acids are provided in Table 2. The methods may also be useful for detecting a pregnancy-associated disorder.

The amount of maternal nucleic acid may be determined using the methylation-based methods of the technology. For example, fetal nucleic acid can be separated (for example, digested using a methylation-sensitive enzyme) from the maternal nucleic acid in a sample, and the maternal nucleic acid can be quantified using the methods of the technology. Once the amount of maternal nucleic acid is determined, that amount can subtracted from the total amount of nucleic acid in a sample to determine the amount of fetal nucleic acid. The amount of fetal nucleic acid can be used to detect fetal traits, including fetal aneuploidy, as described herein.

The methods described herein may also be useful for detecting a pregnancy-associated disorder. The sample may comprise fetal nucleic acid, or fetal nucleic acid and maternal nucleic acid. In the case when the sample comprises fetal and maternal nucleic acid, the fetal nucleic acid and the maternal nucleic acid may have a different methylation status. Nucleic acid species with a different methylation status can be differentiated by any method known in the art. The fetal nucleic acid may be enriched by the selective digestion of maternal nucleic acid by a methylation sensitive restriction enzyme. The fetal nucleic acid may enriched by the selective digestion of maternal nucleic acid using two or more methylation sensitive restriction enzymes in the same assay. The target nucleic acid and control nucleic acid may be both from the fetus. The average size of the fetal nucleic acid may be about 100 bases to about 500 bases in lengthThe chromosomal abnormality may be an aneuploidy, such as trisomy 21. The target nucleic acid may be at least a portion of a chromosome which may be abnormal and the control nucleic acid may be at least a portion of a chromosome which is very rarely abnormal. For example, when the target nucleic acid is from chromosome 21, the control nucleic acid is from a chromosome other than chromosome 21 - preferably another autosome. The binding agent may be a methylation-specific binding protein such as MBD-Fc. Also, the enriched or eluted nucleic acid is amplified and/or quantified by any method known in the art. The fetal DNA may be quantified using a method that does not require the use of a polymorphic sequence. For example, an allelic ratio is not used to quantify the fetal DNA. The method for quantifying the amount of fetal DNA may not require the treatment of DNA with bisulfite to convert cytosine residues to uracil.

The methods described herein may include the additional step of determining the amount of one or more Y-chromosome-specific sequences in a sample. The amount of fetal nucleic acid in a sample as determined by using the methylation-based methods of the technology may be compared to the amount of Y-chromosome nucleic acid present.

Methods for differentiating nucleic acid based on methylation status include, but are not limited to, methylation sensitive capture, for example using, MBD2-Fc fragment; bisulfite conversion methods, for example, MSP (methylation-sensitive PCR), COBRA, methylation-sensitive single nucleotide primer extension (Ms-SNuPE) or Sequenom MassCLEAVE™ technology; and the use of methylation sensitive restriction enzymes. Except where explicitly stated, any method for differentiating nucleic acid based on methylation status can be used with the compositions and methods of the technology.

The methods of the technology described herein may further comprise an amplification step. The amplification step can be performed by PCR, such as methylation-specific PCR. The amplification reaction may be performed on single molecules, for example, by digital PCR, which is further described in US Patent Nos 6,143,496 and 6,440,706. The method may not require amplification. For example, the amount of enriched fetal DNA may be determined by counting the fetal DNA (or sequence tags attached thereto) with a flow cytometer or by sequencing means that do not require amplification. The amount of fetal DNA may be determined by an amplification reaction that generates amplicons larger than the digested maternal nucleic acid, thereby further enriching the fetal nucleic acid.

For methods that require sequence analysis, any one of the following sequencing technologies may be used: a primer extension method (e.g., iPLEX®; Sequenom, Inc.), direct DNA sequencing, restriction fragment length polymorphism (RFLP analysis), allele specific oligonucleotide (ASO) analysis, methylation-specific PCR (MSPCR), pyrosequencing analysis, acycloprime analysis, Reverse dot blot, GeneChip microarrays, Dynamic allele-specific hybridization (DASH), Peptide nucleic acid (PNA) and locked nucleic acids (LNA) probes, TaqMan, Molecular Beacons, Intercalating dye, FRET primers, fluorescence tagged dNTP/ddNTPs, AlphaScreen, SNPstream, genetic bit analysis (GBA), Multiplex minisequencing, SNaPshot, GOOD assay, Microarray miniseq, arrayed primer extension (APEX), Microarray primer extension, Tag arrays, Coded microspheres, Template-directed incorporation (TDI), fluorescence polarization, Colorimetric oligonucleotide ligation assay (OLA), Sequence-coded OLA, Microarray ligation, Ligase chain reaction, Padlock probes, Invader™ assay, hybridization using at least one probe, hybridization using at least one fluorescently labeled probe, electrophoresis, cloning and sequencing, for example as performed on the 454 platform (Roche) (Margulies, M. et al. 2005 Nature 437, 376-380), Illumina Genome Analyzer (or Solexa platform) or SOLiD System (Applied Biosystems) or the Helicos True Single Molecule DNA sequencing technology (Harris T D et al. 2008 Science, 320, 106-109), the single molecule, real-time (SMRT.TM.) technology of Pacific Biosciences, or nanopore-based sequencing (Soni GV and Meller A. 2007 Clin Chem 53: 1996-2001) and combinations thereof. Nanopore-based methods may include sequencing nucleic acid using a nanopore, or counting nucleic acid molecules using a nanopore, for example, based on size wherein sequence information is not determined.

The absolute copy number of one or more nucleic acids can be determined, for example, using mass spectrometry, a system that uses a competitive PCR approach for absolute copy number measurements. See for example, Ding C, Cantor CR (2003), A high-throughput gene expression analysis technique using competitive PCR and matrix-assisted laser desorption ionization time-of-flight MS. Proc Natl Acad Sci U S A 100:3059-3064, and US Patent Application No. 10/655762, which published as US Patent Publication No. 20040081993.

The amount of the genomic sequence may be compared with a standard control, wherein an increase or decrease from the standard control indicates the presence or progression of a pregnancy-associated disorder. For example, the amount of fetal nucleic acid may be compared to the total amount of DNA present in the sample. Or when detecting the presence or absence of fetal aneuploidy, the amount of fetal nucleic acid from target chromosome may be compared to the amount of fetal nucleic acid from a reference chromosome. Preferably the reference chromosome is another autosome that has a low rate of aneuploidy. The ratio of target fetal nucleic acid to reference fetal nucleic acid may be compared to the same ratio from a normal, euploid pregnancy. For example, a control ratio may be determined from a DNA sample obtained from a female carrying a healthy fetus who does not have a chromosomal abnormality. Preferably, one uses a panel of control samples. Where certain chromosome anomalies are known, one can also have standards that are indicative of a specific disease or condition. Thus, for example, to screen for three different chromosomal aneuploidies in a maternal plasma of a pregnant female, one preferably uses a panel of control DNAs that have been isolated from mothers who are known to carry a fetus with, for example, chromosome 13, 18, or 21 trisomy, and a mother who is pregnant with a fetus who does not have a chromosomal abnormality.

Also described herein is a method in which the alleles from the target nucleic acid and control nucleic acid are differentiated by sequence variation. The sequence variation may be a single nucleotide polymorphism (SNP) or an insertion/deletion polymorphism. The fetal nucleic acid may comprise at least one high frequency heterozygous polymorphism (e.g., about 2%, 3%, 4%, 5%, 6%, 7%, 8%, 9%, 10%, 11%, 12%, 13%, 14%, 15%, 16%, 17%, 18%, 19%, 20%, 25% or more frequency rate), which allows the determination of the allelic-ratio of the nucleic acid in order to assess the presence or absence of the chromosomal abnormality. A list of exemplary SNPs is provided in Table 2, however, this does not represent a complete list of polymorphic alleles that can be used as part of the technology. Any SNP meeting the following criteria may also be considered: (a) the SNP has a heterozygosity frequency greater than about 2% (preferably across a range of different populations), (b) the SNP is a heterozygous locus; and (c)(i) the SNP is within nucleic acid sequence described herein, or (c)(iii) the SNP is within about 5 to about 2000 base pairs of a SNP described herein (e.g., within about 5, 10, 15, 20, 25, 30, 40, 50, 60, 70, 80, 90, 100, 200, 300, 400, 500, 600, 700, 800, 900, 1000, 1250, 1500, 1750 or 2000 base pairs of a SNP described herein).

The sequence variation may be a short tandem repeat (STR) polymorphism. The sequence variation may fall in a restriction site, whereby one allele is susceptible to digestion by a restriction enzyme and the one or more other alleles are not. The sequence variation may be a methylation site.

Performing an allelic ratio analysis may comprise determining the ratio of alleles of the target nucleic acid and control nucleic acid from the fetus of a pregnant woman by obtaining an nucleic acid-containing biological sample from the pregnant woman, wherein the biological sample contains fetal nucleic acid, partially or wholly separating the fetal nucleic acid from the maternal nucleic acid based on differential methylation, discriminating the alleles from the target nucleic acid and the control nucleic acid, followed by determination of the ratio of the alleles, and detecting the presence or absence of a chromosomal disorder in the fetus based on the ratio of alleles, wherein a ratio above or below a normal, euploid ratio is indicative of a chromosomal disorder. The target nucleic acid may be from a suspected aneuploid chromosome (e.g., chromosome 21) and the control nucleic acid may be from a euploid chromosome from the same fetus.

The present technology may be combined with other fetal markers to detect the presence or absence of multiple chromosomal abnormalities, wherein the chromosomal abnormalities are selected from the following: trisomy 21, trisomy 18 and trisomy 13, or combinations thereof. The chromosomal disorder may involve the X chromosome or the Y chromosome.

The compositions or processes may be multiplexed in a single reaction. For example, the amount of fetal nucleic acid may be determined at multiple loci across the genome. Or when detecting the presence or absence of fetal aneuploidy, the amount of fetal nucleic acid may be determined at multiple loci on one or more target chromosomes (e.g., chromosomes 13, 18 or 21) and on one or more reference chromosomes. If an allelic ratio is being used, one or more alleles from Table 2 can be detected and discriminated simultaneously. When determining allelic ratios, multiplexing methods are particularly important when the genotype at a polymorphic locus is not known. In some instances, for example when the mother and child are homozygous at the polymorphic locus, the assay may not be informative. Greater than 1, 2, 3, 4, 5, 6, 7, 8, 9, 10, 11, 12, 13, 14, 15, 16, 17, 18, 19, 20, 25, 30, 35, 40, 50, 100, 200, 300 or 500, and any intermediate levels, polynucleotide sequences of the technology are enriched, separated and/or examined according the methods of the technology. When detecting a chromosomal abnormality by analyzing the copy number of target nucleic acid and control nucleic acid, less than 3, 4, 5, 6, 7, 8, 9, 10, 11, 12, 13, or 14 polynucleotide sequences may need to be analyzed to accurately detect the presence or absence of a chromosomal abnormality. The compositions or processes of the technology may be used to assay samples that have been divided into 2, 3, 4, 5, 6, 7, 8, 9, 10, 11, 12, 13, 14, 15, 16, 17, 18, 19, 20, 25, 30, 35, 40, 50, 100 or more replicates, or into single molecule equivalents. Methods for analyzing fetal nucleic acids from a maternal sample in replicates, including single molecule analyses, are provided in US Application No, 11/364,294, which published as US Patent Publication No. US 2007-0207466 A1.

Also described herein is a method wherein a comparison step shows an increased risk of a fetus having a chromosomal disorder if the ratio of the alleles or absolute copy number of the target nucleic acid is higher or lower by 1 standard deviation from the standard control sequence. The comparison step may show an increased risk of a fetus having a chromosomal disorder if the ratio of the alleles or absolute copy number of the target nucleic acid is higher or lower by 2 standard deviation from the standard control sequence. The comparison step may show an increased risk of a fetus having a chromosomal disorder if the ratio of the alleles or absolute copy number of the target nucleic acid is higher or lower by 3 standard deviation from the standard control sequence. The comparison step shows an increased risk of a fetus having a chromosomal disorder if the ratio of the alleles or absolute copy number of the target nucleic acid is higher or lower than a statistically significant standard deviation from the control. The standard control may a maternal reference, and alternatively the standard control may be a fetal reference chromosome (e.g., non-trisomic autosome).

The methods of the technology may be combined with other methods for diagnosing a chromosomal abnormality. For example, a noninvasive diagnostic method may require confirmation of the presence or absence of fetal nucleic acid, such as a sex test for a female fetus or to confirm an RhD negative female fetus in an RhD negative mother. The compositions and methods of the technology may be used to determine the percentage of fetal nucleic acid in a maternal sample in order to enable another diagnostic method that requires the percentage of fetal nucleic acid be known. For example, does a sample meet certain threshold concentration requirements? When determining an allelic ratio to diagnose a fetal aneuploidy from a maternal sample, the amount or concentration of fetal nucleic acid may be required to make a diagnose with a given sensitivity and specificity. The compositions and methods of the technology for detecting a chromosomal abnormality may be combined with other known methods thereby improving the overall sensitivity and specificity of the detection method. For example, mathematical models have suggested that a combined first-trimester screening program utilizing maternal age (MA), nuchal translucency (NT) thickness, serum-free beta-hCG, and serum PAPP-A will detect more than 80% of fetuses with Down's syndrome for a 5% invasive testing rate (Wald and Hackshaw, Prenat Diagn 17(9):921-9 (1997)). However, the combination of commonly used aneuploidy detection methods combined with the non-invasive free fetal nucleic acid-based methods described herein may offer improved accuracy with a lower false positive rate. Examples of combined diagnostic methods are provided in PCT Publication Number WO2008157264A2 (assigned to the Applicant). The methods of the technology may be combined with cell-based methods, wherein fetal cells are procured invasively or non-invasively.

An increased risk for a chromosomal abnormality may be based on the outcome or result(s) produced from the compositions or methods provided herein. An example of an outcome is a deviation from the euploid absolute copy number or allelic ratio, which indicates the presence of chromosomal aneuploidy. This increase or decrease in the absolute copy number or ratio from the standard control indicates an increased risk of having a fetus with a chromosomal abnormality (e.g., trisomy 21). Information pertaining to a method described herein, such as an outcome, result, or risk of trisomy or aneuploidy, for example, may be transfixed, renditioned, recorded and/or displayed in any suitable medium. For example, an outcome may be transfixed in a medium to save, store, share, communicate or otherwise analyze the outcome. A medium can be tangible (e.g., paper) or intangible (e.g., electronic medium), and examples of media include, but are not limited to, computer media, databases, charts, patient charts, records, patient records, graphs and tables, and any other medium of expression. The information sometimes is stored and/or renditioned in computer readable form and sometimes is stored and organized in a database. The information may be transferred from one location to another using a physical medium (e.g., paper) or a computer readable medium (e.g., optical and/or magnetic storage or transmission medium, floppy disk, hard disk, random access memory, computer processing unit, facsimile signal, satellite signal, transmission over an internet or transmission over the world-wide web).

A CpG island may be used as the CpG-containing genomic sequence in some cases, whereas in other cases the CpG-containing genomic sequence may not be a CpG island. Also described herein is a kit for performing the methods of the technology. One component of the kit is a methylation-sensitive binding agent.

### BRIEF DESCRIPTION OF THE DRAWINGS

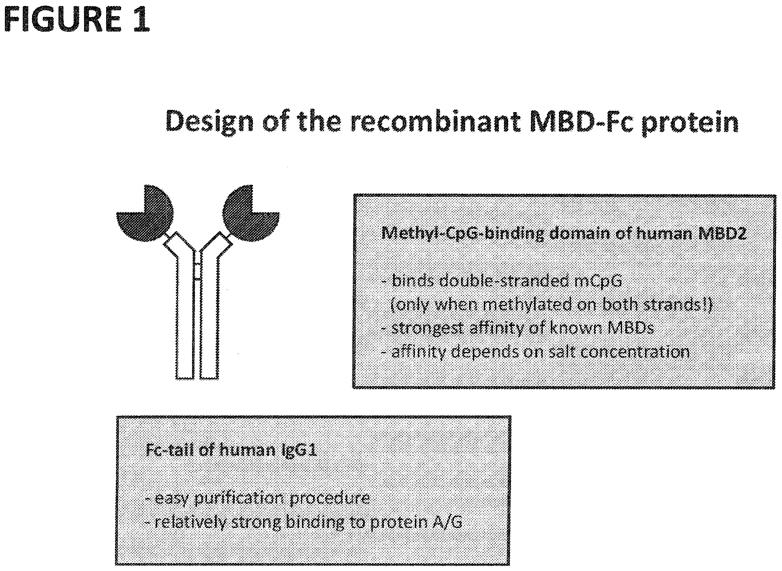

FIGURE 1: Shows the design of the recombinant MBD-Fc protein used to separate differentially methylated DNA.

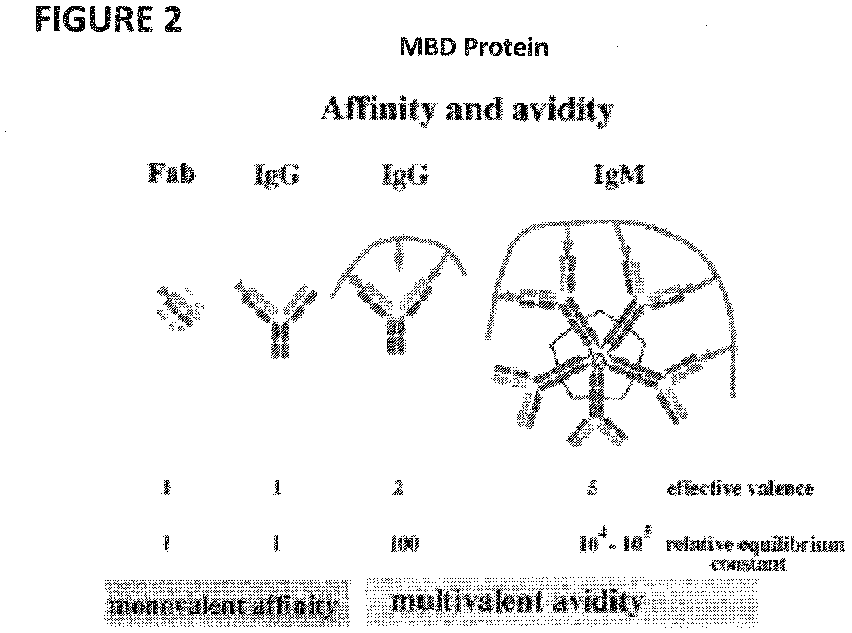

FIGURE 2: Shows the methyl-CpG-binding, antibody-like protein has a high affinity and high avidity to its "antigen", which is preferably DNA that is methylated at CpG di-nucleotides.

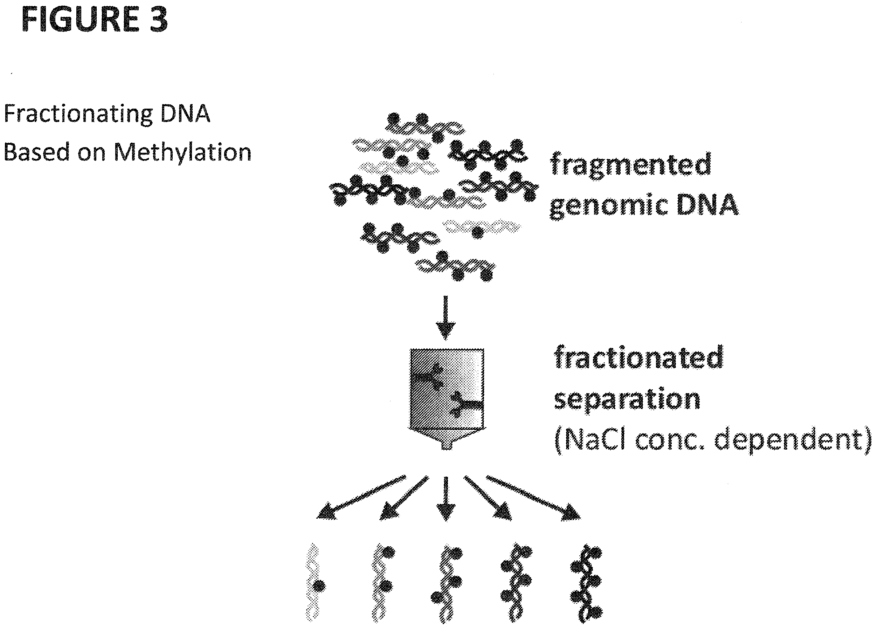

FIGURE 3: Shows the methyl binding domain of MBD-FC binds DNA molecules regardless of their methylation status. The strength of this protein/DNA interaction is defined by the level of DNA methylation. After binding genomic DNA, eluate solutions of increasing salt concentrations can be used to fractionate non-methylated and methylated DNA allowing for a controlled separation.

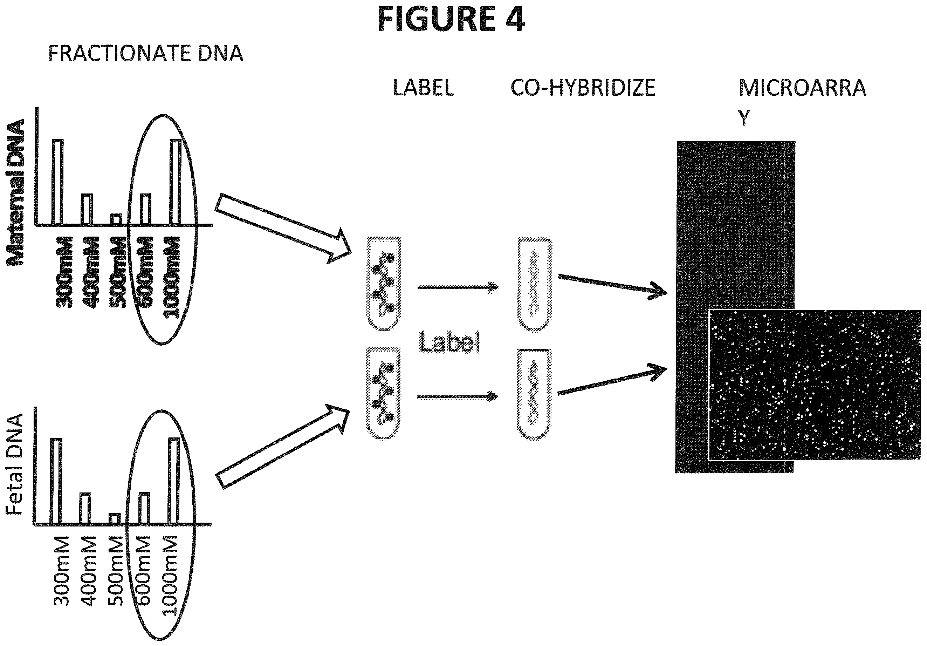

FIGURE 4: Shows the experiment used to identify differentially methylated DNA from a fetus and mother using the recombinant MBD-Fc protein and a microarray.

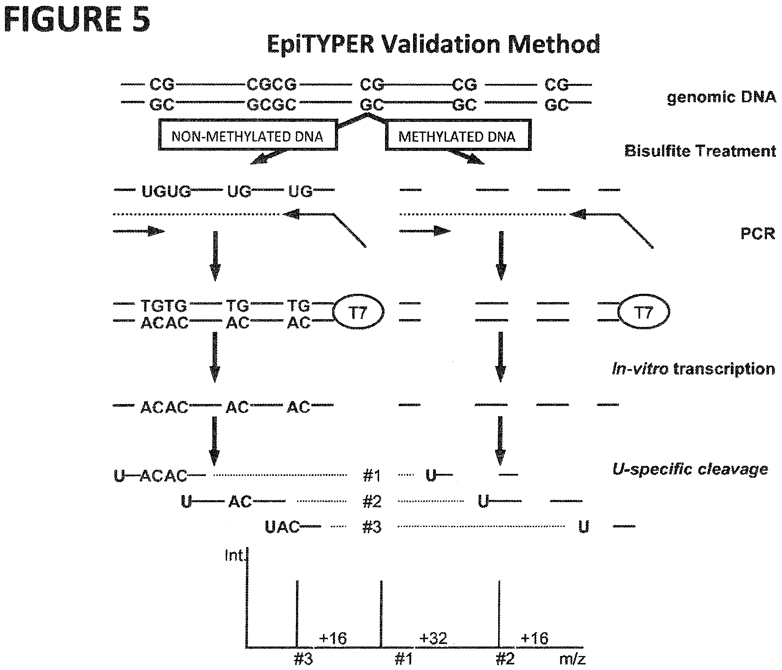

FIGURE 5: Shows typical results generated by Sequenom® EpiTYPER™ method, which was used to validate the results generated from the experiment illustrated in Figure 4.

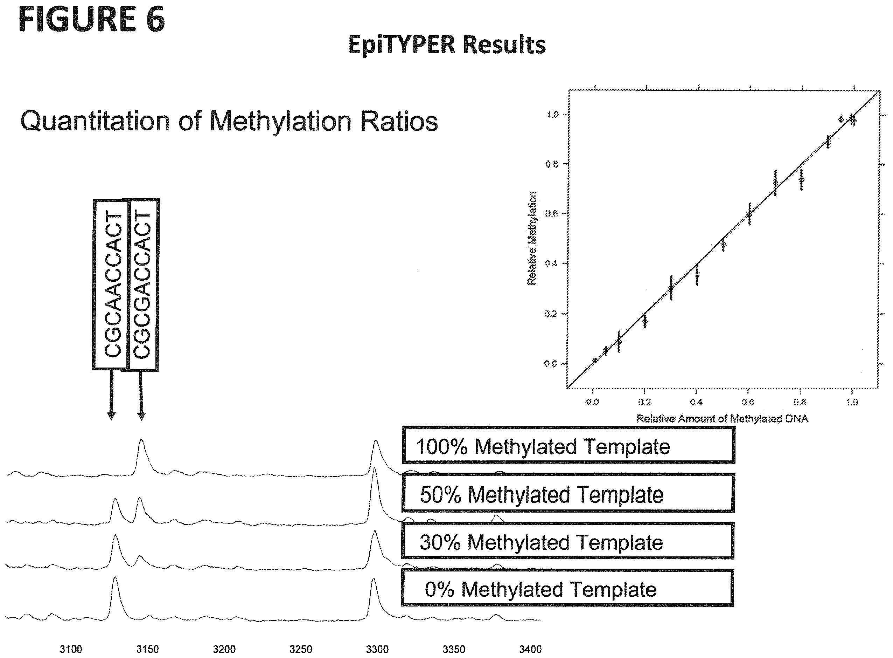

FIGURE 6: Shows the correlation between the log ratios derived from microarray analysis (x axis) and methylation differences obtained by EpiTYPER analysis (y axis). Each data point represents the average for one region across all measured samples. The microarray analysis is comparative in nature because the highly methylated fraction of the maternal DNA is hybridized together with the highly methylated fraction of placenta DNA. Positive values indicate higher methylation of the placenta samples. In mass spectrometry each samples is measured individually. We first calculated difference in methylation by subtracting the maternal methylation values from the placenta methylation value. To compare the results with the microarray data we calculated the average of the differences for all maternal / placenta DNA pairs.

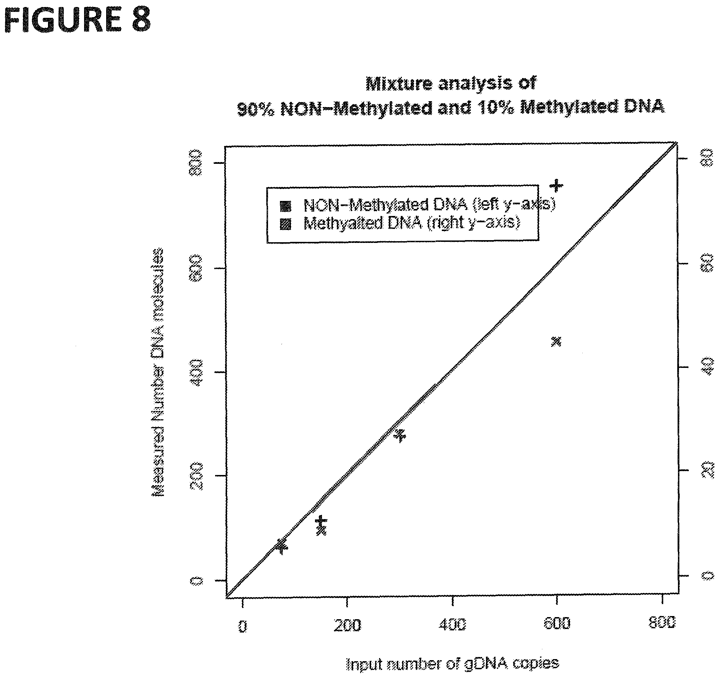

FIGURE 8: Shown is the correlation between the number of gDNA molecules that were expected and the number of molecules measured by competitive PCR in combination with mass spectrometry analysis. In this experiment we used DNA derived from whole blood (black plus signs) and commercially available fully methylated DNA(red crosses) in a 90 to 10 ratio. We used the MBD-FC fusion protein to separate the non-methylated and the methylated fraction of DNA. Each fraction was subject to competitive PCR analysis with mass spectrometry readout. The method has been described earlier for the analysis of copy number variations and is commercially available for gene expression analysis. The approach allows absolute quantification of DNA molecules with the help of a synthetic oligonucleotides of know concentration. In this experiment we targeted the MGMT locus, which was not methylated in the whole blood sample used here. Using an input of 300 total gDNA copies we expect to see 270 copies of non-methylated DNA and 30 copies of methylated DNA. The measured copy numbers are largely in agreement with the expected values. The data point at 600 copies of input DNA indicates a bias in the reaction and shows that this initial proof of concept experiment needs to be followed up with more development work, before the assay can be used. However, this initial data indicates the feasibility of the approach for capturing and quantifying of a few copies of methylated DNA in the presence of an excess of unmethylated DNA species.

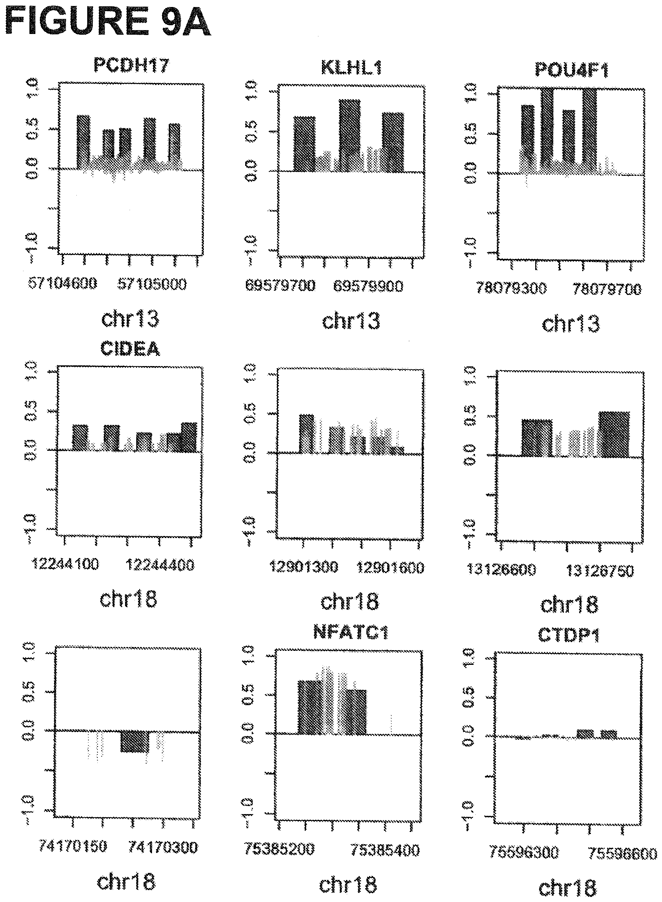

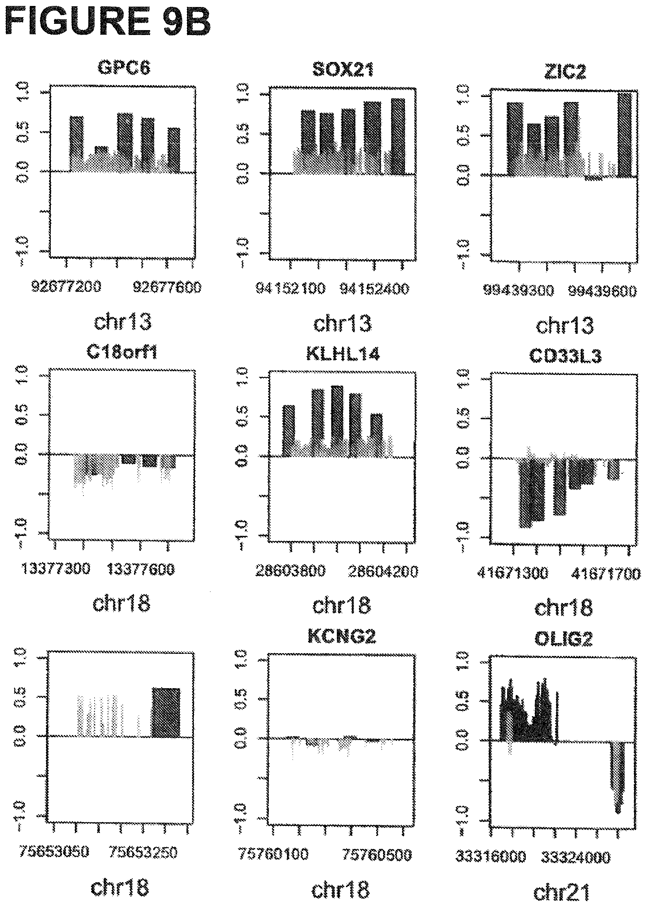

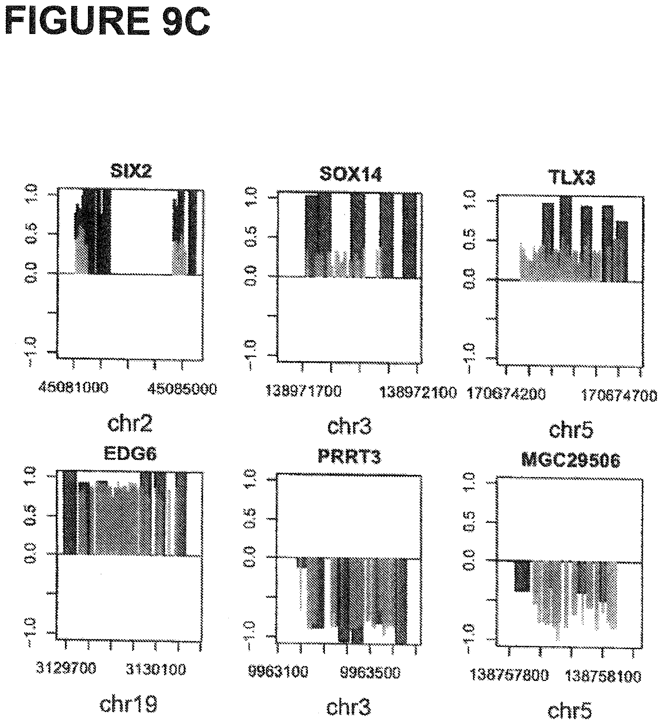

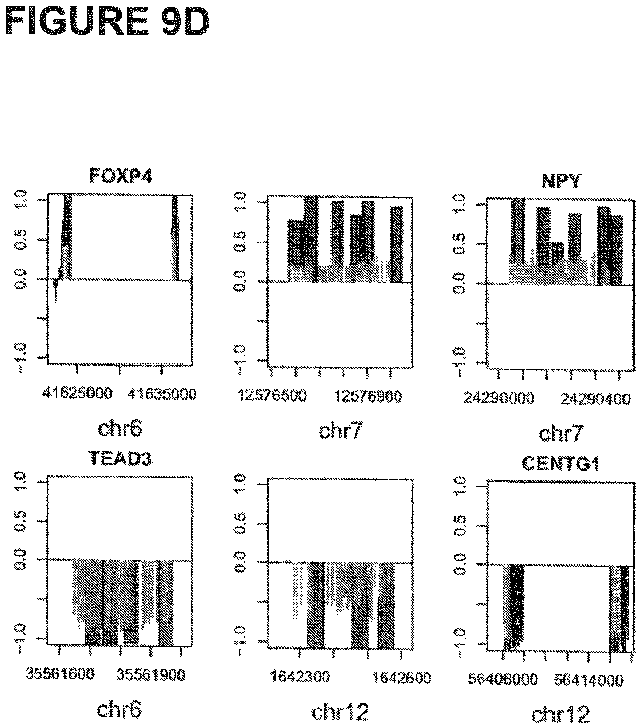

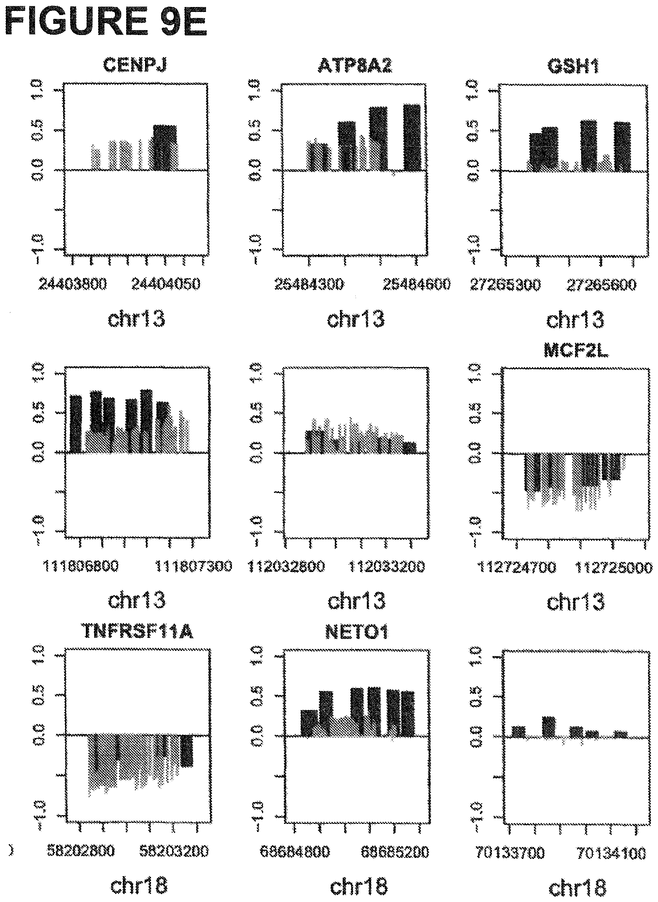

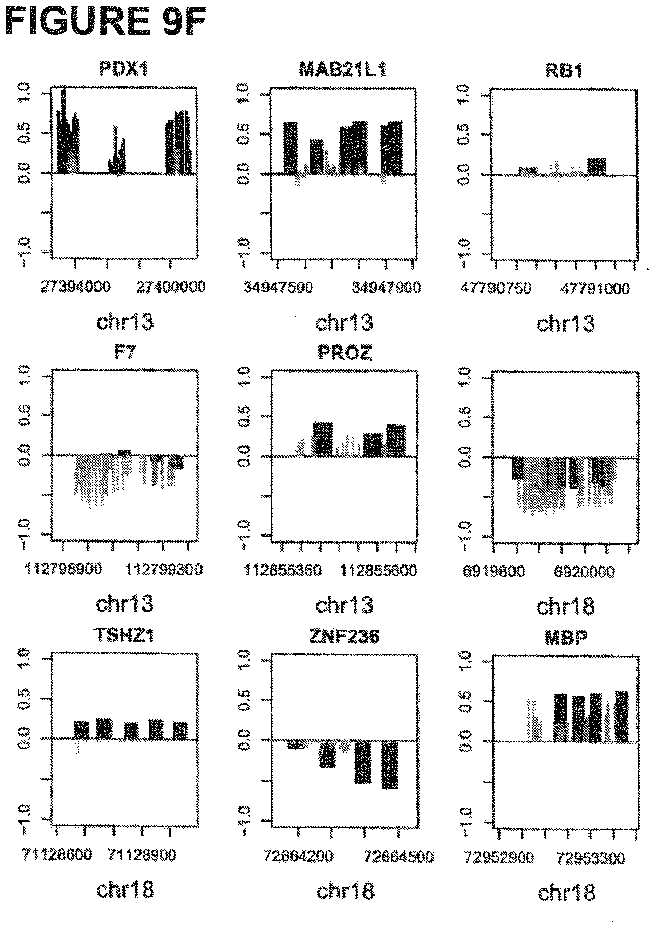

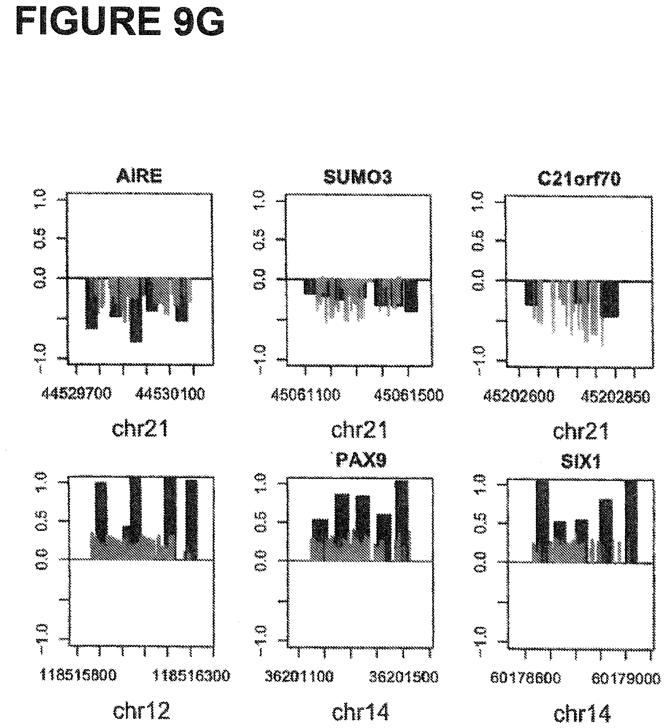

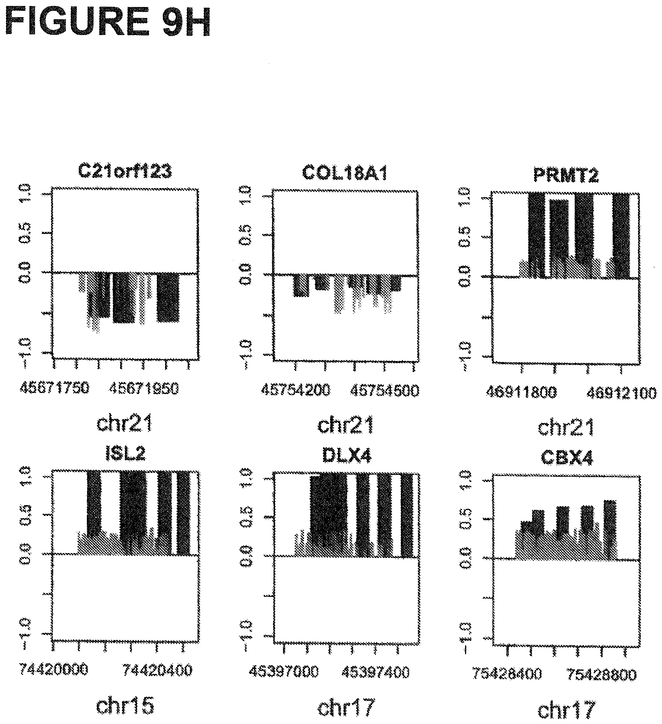

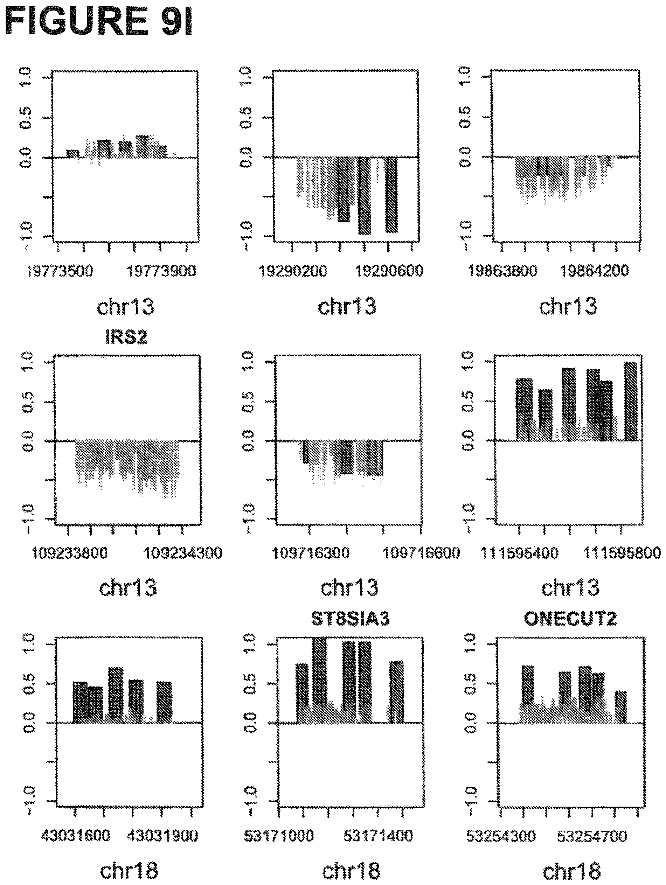

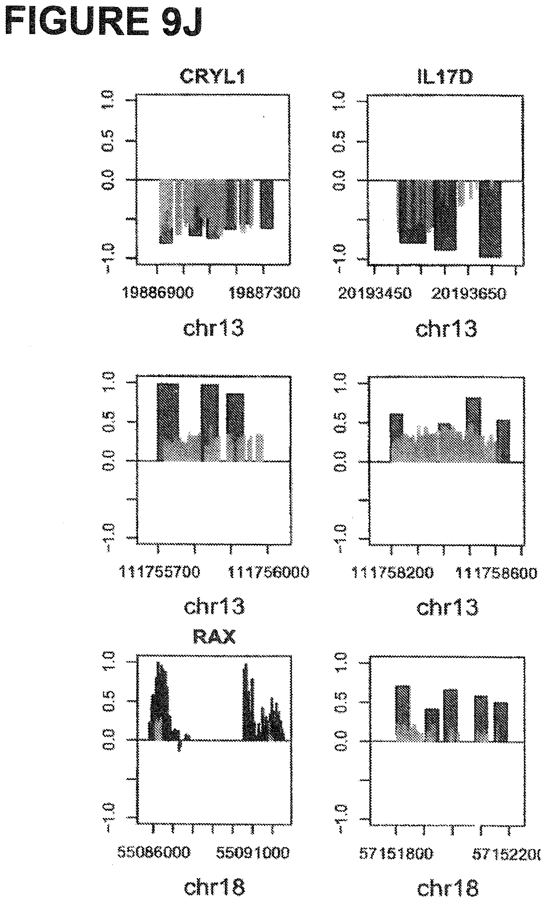

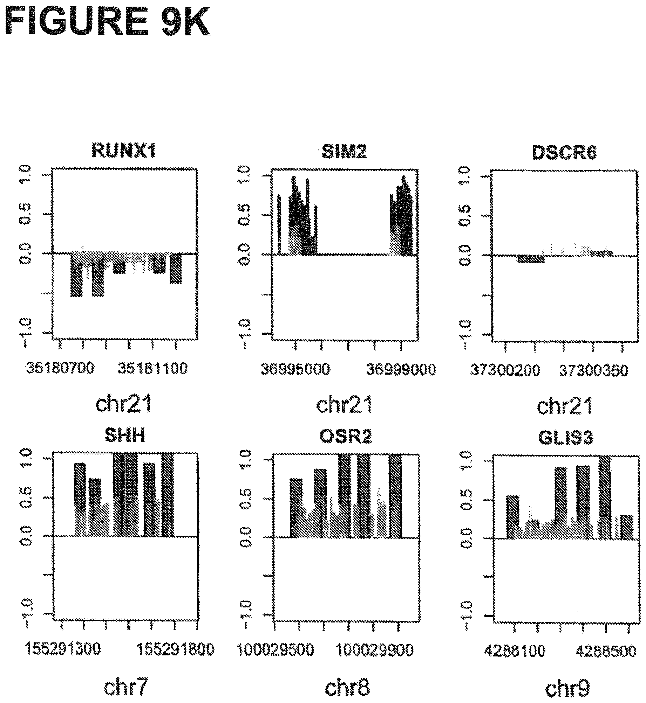

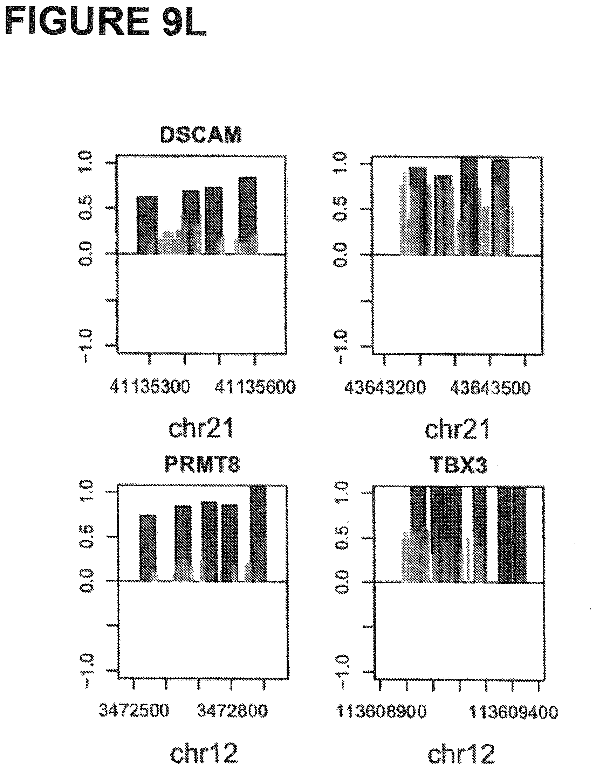

FIGURE 9A-9C: Shown are bar graph plots of the methylation differences obtained from the microarray analysis (dark bars) and the mass spectrometry analysis (light grey bars) with respect to their genomic location. For each of the 85 region that were identified to be differentially methylated by microarray an individual plot is provided. The x axis for each plot shows the chromosomal position of the region. The y axis depicts the log ration (in case of the microarrays) and the methylation differences (in case of the mass spectrometry results). For the microarrays each hybridization probe in the area is shown as a single black (or dark grey) bar. For the mass spectrometry results each CpG site, is shown as a light grey bar. Bars showing values greater than zero indicate higher DNA methylation in the placenta samples compared to the maternal DNA. For some genes the differences are small (i.e. RB1 or DSCR6) but still statistically significant. Those regions would be less suitable for a fetal DNA enrichment strategy.

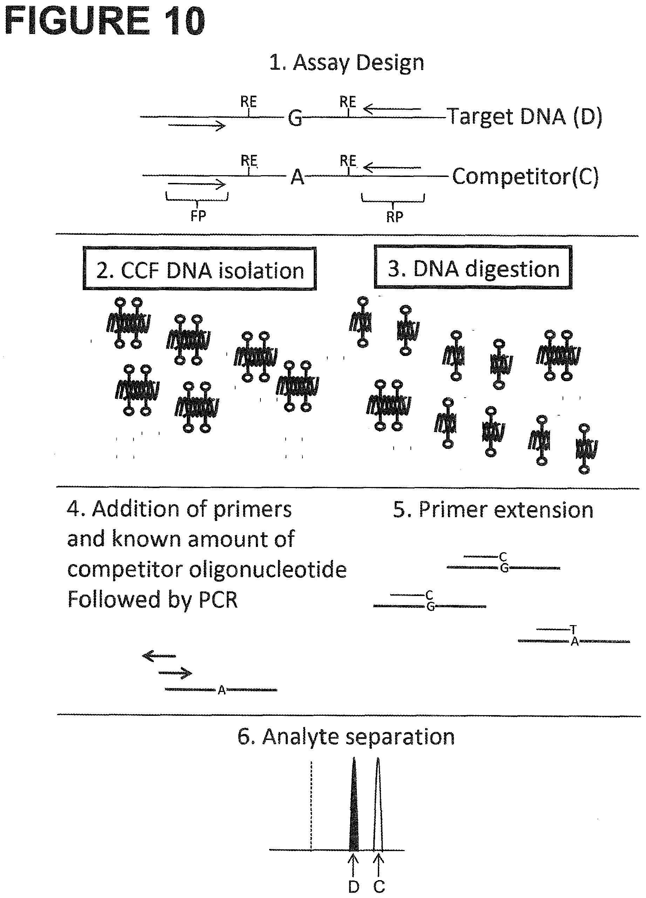

FIGURE 10: Shows one example of the Fetal Quantifier Method. Maternal nucleic acid is selectively digested and the remaining fetal nucleic acid is quantified using a competitor of known concentration. In this schema, the analyte is separated and quantified by a mass spectromter.

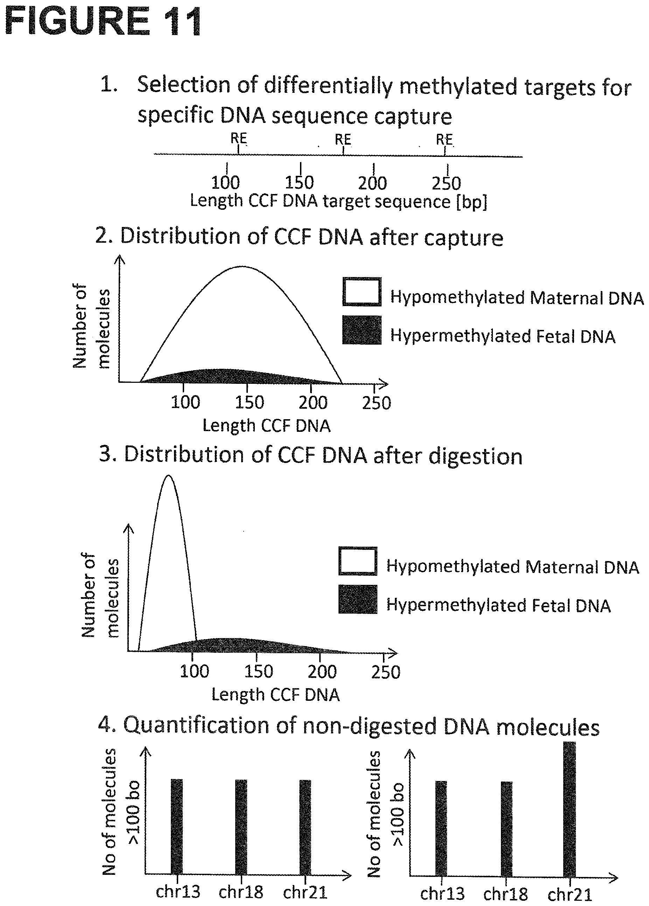

FIGURE 11: Shows one example of the Methylation-Based Fetal Diagnostic Method. Maternal nucleic acid is selectively digested and the remaining fetal nucleic acid is quantified for three different chromosomes (13, 18 and 21). Parts 2 and 3 of the Figure illustrate the size distribution of the nucleic acid in the sample before and after digestion. The amplification reactions can be size-specific (e.g., greater than 100 base pair amplicons) such that they favor the longer, non-digested fetal nucleic acid over the digested maternal nucleic acid, thereby further enriching the fetal nucleic acid. The spectra at the bottom of the Figure show an increased amount of chromosome 21 fetal nucleic acid indicative of trisomy 21.

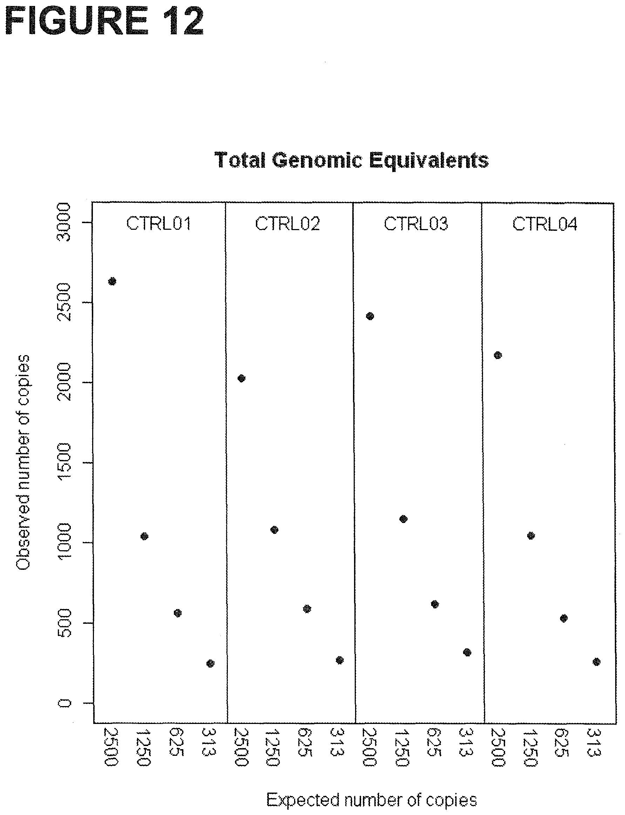

FIGURE 12: Shows the total number of amplifiable genomic copies from four different DNA samples isolated from the blood of non-pregnant women. Each sample was diluted to contain approximately 2500, 1250, 625 or 313 copies per reaction. Each measurement was obtained by taking the mean DNA/competitor ratio obtained from two total copy number assays (ALB and RNAseP in Table X). As Figure 12 shows, the total copy number is accurate and stable across the different samples, thus validating the usefulness of the competitor-based approach.

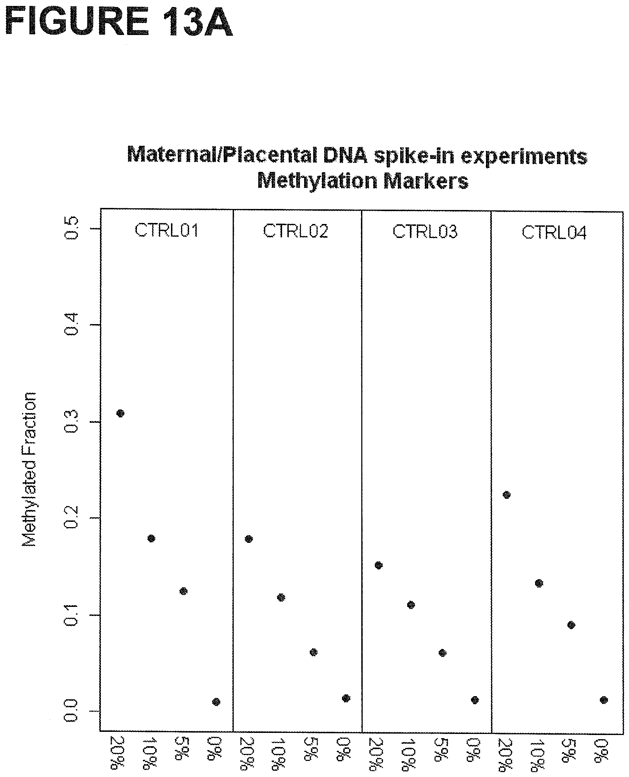

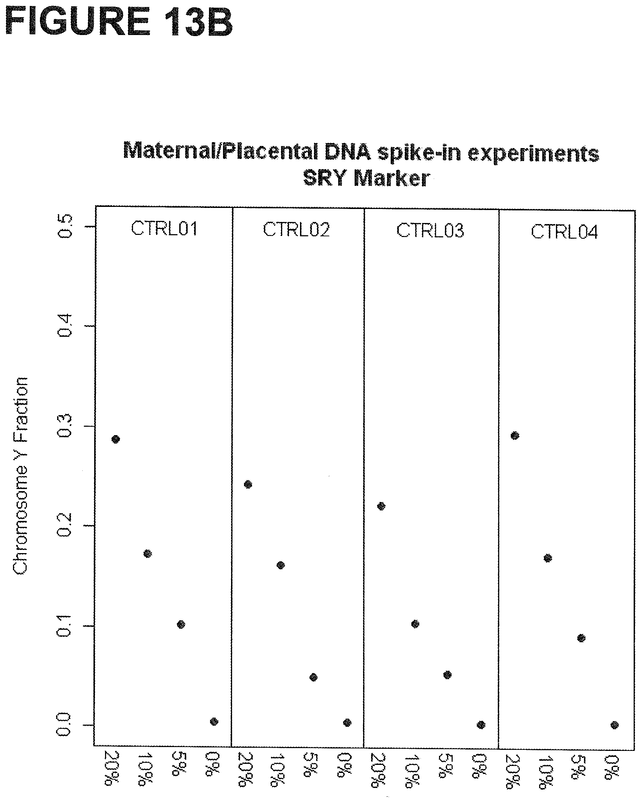

FIGURES 13A and B: A model system was created that contained a constant number of maternal non-methylated DNA with varying amounts of male placental methylated DNA spiked-in. The samples were spiked with male placental amounts ranging from approximately 0 to 25% relative to the maternal non-methylated DNA. The fraction of placental DNA was calculated using the ratios obtained from the methylation assays (Figure 13A) and the Y-chromosome marker (Figure 13B) as compared to the total copy number assay. The methylation and Y-chromosome markers are provided in Table X.

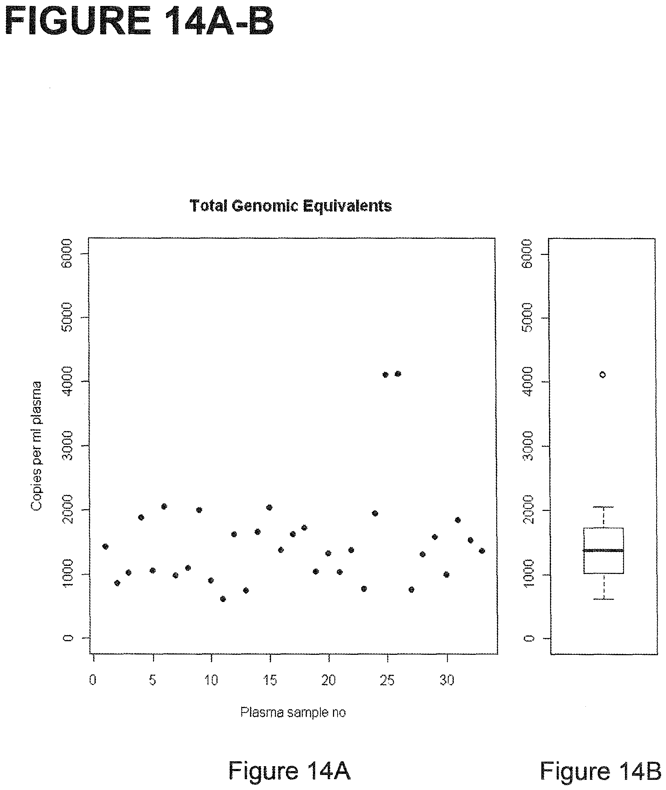

FIGURES 14A and B: Show the results of the total copy number assay from plasma samples. In Figure 14A, the copy number for each sample is shown. Two samples (no 25 and 26) have a significantly higher total copy number than all the other samples. A mean of approximately 1300 amplifiable copies/ml plasma was obtained (range 766-2055). Figure 14B shows a box-and-whisker plot of the given values, summarizing the results.

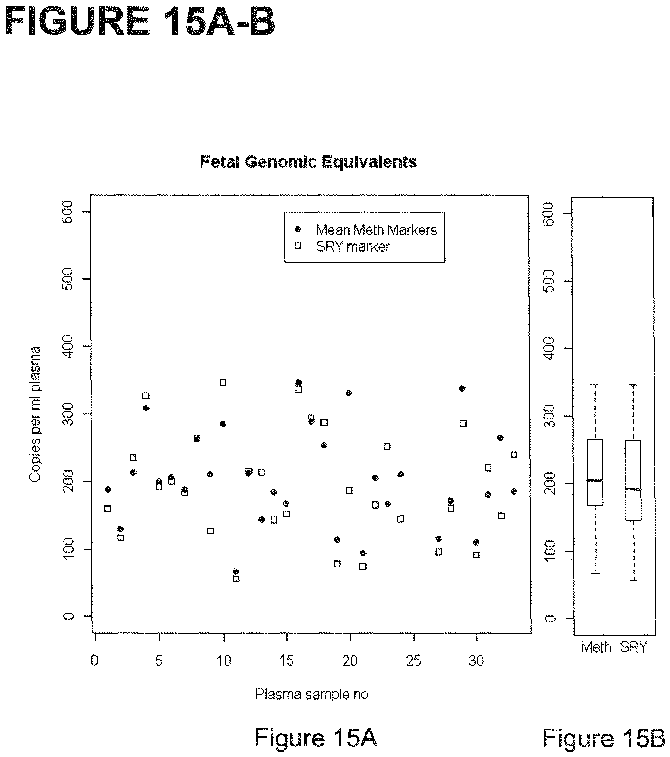

FIGURES 15A and B: The amount (or copy numbers) of fetal nucleic acid from 33 different plasma samples taken from pregnant women with male fetuses are plotted. The copy numbers obtained were calculated using the methylation markers and the Y-chromosome-specific markers using the assays provided in Table X. As can be seen in Figure 15B, the box-and-whisker plot of the given values indicated minimal difference between the two different measurements, thus validating the accuracy and stability of the method.

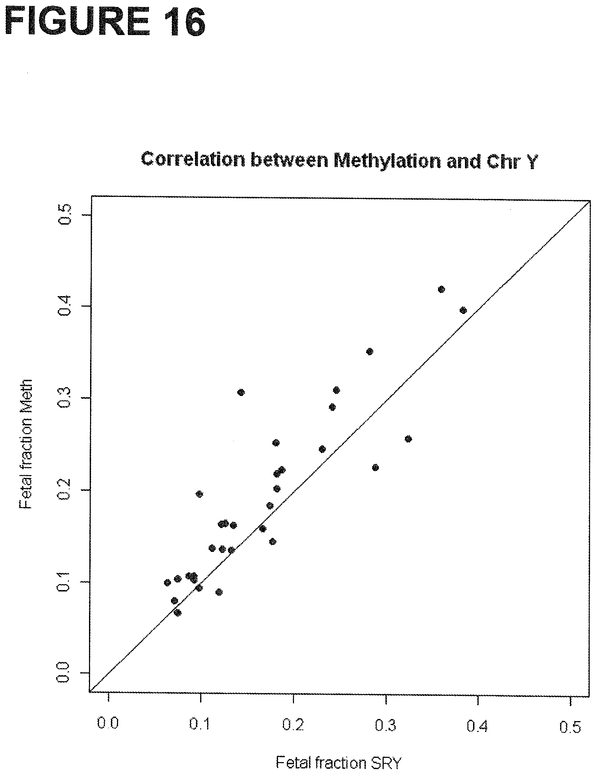

FIGURE 16: Shows a paired correlation between the results obtained using the methylation markers versus the Y-chromosome marker from Figure 15A.

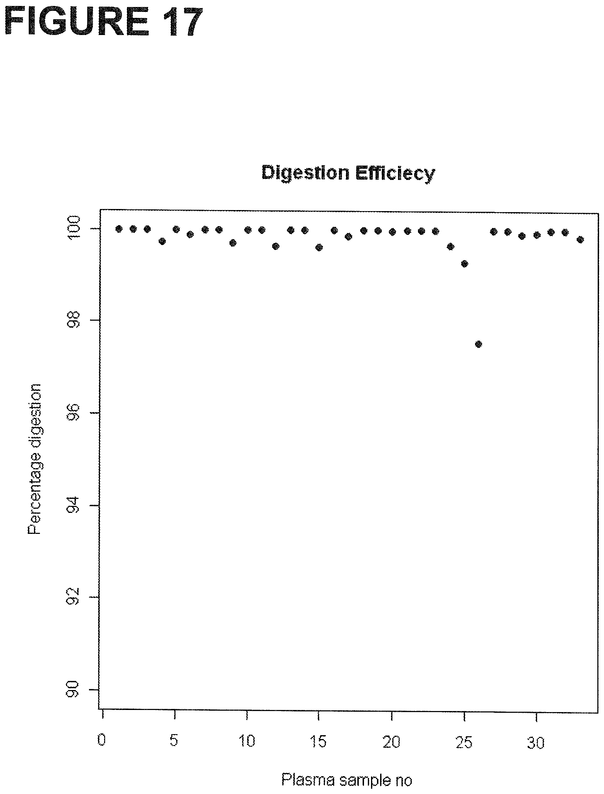

FIGURE 17: Shows the digestion efficiency of the restriction enzymes using the ratio of digestion for the control versus the competitor and comparing this value to the mean total copy number assays. Apart from sample 26 all reactions indicate the efficiency to be above about 99%.

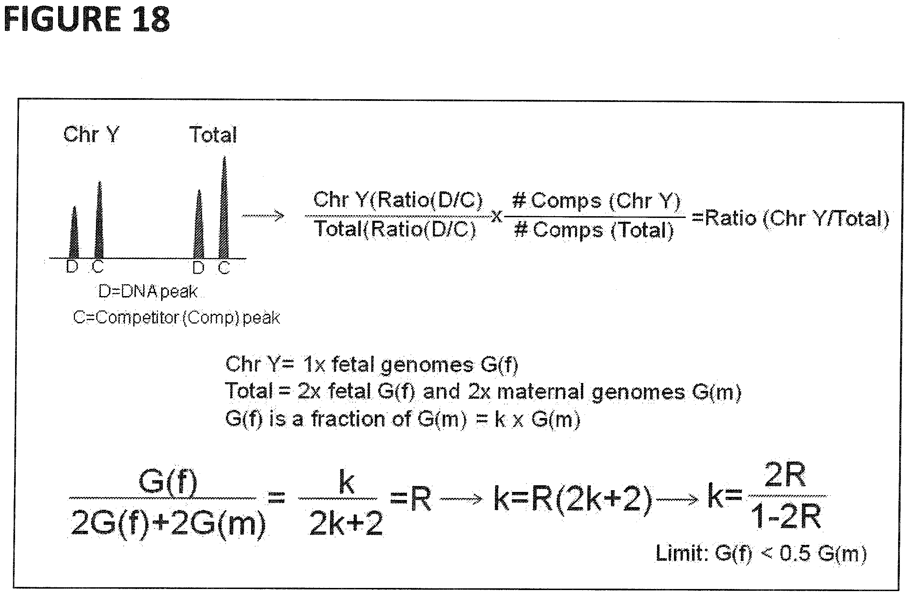

FIGURE 18: Provides a specific method for calculating fetal DNA fraction (or concentration) in a sample using the Y-chromosome-specific markers for male pregnancies and the mean of the methylated fraction for all pregnancies (regardless of fetal sex).

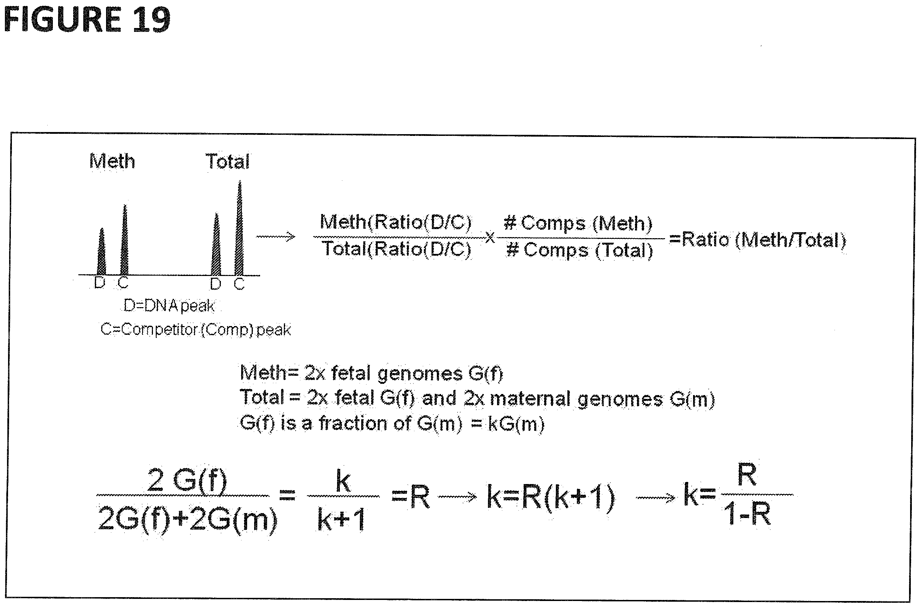

FIGURE 19: Provides a specific method for calculating fetal DNA fraction (or concentration) in a sample without the Y-chromosome-specific markers. Instead, only the Assays for Methylation Quantification were used to determine the concentration of fetal DNA.

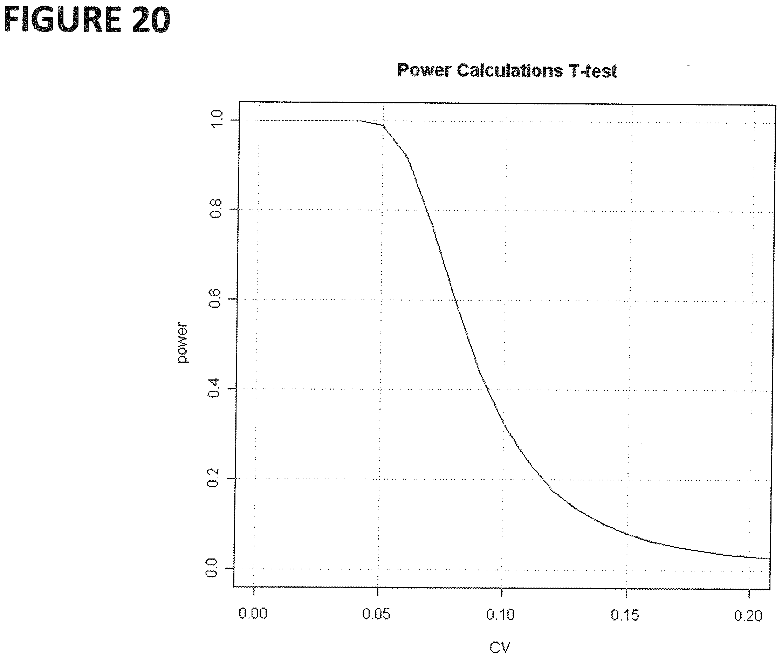

FIGURE 20: Shows a power calculation t-test for a simulated trisomy 21 diagnosis using the methods of the technology. The Figure shows the relationship between the coefficient of variation (CV) on the x-axis and the power to discriminate the assay populations using a simple t-test (y-axis). The data indicates that in 99% of all cases, one can discriminate the two population (euploid vs. aneuploid) on a significance level of 0.001 provided a CV of 5% or less.

### DEFINITIONS

The term "pregnancy-associated disorder," as used in this application, refers to any condition or disease that may affect a pregnant woman, the fetus, or both the woman and the fetus. Such a condition or disease may manifest its symptoms during a limited time period, e.g., during pregnancy or delivery, or may last the entire life span of the fetus following its birth. Some examples of a pregnancy-associated disorder include ectopic pregnancy, preeclampsia, preterm labor, RhD incompatibility, fetal chromosomal abnormalities such as trisomy 21, and genetically inherited fetal disorders such as cystic fibrosis, beta-thalassemia or other monogenic disorders. The ability to enrich fetal nucleic from a maternal sample may prove particularly useful for the noninvasive prenatal diagnosis of autosomal recessive diseases such as the case when a mother and father share an identical disease causing mutation, an occurrence previously perceived as a challenge for maternal plasma-based non-trisomy prenatal diagnosis.

The term "chromosomal abnormality" or "aneuploidy" as used herein refers to a deviation between the structure of the subject chromosome and a normal homologous chromosome. The term "normal" refers to the predominate karyotype or banding pattern found in healthy individuals of a particular species, for example, a euploid genome (in humans, 46XX or 46XY). A chromosomal abnormality can be numerical or structural, and includes but is not limited to aneuploidy, polyploidy, inversion, a trisomy, a monosomy, duplication, deletion, deletion of a part of a chromosome, addition, addition of a part of chromosome, insertion, a fragment of a chromosome, a region of a chromosome, chromosomal rearrangement, and translocation. Chromosomal abnormality may also refer to a state of chromosomal abnormality where a portion of one or more chromosomes is not an exact multiple of the usual haploid number due to, for example, chromosome translocation. Chromosomal translocation (e.g. translocation between chromosome 21 and 14 where some of the 14th chromosome is replaced by extra 21st chromosome) may cause partial trisomy 21. A chromosomal abnormality can be correlated with presence of a pathological condition or with a predisposition to develop a pathological condition. A chromosomal abnormality may be detected by quantitative analysis of nucleic acid.

The terms "nucleic acid" and "nucleic acid molecule" may be used interchangeably throughout the disclosure. The terms refer to nucleic acids of any composition from, such as DNA (e.g., complementary DNA (cDNA), genomic DNA (gDNA) and the like), RNA (e.g., message RNA (mRNA), short inhibitory RNA (siRNA), ribosomal RNA (rRNA), tRNA, microRNA, RNA highly expressed by the fetus or placenta, and the like), and/or DNA or RNA analogs (e.g., containing base analogs, sugar analogs and/or a non-native backbone and the like), RNA/DNA hybrids and polyamide nucleic acids (PNAs), all of which can be in single-or double-stranded form, and unless otherwise limited, can encompass known analogs of natural nucleotides that can function in a similar manner as naturally occurring nucleotides. For example, the nucleic acids provided in SEQ ID Nos: 1-89 (see Table 4) can be in any form useful for conducting processes herein (e.g., linear, circular, supercoiled, single-stranded, double-stranded and the like) or may include variations (e.g., insertions, deletions or substitutions) that do not alter their utility as part of the present technology. A nucleic acid may be, or may be from, a plasmid, phage, autonomously replicating sequence (ARS), centromere, artificial chromosome, chromosome, or other nucleic acid able to replicate or be replicated in vitro or in a host cell, a cell, a cell nucleus or cytoplasm of a cell. A template nucleic acid can be from a single chromosome (e.g., a nucleic acid sample may be from one chromosome of a sample obtained from a diploid organism). Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). The term nucleic acid is used interchangeably with locus, gene, cDNA, and mRNA encoded by a gene. The term also may include, as equivalents, derivatives, variants and analogs of RNA or DNA synthesized from nucleotide analogs, single-stranded ("sense" or "antisense", "plus" strand or "minus" strand, "forward" reading frame or "reverse" reading frame) and double-stranded polynucleotides. Deoxyribonucleotides include deoxyadenosine, deoxycytidine, deoxyguanosine and deoxythymidine. For RNA, the base cytosine is replaced with uracil. A template nucleic acid may be prepared using a nucleic acid obtained from a subject as a template.

A "nucleic acid comprising one or more CpG sites" or a "CpG-containing genomic sequence" as used herein refers to a segment of DNA sequence at a defined location in the genome of an individual such as a human fetus or a pregnant woman. Typically, a "CpG-containing genomic sequence" is at least 15 nucleotides in length and contains at least one cytosine. Preferably, it can be at least 30, 50, 80, 100, 150, 200, 250, or 300 nucleotides in length and contains at least 2, 5, 10, 15, 20, 25, or 30 cytosines. For anyone "CpG-containing genomic sequence" at a given location, e.g., within a region centering around a given genetic locus (see Table 1), nucleotide sequence variations may exist from individual to individual and from allele to allele even for the same individual. Typically, such a region centering around a defined genetic locus (e.g., a CpG island) contains the locus as well as upstream and/or downstream sequences. Each of the upstream or downstream sequence (counting from the 5' or 3' boundary of the genetic locus, respectively) can be as long as 10 kb, in other cases may be as long as 5 kb, 2 kb, 1 kb, 500 bp, 200 bp, or 100 bp. Furthermore, a "CpG-containing genomic sequence" may encompass a nucleotide sequence transcribed or not transcribed for protein production, and the nucleotide sequence can be an inter-gene sequence, intra-gene sequence, protein-coding sequence, a non protein-coding sequence (such as a transcription promoter), or a combination thereof.

As used herein, a "methylated nucleotide" or a "methylated nucleotide base" refers to the presence of a methyl moiety on a nucleotide base, where the methyl moiety is not present in a recognized typical nucleotide base. For example, cytosine does not contain a methyl moiety on its pyrimidine ring, but 5-methylcytosine contains a methyl moiety at position 5 of its pyrimidine ring. Therefore, cytosine is not a methylated nucleotide and 5-methylcytosine is a methylated nucleotide. In another example, thymine contains a methyl moiety at position 5 of its pyrimidine ring, however, for purposes herein, thymine is not considered a methylated nucleotide when present in DNA since thymine is a typical nucleotide base of DNA. Typical nucleoside bases for DNA are thymine, adenine, cytosine and guanine. Typical bases for RNA are uracil, adenine, cytosine and guanine. Correspondingly a "methylation site" is the location in the target gene nucleic acid region where methylation has, or has the possibility of occurring. For example a location containing CpG is a methylation site wherein the cytosine may or may not be methylated.

As used herein, a "CpG site" or "methylation site" is a nucleotide within a nucleic acid that is susceptible to methylation either by natural occurring events in vivo or by an event instituted to chemically methylate the nucleotide in vitro.

As used herein, a "methylated nucleic acid molecule" refers to a nucleic acid molecule that contains one or more methylated nucleotides that is/are methylated.

A "CpG island" as used herein describes a segment of DNA sequence that comprises a functionally or structurally deviated CpG density. For example, Yamada et al. (Genome Research 14:247-266, 2004) have described a set of standards for determining a CpG island: it must be at least 400 nucleotides in length, has a greater than 50% GC content, and an OCF/ECF ratio greater than 0.6. Others (Takai et al., Proc. Natl. Acad. Sci. U.S.A. 99:3740-3745, 2002) have defined a CpG island less stringently as a sequence at least 200 nucleotides in length, having a greater than 50% GC content, and an OCF/ECF ratio greater than 0.6.

The term "epigenetic state" or "epigenetic status" as used herein refers to any structural feature at a molecular level of a nucleic acid (e.g., DNA or RNA) other than the primary nucleotide sequence. For instance, the epigenetic state of a genomic DNA may include its secondary or tertiary structure determined or influenced by, e.g., its methylation pattern or its association with cellular proteins.

The term "methylation profile" "methylation state" or "methylation status," as used herein to describe the state of methylation of a genomic sequence, refers to the characteristics of a DNA segment at a particular genomic locus relevant to methylation. Such characteristics include, but are not limited to, whether any of the cytosine (C) residues within this DNA sequence are methylated, location of methylated C residue(s), percentage of methylated C at any particular stretch of residues, and allelic differences in methylation due to, e.g., difference in the origin of the alleles. The term "methylation" profile" or "methylation status" also refers to the relative or absolute concentration of methylated C or unmethylated C at any particular stretch of residues in a biological sample. For example, if the cytosine (C) residue(s) within a DNA sequence are methylated it may be referred to as "hypermethylated"; whereas if the cytosine (C) residue(s) within a DNA sequence are not methylated it may be referred to as "hypomethylated". Likewise, if the cytosine (C) residue(s) within a DNA sequence (e.g., fetal nucleic acid) are methylated as compared to another sequence from a different region or from a different individual (e.g., relative to maternal nucleic acid), that sequence is considered hypermethylated compared to the other sequence. Alternatively, if the cytosine (C) residue(s) within a DNA sequence are not methylated as compared to another sequence from a different region or from a different individual (e.g., the mother), that sequence is considered hypomethylated compared to the other sequence. These sequences are said to be "differentially methylated", and more specifically, when the methylation status differs between mother and fetus, the sequences are considered "differentially methylated maternal and fetal nucleic acid".

The term "agent that binds to methylated nucleotides" as used herein refers to a substance that is capable of binding to methylated nucleic acid. The agent may be naturally-occurring or synthetic, and may be modified or unmodified. The agent may allow for the separation of different nucleic acid species according to their respective methylation states. An example of an agent that binds to methylated nucleotides is described in WO06056480A2. The described agent is a bifunctional polypeptide comprising the DNA-binding domain of a protein belonging to the family of Methyl-CpG binding proteins (MBDs) and an Fc portion of an antibody (see Figure 1). The recombinant methyl-CpG-binding, antibody-like protein can preferably bind CpG methylated DNA in an antibody-like manner. That means, the methyl-CpG-binding, antibody-like protein has a high affinity and high avidity to its "antigen", which is preferably DNA that is methylated at CpG dinucleotides. The agent may also be a multivalent MBD (see Figure 2).

The term "polymorphism" as used herein refers to a sequence variation within different alleles of the same genomic sequence. A sequence that contains a polymorphism is considered "polymorphic sequence". Detection of one or more polymorphisms allows differentiation of different alleles of a single genomic sequence or between two or more individuals. As used herein, the term "polymorphic marker" or "polymorphic sequence" refers to segments of genomic DNA that exhibit heritable variation in a DNA sequence between individuals. Such markers include, but are not limited to, single nucleotide polymorphisms (SNPs), restriction fragment length polymorphisms (RFLPs), short tandem repeats, such as di-, tri- or tetra-nucleotide repeats (STRs), and the like. Polymorphic markers according to the present technology can be used to specifically differentiate between a maternal and paternal allele in the enriched fetal nucleic acid sample.

The terms "single nucleotide polymorphism" or "SNP" as used herein refer to the polynucleotide sequence variation present at a single nucleotide residue within different alleles of the same genomic sequence. This variation may occur within the coding region or non-coding region (i.e., in the promoter or intronic region) of a genomic sequence, if the genomic sequence is transcribed during protein production. Detection of one or more SNP allows differentiation of different alleles of a single genomic sequence or between two or more individuals.

The term "allele" as used herein is one of several alternate forms of a gene or non-coding regions of DNA that occupy the same position on a chromosome. The term allele can be used to describe DNA from any organism including but not limited to bacteria, viruses, fungi, protozoa, molds, yeasts, plants, humans, non-humans, animals, and archeabacteria.

The terms "ratio of the alleles" or "allelic ratio" as used herein refer to the ratio of the population of one allele and the population of the other allele in a sample. In some trisomic cases, it is possible that a fetus may be tri-allelic for a particular locus. In such cases, the term "ratio of the alleles" refers to the ratio of the population of any one allele against one of the other alleles, or any one allele against the other two alleles.

The term "non-polymorphism-based quantitative method" as used herein refers to a method for determining the amount of an analyte (e.g., total nucleic acid, Y-chromosome nucleic acid, or fetal nucleic acid) that does not require the use of a polymorphic marker or sequence. Although a polymorphism may be present in the sequence, said polymorphism is not required to quantify the sequence. Examples of non-polymorphism-based quantitative methods include, but are not limited to, RT-PCR, digital PCR, array-based methods, sequencing methods, nanopore-based methods, nucleic acid-bound bead-based counting methods and competitor-based methods wherein one or more competitors are introduced at a known concentration(s) to determine the amount of one or more analytes. Some of the above exemplary methods (for example, sequencing) may need to be actively modified or designed such that one or more polymorphisms are not interrogated.

The terms "absolute amount" or "copy number" as used herein refers to the amount or quantity of an analyte (e.g., total nucleic acid or fetal nucleic acid). The present technology provides compositions and processes for determining the absolute amount of fetal nucleic acid in a mixed maternal sample. Absolute amount or copy number represents the number of molecules available for detection, and may be expressed as the genomic equivalents per unit. The term "concentration" refers to the amount or proportion of a substance in a mixture or solution (e.g., the amount of fetal nucleic acid in a maternal sample that comprises a mixture of maternal and fetal nucleic acid). The concentration may be expressed as a percentage, which is used to express how large/small one quantity is, relative to another quantity as a fraction of 100. Platforms for determining the quantity or amount of an analyte (e.g., target nucleic acid) include, but are not limited to, mass spectrometery, digital PCR, sequencing by synthesis platforms (e.g., pyrosequencing), fluorescence spectroscopy and flow cytometry.

The term "sample" as used herein refers to a specimen containing nucleic acid. Examples of samples include, but are not limited to, tissue, bodily fluid (for example, blood, serum, plasma, saliva, urine, tears, peritoneal fluid, ascitic fluid, vaginal secretion, breast fluid, breast milk, lymph fluid, cerebrospinal fluid or mucosa secretion), umbilical cord blood, chorionic villi, amniotic fluid, lymph fluid, cerebrospinal fluid, mucosa secretion, or other body exudate, fecal matter, an individual cell or extract of the such sources that contain the nucleic acid of the same, and subcellular structures such as mitochondria, using protocols well established within the art.

Fetal DNA can be obtained from sources including but not limited to maternal blood, maternal serum, maternal plasma, fetal cells, umbilical cord blood, chorionic villi, amniotic fluid, urine, saliva, lung lavage, cells or tissues.

The term "blood" as used herein refers to a blood sample or preparation from a pregnant woman or a woman being tested for possible pregnancy. The term encompasses whole blood or any fractions of blood, such as serum and plasma as conventionally defined.

The term "bisulfite" as used herein encompasses any suitable types of bisulfites, such as sodium bisulfite, that are capable of chemically converting a cytosine (C) to a uracil (U) without chemically modifying a methylated cytosine and therefore can be used to differentially modify a DNA sequence based on the methylation status of the DNA.

As used herein, a reagent that "differentially modifies" methylated or non-methylated DNA encompasses any reagent that modifies methylated and/or unmethylated DNA in a process through which distinguishable products result from methylated and non-methylated DNA, thereby allowing the identification of the DNA methylation status. Such processes may include, but are not limited to, chemical reactions (such as a C.fwdarw.U conversion by bisulfite) and enzymatic treatment (such as cleavage by a methylation-dependent endonuclease). Thus, an enzyme that preferentially cleaves or digests methylated DNA is one capable of cleaving or digesting a DNA molecule at a much higher efficiency when the DNA is methylated, whereas an enzyme that preferentially cleaves or digests unmethylated DNA exhibits a significantly higher efficiency when the DNA is not methylated.

The terms "non-bisulfite-based method" and "non-bisulfite-based quantitative method" as used herein refer to any method for quantifying methylated or non-methylated nucleic acid that does not require the use of bisulfite. The terms also refer to methods for preparing a nucleic acid to be quantified that do not require bisulfite treatment. Examples of non-bisulfite-based methods include, but are not limited to, methods for digesting nucleic acid using one or more methylation sensitive enzymes and methods for separating nucleic acid using agents that bind nucleic acid based on methylation status.

The terms "methyl-sensitive enzymes" and "methylation sensitive restriction enzymes" are DNA restriction endonucleases that are dependent on the methylation state of their DNA recognition site for activity. For example, there are methyl-sensitive enzymes that cleave or digest at their DNA recognition sequence only if it is not methylated. Thus, an unmethylated DNA sample will be cut into smaller fragments than a methylated DNA sample. Similarly, a hypermethylated DNA sample will not be cleaved. In contrast, there are methyl-sensitive enzymes that cleave at their DNA recognition sequence only if it is methylated. As used herein, the terms "cleave", "cut" and "digest" are used interchangeably.

The term "target nucleic acid" as used herein refers to a nucleic acid examined using the methods disclosed herein to determine if the nucleic acid is part of a pregnancy-related disorder or chromosomal abnormality. For example, a target nucleic acid from chromosome 21 could be examined using the methods of the technology to detect Down's Syndrome.

The term "control nucleic acid" as used herein refers to a nucleic acid used as a reference nucleic acid according to the methods disclosed herein to determine if the nucleic acid is part of a chromosomal abnormality. For example, a control nucleic acid from a chromosome other than chromosome 21 (herein referred to as a "reference chromosome") could be as a reference sequence to detect Down's Syndrome. The control sequence may have a known or predetermined quantity.

The term "sequence-specific" or "locus-specific method" as used herein refers to a method that interrogates (for example, quantifies) nucleic acid at a specific location (or locus) in the genome based on the sequence composition. Sequence-specific or locus-specific methods allow for the quantification of specific regions or chromosomes.

The term "gene" means the segment of DNA involved in producing a polypeptide chain; it includes regions preceding and following the coding region (leader and trailer) involved in the transcription/translation of the gene product and the regulation of the transcription/translation, as well as intervening sequences (introns) between individual coding segments (exons).

In this application, the terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins (i.e., antigens), wherein the amino acid residues are linked by covalent peptide bonds.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, .gamma.-carboxyglutamate, and O-phosphoserine.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

"Primers" as used herein refer to oligonucleotides that can be used in an amplification method, such as a polymerase chain reaction (PCR), to amplify a nucleotide sequence based on the polynucleotide sequence corresponding to a particular genomic sequence, e.g., one located within the CpG island CGI137, PDE9A, or CGI009 on chromosome 21, in various methylation status. At least one of the PCR primers for amplification of a polynucleotide sequence is sequence-specific for the sequence.

The term "template" refers to any nucleic acid molecule that can be used for amplification in the technology. RNA or DNA that is not naturally double stranded can be made into double stranded DNA so as to be used as template DNA. Any double stranded DNA or preparation containing multiple, different double stranded DNA molecules can be used as template DNA to amplify a locus or loci of interest contained in the template DNA.

The term "amplification reaction" as used herein refers to a process for copying nucleic acid one or more times. The method of amplification may include but is not limited to polymerase chain reaction, self-sustained sequence reaction, ligase chain reaction, rapid amplification of cDNA ends, polymerase chain reaction and ligase chain reaction, Q-beta phage amplification, strand displacement amplification, or splice overlap extension polymerase chain reaction. A single molecule of nucleic acid may be amplified, for example, by digital PCR.

The term "sensitivity" as used herein refers to the number of true positives divided by the number of true positives plus the number of false negatives, where sensitivity (sens) may be within the range of 0 ≤ sens ≤ 1. Ideally, methods herein have the number of false negatives equaling zero or close to equaling zero, so that no subject is wrongly identified as not having at least one chromosome abnormality or other genetic disorder when they indeed have at least one chromosome abnormality or other genetic disorder. Conversely, an assessment often is made of the ability of a prediction algorithm to classify negatives correctly, a complementary measurement to sensitivity. The term "specificity" as used herein refers to the number of true negatives divided by the number of true negatives plus the number of false positives, where sensitivity (spec) may be within the range of 0 ≤ spec ≤ 1. Ideally, methods described herein have the number of false positives equaling zero or close to equaling zero, so that no subject wrongly identified as having at least one chromosome abnormality other genetic disorder when they do not have the chromosome abnormality other genetic disorder being assessed. Hence, a method that has sensitivity and specificity equaling one, or 100%, sometimes is selected.

One or more prediction algorithms may be used to determine significance or give meaning to the detection data collected under variable conditions that may be weighed independently of or dependently on each other. The term "variable" as used herein refers to a factor, quantity, or function of an algorithm that has a value or set of values. For example, a variable may be the design of a set of amplified nucleic acid species, the number of sets of amplified nucleic acid species, percent fetal genetic contribution tested, percent maternal genetic contribution tested, type of chromosome abnormality assayed, type of genetic disorder assayed, type of sex-linked abnormalities assayed, the age of the mother and the like. The term "independent" as used .herein refers to not being influenced or not being controlled by another. The term "dependent" as used herein refers to being influenced or controlled by another. For example, a particular chromosome and a trisomy event occurring for that particular chromosome that results in a viable being are variables that are dependent upon each other.

One of skill in the art may use any type of method or prediction algorithm to give significance to the data of the present technology within an acceptable sensitivity and/or specificity. For example, prediction algorithms such as Chi-squared test, z-test, t-test, ANOVA (analysis of variance), regression analysis, neural nets, fuzzy logic, Hidden Markov Models, multiple model state estimation, and the like may be used. One or more methods or prediction algorithms may be determined to give significance to the data having different independent and/or dependent variables of the present technology. And one or more methods or prediction algorithms may be determined not to give significance to the data having different independent and/or dependent variables of the present technology. One may design or change parameters of the different variables of methods described herein based on results of one or more prediction algorithms (e.g., number of sets analyzed, types of nucleotide species in each set). For example, applying the Chi-squared test to detection data may suggest that specific ranges of maternal age are correlated to a higher likelihood of having an offspring with a specific chromosome abnormality, hence the variable of maternal age may be weighed differently verses being weighed the same as other variables.

Several algorithms may be chosen to be tested. These algorithms can be trained with raw data. For each new raw data sample, the trained algorithms will assign a classification to that sample (i.e. trisomy or normal). Based on the classifications of the new raw data samples, the trained algorithms' performance may be assessed based on sensitivity and specificity. Finally, an algorithm with the highest sensitivity and/or specificity or combination thereof may be identified.

### DETAILED DESCRIPTION

### Introduction

The presence of fetal nucleic acid in maternal plasma was first reported in 1997 and offers the possibility for non-invasive prenatal diagnosis simply through the analysis of a maternal blood sample (Lo et al., Lancet 350:485-487, 1997). To date, numerous potential clinical applications have been developed. In particular, quantitative abnormalities of fetal nucleic acid, for example DNA, concentrations in maternal plasma have been found to be associated with a number of pregnancy-associated disorders, including preeclampsia, preterm labor, antepartum hemorrhage, invasive placentation, fetal Down syndrome, and other fetal chromosomal aneuploidies. Hence, fetal nucleic acid analysis in maternal plasma represents a powerful mechanism for the monitoring of fetomaternal well-being.

However, fetal DNA co-exists with background maternal DNA in maternal plasma. Hence, most reported applications have relied on the detection of Y-chromosome sequences as these are most conveniently distinguishable from maternal DNA. Such an approach limits the applicability of the existing assays to only 50% of all pregnancies, namely those with male fetuses. Thus, there is much need for the development of sex-independent compositions and methods for enriching and analyzing fetal nucleic acid from a maternal sample. Also, methods that rely on polymorphic markers to quantify fetal nucleic acid may be susceptible to varying heterozygosity rates across different ethnicities thereby limiting their applicability (e.g., by increasing the number of markers that are needed).

It was previously demonstrated that fetal and maternal DNA can be distinguished by their differences in methylation status (U.S. Patent No. 6,927,028). Methylation is an epigenetic phenomenon, which refers to processes that alter a phenotype without involving changes in the DNA sequence. By exploiting the difference in the DNA methylation status between mother and fetus, one can successfully detect and analyze fetal nucleic acid in a background of maternal nucleic acid.

The present inventors provides novel genomic polynucleotides that are differentially methylated between the fetal DNA from the fetus (e.g., from the placenta) and the maternal DNA from the mother, for example from peripheral blood cells. This discovery thus provides a new approach for distinguishing fetal and maternal genomic DNA and new methods for accurately quantifying fetal nucleic which may be used for non-invasive prenatal diagnosis.

### Methodology

Practicing the technology utilizes routine techniques in the field of molecular biology. Basic texts disclosing the general methods of use in the technology include Sambrook and Russell, Molecular Cloning, A Laboratory Manual (3rd ed. 2001); Kriegler, Gene Transfer and Expression: A Laboratory Manual (1990); and Current Protocols in Molecular Biology (Ausubel et al., eds., 1994)).

For nucleic acids, sizes are given in either kilobases (kb) or base pairs (bp). These are estimates derived from agarose or acrylamide gel electrophoresis, from sequenced nucleic acids, or from published DNA sequences. For proteins, sizes are given in kilodaltons (kDa) or amino acid residue numbers. Protein sizes are estimated from gel electrophoresis, from sequenced proteins, from derived amino acid sequences, or from published protein sequences.

Oligonucleotides that are not commercially available can be chemically synthesized, e.g., according to the solid phase phosphoramidite triester method first described by Beaucage & Caruthers, Tetrahedron Lett. 22: 1859-1862 (1981), using an automated synthesizer, as described in Van Devanter et. al., Nucleic Acids Res. 12: 6159-6168 (1984). Purification of oligonucleotides is performed using any art-recognized strategy, e.g., native acrylamide gel electrophoresis or anion-exchange high performance liquid chromatography (HPLC) as described in Pearson & Reanier, J. Chrom. 255: 137-149 (1983).

### Acquisition of Blood Samples and Extraction of DNA

The present technology relates to separating, enriching and analyzing fetal DNA found in maternal blood as a non-invasive means to detect the presence and/or to monitor the progress of a pregnancy-associated condition or disorder. Thus, the first steps of practicing the technology are to obtain a blood sample from a pregnant woman and extract DNA from the sample.

### A. Acquisition of Blood Samples

A blood sample is obtained from a pregnant woman at a gestational age suitable for testing using a method of the present technology. The suitable gestational age may vary depending on the disorder tested, as discussed below. Collection of blood from a woman is performed in accordance with the standard protocol hospitals or clinics generally follow. An appropriate amount of peripheral blood, e.g., typically between 5-50 ml, is collected and may be stored according to standard procedure prior to further preparation. Blood samples may be collected, stored or transported in a manner known to the person of ordinary skill in the art to minimize degradation or the quality of nucleic acid present in the sample.

### B. Preparation of Blood Samples

The analysis of fetal DNA found in maternal blood according to the present technology may be performed using, e.g., the whole blood, serum, or plasma. The methods for preparing serum or plasma from maternal blood are well known among those of skill in the art. For example, a pregnant woman's blood can be placed in a tube containing EDTA or a specialized commercial product such as Vacutainer SST (Becton Dickinson, Franklin Lakes, N.J.) to prevent blood clotting, and plasma can then be obtained from whole blood through centrifugation. On the other hand, serum may be obtained with or without centrifugation-following blood clotting. If centrifugation is used then it is typically, though not exclusively, conducted at an appropriate speed, e.g., 1,500-3,000 times g. Plasma or serum may be subjected to additional centrifugation steps before being transferred to a fresh tube for DNA extraction.

In addition to the acellular portion of the whole blood, DNA may also be recovered from the cellular fraction, enriched in the buffy coat portion, which can be obtained following centrifugation of a whole blood sample from the woman and removal of the plasma.

### C. Extraction of DNA

There are numerous known methods for extracting DNA from a biological sample including blood. The general methods of DNA preparation (e.g., described by Sambrook and Russell, Molecular Cloning: A Laboratory Manual 3d ed., 2001) can be followed; various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep™ Blood DNA Isolation Kit (Promega, Madison, Wis.), and GFX™ Genomic Blood DNA Purification Kit (Amersham, Piscataway, N.J.), may also be used to obtain DNA from a blood sample from a pregnant woman. Combinations of more than one of these methods may also be used.

The sample may first be enriched or relatively enriched for fetal nucleic acid by one or more methods. For example, the discrimination of fetal and maternal DNA can be performed using the compositions and processes of the present technology alone or in combination with other discriminating factors. Examples of these factors include, but are not limited to, single nucleotide differences between chromosome X and Y, chromosome Y-specific sequences, polymorphisms located elsewhere in the genome, size differences between fetal and maternal DNA and differences in methylation pattern between maternal and fetal tissues.

Other methods for enriching a sample for a particular species of nucleic acid are described in PCT Patent Application Number PCT/US07/69991, filed May 30, 2007, PCT Patent Application Number PCT/US2007/071232, filed June 15, 2007, US Provisional Application Numbers 60/968,876 and 60/968,878 (assigned to the Applicant), (PCT Patent Application Number PCT/EP05/012707, filed November 28, 2005). Maternal nucleic acid may be selectively removed (either partially, substantially, almost completely or completely) from the sample.

### Methylation Specific Separation of Nucleic Acid

The methods provided herein offer an alternative approach for the enrichment of fetal DNA based on the methylation-specific separation of differentially methylated DNA. It has recently been discovered that many genes involved in developmental regulation are controlled through epigenetics in embryonic stem cells. Consequently, multiple genes can be expected to show differential DNA methylation between nucleic acid of fetal origin and maternal origin. Once these regions are identified, a technique to capture methylated DNA can be used to specifically enrich fetal DNA. For identification of differentially methylated regions, a novel approach was used to capture methylated DNA. This approach uses a protein, in which the methyl binding domain of MBD2 is fused to the Fc fragment of an antibody (MBD-FC) (Gebhard C, Schwarzfischer L, Pham TH, Schilling E, Klug M, Andreesen R, Rehli M (2006) Genomewide profiling of CpG methylation identifies novel targets of aberrant hypermethylation in myeloid leukemia. Cancer Res 66:6118-6128). This fusion protein has several advantages over conventional methylation specific antibodies. The MBD-FC has a higher affinity to methylated DNA and it binds double stranded DNA. Most importantly the two proteins differ in the way they bind DNA. Methylation specific antibodies bind DNA stochastically, which means that only a binary answer can be obtained. The methyl binding domain of MBD-FC on the other hand binds DNA molecules regardless of their methylation status. The strength of this protein - DNA interaction is defined by the level of DNA methylation. After binding genomic DNA, eluate solutions of increasing salt concentrations can be used to fractionate non-methylated and methylated DNA allowing for a more controlled separation (Gebhard C, Schwarzfischer L, Pham TH, Andreesen R, Mackensen A, Rehli M (2006) Rapid and sensitive detection of CpG-methylation using methyl-binding (MB)-PCR. Nucleic Acids Res 34:e82). Consequently this method, called Methyl-CpG immunoprecipitation (MCIP), cannot only enrich, but also fractionate genomic DNA according to methylation level, which is particularly helpful when the unmethylated DNA fraction should be investigated as well.

### Methylation Sensitive Restriction Enzyme Digestion

The technology also provides compositions and processes for determining the amount of fetal nucleic acid from a maternal sample. The technology allows for the enrichment of fetal nucleic acid regions in a maternal sample by selectively digesting nucleic acid from said maternal sample with an enzyme that selectively and completely or substantially digests the maternal nucleic acid to enrich the sample for at least one fetal nucleic acid region. Preferably, the digestion efficiency is greater than about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%. Following enrichment, the amount of fetal nucleic acid can be determined by quantitative methods that do not require polymorphic sequences or bisulfite treatment, thereby, offering a solution that works equally well for female fetuses and across different ethnicities and preserves the low copy number fetal nucleic acid present in the sample.

For example, there are methyl-sensitive enzymes that preferentially or substantially cleave or digest at their DNA recognition sequence if it is non-methylated. Thus, an unmethylated DNA sample will be cut into smaller fragments than a methylated DNA sample. Similarly, a hypermethylated DNA sample will not be cleaved. In contrast, there are methyl-sensitive enzymes that cleave at their DNA recognition sequence only if it is methylated.

Methyl-sensitive enzymes that digest unmethylated DNA suitable for use in methods of the technology include, but are not limited to, Hpall, Hhal, Maell, BstUI and Acil. An enzyme that can be used is Hpall that cuts only the unmethylated sequence CCGG. Another enzyme that can be used is Hhal that cuts only the unmethylated sequence GCGC. Both enzymes are available from New England BioLabs®, Inc. Combinations of two or more methyl-sensitive enzymes that digest only unmethylated DNA can also be used. Suitable enzymes that digest only methylated DNA include, but are not limited to, Dpnl, which cuts at a recognition sequence GATC, and McrBC, which belongs to the family of AAA.sup.+ proteins and cuts DNA containing modified cytosines and cuts at recognition site 5' ... Pu.sup.mC(N.sub.40-3000) Pu.sup.mC... 3' (New England BioLabs, Inc., Beverly, Mass.).

Cleavage methods and procedures for selected restriction enzymes for cutting DNA at specific sites are well known to the skilled artisan. For example, many suppliers of restriction enzymes provide information on conditions and types of DNA sequences cut by specific restriction enzymes, including New England BioLabs, Pro-Mega Biochems, Boehringer-Mannheim, and the like. Sambrook et al. (See Sambrook et al., Molecular Biology: A laboratory Approach, Cold Spring Harbor, N.Y. 1989) provide a general description of methods for using restriction enzymes and other enzymes. In methods of the present technology enzymes often are used under conditions that will enable cleavage of the maternal DNA with about 95%-100% efficiency, preferably with about 98%-100% efficiency.

### Other Methods for Methylation Analysis

Various methylation analysis procedures are known in the art, and can be used in conjunction with the present technology. These assays allow for determination of the methylation state of one or a plurality of CpG islands within a DNA sequence. In addition, the methods maybe used to quantify methylated nucleic acid. Such assays involve, among other techniques, DNA sequencing of bisulfite-treated DNA, PCR (for sequence-specific amplification), Southern blot analysis, and use of methylation-sensitive restriction enzymes.

Genomic sequencing is a technique that has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA may be used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

COBRA analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the interested CpG islands, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (e.g., as might be found in a typical COBRA-based kit) for COBRA analysis may include, but are not limited to: PCR primers for specific gene (or methylation-altered DNA sequence or CpG island); restriction enzyme and appropriate buffer; gene-hybridization oligo; control hybridization oligo; kinase labeling kit for oligo probe; and radioactive nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

The MethyLight™ assay is a high-throughput quantitative methylation assay that utilizes fluorescence-based real-time PCR (TaqMan.RTM.) technology that requires no further manipulations after the PCR step (Eads et al., Cancer Res. 59:2302-2306, 1999). Briefly, the MethyLight.TM. process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight process can by used with a "TaqMan" probe in the amplification process. For example, double-stranded genomic DNA is treated with sodium bisulfite and subjected to one of two sets of PCR reactions using TaqMan.RTM. probes; e.g., with either biased primers and TaqMan.RTM. probe, or unbiased primers and TaqMan.RTM. probe. The TaqMan.RTM. probe is dual-labeled with fluorescent "reporter" and "quencher" molecules, and is designed to be specific for a relatively high GC content region so that it melts out at about 10.degree. C. higher temperature in the PCR cycle than the forward or reverse primers. This allows the TaqMan.RTM. probe to remain fully hybridized during the PCR annealing/extension step. As the Taq polymerase enzymatically synthesizes a new strand during PCR, it will eventually reach the annealed TaqMan.RTM. probe. The Taq polymerase 5' to 3' endonuclease activity will then displace the TaqMan.RTM. probe by digesting it to release the fluorescent reporter molecule for quantitative detection of its now unquenched signal using a real-time fluorescent detection system.

Typical reagents (e.g., as might be found in a typical MethyLight.TM.-based kit) for MethyLight.TM. analysis may include, but are not limited to: PCR primers for specific gene (or methylation-altered DNA sequence or CpG island); TaqMan.RTM. probes; optimized PCR buffers and deoxynucleotides; and Taq polymerase.

The Ms-SNuPE technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997).

Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest.

Small amounts of DNA can be analyzed (e.g., microdissected pathology sections), and it avoids utilization of restriction enzymes for determining the methylation status at CpG sites.

Typical reagents (e.g., as might be found in a typical Ms-SNuPE-based kit) for Ms-SNuPE analysis may include, but are not limited to: PCR primers for specific gene (or methylation-altered DNA sequence or CpG island); optimized PCR buffers and deoxynucleotides; gel extraction kit; positive control primers; Ms-SNuPE primers for specific gene; reaction buffer (for the Ms-SNuPE reaction); and radioactive nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

MSP (methylation-specific PCR) allows for assessing the methylation status of virtually any group of CpG sites within a CpG island, independent of the use of methylation-sensitive restriction enzymes (Herman et al. Proc. Nat. Acad. Sci. USA 93:9821-9826, 1996; U.S. Pat. No. 5,786,146). Briefly, DNA is modified by sodium bisulfite converting unmethylated, but not methylated cytosines to uracil, and subsequently amplified with primers specific for methylated versus umnethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or methylation-altered DNA sequence or CpG island), optimized PCR buffers and deoxynucleotides, and specific probes.

The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (e.g., as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Another method for analyzing methylation sites is a primer extension assay, including an optimized PCR amplification reaction that produces amplified targets for subsequent primer extension genotyping analysis using mass spectrometry. The assay can also be done in multiplex. This method (particularly as it relates to genotyping single nucleotide polymorphisms) is described in detail in PCT publication WO05012578A1 and US publication US20050079521A1. For methylation analysis, the assay can be adopted to detect bisulfite introduced methylation dependent C to T sequence changes. These methods are particularly useful for performing multiplexed amplification reactions and multiplexed primer extension reactions (e.g., multiplexed homogeneous primer mass extension (hME) assays) in a single well to further increase the throughput and reduce the cost per reaction for primer extension reactions.

Four additional methods for DNA methylation analysis include restriction landmark genomic scanning (RLGS, Costello et al., 2000), methylation-sensitive-representational difference analysis (MS-RDA), methylation-specific AP-PCR (MS-AP-PCR) and methyl-CpG binding domain column/segregation of partly melted molecules (MBD/SPM).

Additional methylation analysis methods that may be used in conjunction with the present technology are described in the following papers: Laird, P.W. Nature Reviews Cancer 3, 253-266 (2003); Biotechniques; Uhlmann, K. et al. Electrophoresis 23:4072-4079 (2002) - PyroMeth; Colella et al. Biotechniques. 2003 Jul;35(1):146-50; Dupont JM, Tost J, Jammes H, and Gut IG. Anal Biochem, Oct 2004; 333(1): 119-27; and Tooke N and Pettersson M. IVDT. Nov 2004; 41.

### Polynucleotide Sequence Amplification and Determination

Following separation of nucleic acid in a methylation-differential manner, the nucleic acid may be subjected to sequence-based analysis. Furthermore, once it is determined that one particular genomic sequence of fetal origin is hypermethylated or hypomethylated compared to the maternal counterpart, the amount of this fetal genomic sequence can be determined. Subsequently, this amount can be compared to a standard control value and serve as an indication for the potential of certain pregnancy-associated disorder.

### A. Amplification of Nucleotide Sequences

In many instances, it is desirable to amplify a nucleic acid sequence of the technology using any of several nucleic acid amplification procedures which are well known in the art (listed above and described in greater detail below). Specifically, nucleic acid amplification is the enzymatic synthesis of nucleic acid amplicons (copies) which contain a sequence that is complementary to a nucleic acid sequence being amplified. Nucleic acid amplification is especially beneficial when the amount of target sequence present in a sample is very low. By amplifying the target sequences and detecting the amplicon synthesized, the sensitivity of an assay can be vastly improved, since fewer target sequences are needed at the beginning of the assay to better ensure detection of nucleic acid in the sample belonging to the organism or virus of interest.

A variety of polynucleotide amplification methods are well established and frequently used in research. For instance, the general methods of polymerase chain reaction (PCR) for polynucleotide sequence amplification are well known in the art and are thus not described in detail herein. For a review of PCR methods, protocols, and principles in designing primers, see, e.g., Innis, et al., PCR Protocols: A Guide to Methods and Applications, Academic Press, Inc. N.Y., 1990. PCR reagents and protocols are also available from commercial vendors, such as Roche Molecular Systems.

PCR is most usually carried out as an automated process with a thermostable enzyme. In this process, the temperature of the reaction mixture is cycled through a denaturing region, a primer annealing region, and an extension reaction region automatically. Machines specifically adapted for this purpose are commercially available.

Although PCR amplification of a polynucleotide sequence is typically used in practicing the present technology, one of skill in the art will recognize that the amplification of a genomic sequence found in a maternal blood sample may be accomplished by any known method, such as ligase chain reaction (LCR), transcription-mediated amplification, and self-sustained sequence replication or nucleic acid sequence-based amplification (NASBA), each of which provides sufficient amplification. More recently developed branched-DNA technology may also be used to qualitatively demonstrate the presence of a particular genomic sequence of the technology, which represents a particular methylation pattern, or to quantitatively determine the amount of this particular genomic sequence in the maternal blood. For a review of branched-DNA signal amplification for direct quantitation of nucleic acid sequences in clinical samples, see Nolte, Adv. Clin. Chem. 33:201-235, 1998.

The compositions and processes of the technology are also particularly useful when practiced with digital PCR. Digital PCR was first developed by Kalinina and colleagues (Kalinina et al., "Nanoliter scale PCR with TaqMan detection." Nucleic Acids Research. 25; 1999-2004, (1997)) and further developed by Vogelstein and Kinzler (Digital PCR. Proc Natl Acad Sci U S A. 96; 9236-41, (1999)). The application of digital PCR for use with fetal diagnostics was first described by Cantor et al. (PCT Patent Publication No. WO05023091A2) and subsequently described by Quake et al. (US Patent Publication No. US 20070202525). Digital PCR takes advantage of nucleic acid (DNA, cDNA or RNA) amplification on a single molecule level, and offers a highly sensitive method for quantifying low copy number nucleic acid. Fluidigm® Corporation offers systems for the digital analysis of nucleic acids.

### B. Determination of Polynucleotide Sequences.

Techniques for polynucleotide sequence determination are also well established and widely practiced in the relevant research field. For instance, the basic principles and general techniques for polynucleotide sequencing are described in various research reports and treatises on molecular biology and recombinant genetics, such as Wallace et al., supra; Sambrook and Russell, supra, and Ausubel et al., supra. DNA sequencing methods routinely practiced in research laboratories, either manual or automated, can be used for practicing the present technology. Additional means suitable for detecting changes in a polynucleotide sequence for practicing the methods of the present technology include but are not limited to mass spectrometry, primer extension, polynucleotide hybridization, real-time PCR, and electrophoresis.

Use of a primer extension reaction also can be applied in methods of the technology. A primer extension reaction operates, for example, by discriminating the SNP alleles by the incorporation of deoxynucleotides and/or dideoxynucleotides to a primer extension primer which hybridizes to a region adjacent to the SNP site. The primer is extended with a polymerase. The primer extended SNP can be detected physically by mass spectrometry or by a tagging moiety such as biotin. As the SNP site is only extended by a complementary deoxynucleotide or dideoxynucleotide that is either tagged by a specific label or generates a primer extension product with a specific mass, the SNP alleles can be discriminated and quantified.

Reverse transcribed and amplified nucleic acids may be modified nucleic acids. Modified nucleic acids can include nucleotide analogs, and may include a detectable label and/or a capture agent. Examples of detectable labels include without limitation fluorophores, radioisotopes, colormetric agents, light emitting agents, chemiluminescent agents, light scattering agents, enzymes and the like. Examples of capture agents include without limitation an agent from a binding pair selected from antibody/antigen, antibody/antibody, antibody/antibody fragment, antibody/antibody receptor, antibody/protein A or protein G, hapten/anti-hapten, biotin/avidin, biotin/streptavidin, folic acid/folate binding protein, vitamin B12/intrinsic factor, chemical reactive group/complementary chemical reactive group (e.g., sulfhydryl/maleimide, sulfhydryl/haloacetyl derivative, amine/isotriocyanate, amine/succinimidyl ester, and amine/sulfonyl halides) pairs, and the like. Modified nucleic acids having a capture agent can be immobilized to a solid support in certain methods.

Mass spectrometry is a particularly effective method for the detection of a polynucleotide of the technology, for example a PCR amplicon, a primer extension product or a detector probe that is cleaved from a target nucleic acid. The presence of the polynucleotide sequence is verified by comparing the mass of the detected signal with the expected mass of the polynucleotide of interest. The relative signal strength, e.g., mass peak on a spectra, for a particular polynucleotide sequence indicates the relative population of a specific allele, thus enabling calculation of the allele ratio directly from the data. For a review of genotyping methods using Sequenom® standard iPLEX™ assay and MassARRAY® technology, see Jurinke, C., Oeth, P., van den Boom, D., "MALDI-TOF mass spectrometry: a versatile tool for high-performance DNA analysis." Mol. Biotechnol. 26, 147-164 (2004); and Oeth, P. et al., "iPLEX™ Assay: Increased Plexing Efficiency and Flexibility for MassARRAY® System through single base primer extension with mass-modified Terminators." SEQUENOM Application Note (2005). For a review of detecting and quantifying target nucleic using cleavable detector probes that are cleaved during the amplification process and detected by mass spectrometry, see US Patent Application Number 11/950,395, which was filed December 4, 2007.

Sequencing technologies are improving in terms of throughput and cost. Sequencing technologies, such as that achievable on the 454 platform (Roche) (Margulies, M. et al. 2005 Nature 437, 376-380), Illumina Genome Analyzer (or Solexa platform) or SOLiD System (Applied Biosystems) or the Helicos True Single Molecule DNA sequencing technology (Harris T D et al. 2008 Science, 320, 106-109), the single molecule, real-time (SMRT.TM.) technology of Pacific Biosciences, and nanopore sequencing (Soni GV and Meller A. 2007 Clin Chem 53: 1996-2001), allow the sequencing of many nucleic acid molecules isolated from a specimen at high orders of multiplexing in a parallel fashion (Dear Brief Funct Genomic Proteomic 2003; 1: 397-416).

Each of these platforms allows sequencing of clonally expanded or non-amplified single molecules of nucleic acid fragments. Certain platforms involve, for example, (i) sequencing by ligation of dye-modified probes (including cyclic ligation and cleavage), (ii) pyrosequencing, and (iii) single-molecule sequencing. Nucleotide sequence species, amplification nucleic acid species and detectable products generated there from can be considered a "study nucleic acid" for purposes of analyzing a nucleotide sequence by such sequence analysis platforms.

Sequencing by ligation is a nucleic acid sequencing method that relies on the sensitivity of DNA ligase to base-pairing mismatch. DNA ligase joins together ends of DNA that are correctly base paired. Combining the ability of DNA ligase to join together only correctly base paired DNA ends, with mixed pools of fluorescently labeled oligonucleotides or primers, enables sequence determination by fluorescence detection. Longer sequence reads may be obtained by including primers containing cleavable linkages that can be cleaved after label identification. Cleavage at the linker removes the label and regenerates the 5' phosphate on the end of the ligated primer, preparing the primer for another round of ligation. Primers may be labeled with more than one fluorescent label (e.g., 1 fluorescent label, 2, 3, or 4 fluorescent labels).

An example of a system that can be used by a person of ordinary skill based on sequencing by ligation generally involves the following steps. Clonal bead populations can be prepared in emulsion microreactors containing study nucleic acid ("template"), amplification reaction components, beads and primers. After amplification, templates are denatured and bead enrichment is performed to separate beads with extended templates from undesired beads (e.g., beads with no extended templates). The template on the selected beads undergoes a 3' modification to allow covalent bonding to the slide, and modified beads can be deposited onto a glass slide. Deposition chambers offer the ability to segment a slide into one, four or eight chambers during the bead loading process. For sequence analysis, primers hybridize to the adapter sequence. A set of four color dye-labeled probes competes for ligation to the sequencing primer. Specificity of probe ligation is achieved by interrogating every 4th and 5th base during the ligation series. Five to seven rounds of ligation, detection and cleavage record the color at every 5th position with the number of rounds determined by the type of library used. Following each round of ligation, a new complimentary primer offset by one base in the 5' direction is laid down for another series of ligations. Primer reset and ligation rounds (5-7 ligation cycles per round) are repeated sequentially five times to generate 25-35 base pairs of sequence for a single tag. With mate-paired sequencing, this process is repeated for a second tag. Such a system can be used to exponentially amplify amplification products generated by a process described herein, e.g., by ligating a heterologous nucleic acid to the first amplification product generated by a process described herein and performing emulsion amplification using the same or a different solid support originally used to generate the first amplification product. Such a system also may be used to analyze amplification products directly generated by a process described herein by bypassing an exponential amplification process and directly sorting the solid supports described herein on the glass slide.

Pyrosequencing is a nucleic acid sequencing method based on sequencing by synthesis, which relies on detection of a pyrophosphate released on nucleotide incorporation. Generally, sequencing by synthesis involves synthesizing, one nucleotide at a time, a DNA strand complimentary to the strand whose sequence is being sought. Study nucleic acids may be immobilized to a solid support, hybridized with a sequencing primer, incubated with DNA polymerase, ATP sulfurylase, luciferase, apyrase, adenosine 5' phosphsulfate and luciferin. Nucleotide solutions are sequentially added and removed. Correct incorporation of a nucleotide releases a pyrophosphate, which interacts with ATP sulfurylase and produces ATP in the presence of adenosine 5' phosphsulfate, fueling the luciferin reaction, which produces a chemiluminescent signal allowing sequence determination.

An example of a system that can be used by a person of ordinary skill based on pyrosequencing generally involves the following steps: ligating an adaptor nucleic acid to a study nucleic acid and hybridizing the study nucleic acid to a bead; amplifying a nucleotide sequence in the study nucleic acid in an emulsion; sorting beads using a picoliter multiwell solid support; and sequencing amplified nucleotide sequences by pyrosequencing methodology (e.g., Nakano et al., "Single-molecule PCR using water-in-oil emulsion;" Journal of Biotechnology 102: 117-124 (2003)). Such a system can be used to exponentially amplify amplification products generated by a process described herein, e.g., by ligating a heterologous nucleic acid to the first amplification product generated by a process described herein.

Certain single-molecule sequencing methods are based on the principal of sequencing by synthesis, and utilize single-pair Fluorescence Resonance Energy Transfer (single pair FRET) as a mechanism by which photons are emitted as a result of successful nucleotide incorporation. The emitted photons often are detected using intensified or high sensitivity cooled charge-couple-devices in conjunction with total internal reflection microscopy (TIRM). Photons are only emitted when the introduced reaction solution contains the correct nucleotide for incorporation into the growing nucleic acid chain that is synthesized as a result of the sequencing process. In FRET based single-molecule sequencing, energy is transferred between two fluorescent dyes, sometimes polymethine cyanine dyes Cy3 and Cy5, through long-range dipole interactions. The donor is excited at its specific excitation wavelength and the excited state energy is transferred, non-radiatively to the acceptor dye, which in turn becomes excited. The acceptor dye eventually returns to the ground state by radiative emission of a photon. The two dyes used in the energy transfer process represent the "single pair", in single pair FRET. Cy3 often is used as the donor fluorophore and often is incorporated as the first labeled nucleotide. Cy5 often is used as the acceptor fluorophore and is used as the nucleotide label for successive nucleotide additions after incorporation of a first Cy3 labeled nucleotide. The fluorophores generally are within 10 nanometers of each for energy transfer to occur successfully.

An example of a system that can be used based on single-molecule sequencing generally involves hybridizing a primer to a study nucleic acid to generate a complex; associating the complex with a solid phase; iteratively extending the primer by a nucleotide tagged with a fluorescent molecule; and capturing an image of fluorescence resonance energy transfer signals after each iteration (e.g., U.S. Patent No. 7,169,314; Braslavsky et al., PNAS 100(7): 3960-3964 (2003)). Such a system can be used to directly sequence amplification products generated by processes described herein. The released linear amplification product may be hybridized to a primer that contains sequences complementary to immobilized capture sequences present on a solid support, a bead or glass slide for example. Hybridization of the primer--released linear amplification product complexes with the immobilized capture sequences, immobilizes released linear amplification products to solid supports for single pair FRET based sequencing by synthesis. The primer often is fluorescent, so that an initial reference image of the surface of the slide with immobilized nucleic acids can be generated. The initial reference image is useful for determining locations at which true nucleotide incorporation is occurring. Fluorescence signals detected in array locations not initially identified in the "primer only" reference image are discarded as non-specific fluorescence. Following immobilization of the primer--released linear amplification product complexes, the bound nucleic acids often are sequenced in parallel by the iterative steps of, a) polymerase extension in the presence of one fluorescently labeled nucleotide, b) detection of fluorescence using appropriate microscopy, TIRM for example, c) removal of fluorescent nucleotide, and d) return to step a with a different fluorescently labeled nucleotide.

Nucleotide sequencing may be by solid phase single nucleotide sequencing methods and processes. Solid phase single nucleotide sequencing methods involve contacting sample nucleic acid and solid support under conditions in which a single molecule of sample nucleic acid hybridizes to a single molecule of a solid support. Such conditions can include providing the solid support molecules and a single molecule of sample nucleic acid in a "microreactor." Such conditions also can include providing a mixture in which the sample nucleic acid molecule can hybridize to solid phase nucleic acid on the solid support. Single nucleotide sequencing methods useful in the methods described herein are described in United States Provisional Patent Application Serial Number 61/021,871 filed January 17, 2008.

Nanopore sequencing detection methods may include (a) contacting a nucleic acid for sequencing ("base nucleic acid," e.g., linked probe molecule) with sequence-specific detectors, under conditions in which the detectors specifically hybridize to substantially complementary subsequences of the base nucleic acid; (b) detecting signals from the detectors and (c) determining the sequence of the base nucleic acid according to the signals detected. The detectors hybridized to the base nucleic acid may be disassociated from the base nucleic acid (e.g., sequentially dissociated) when the detectors interfere with a nanopore structure as the base nucleic acid passes through a pore, and the detectors disassociated from the base sequence are detected. A detector disassociated from a base nucleic acid may emit a detectable signal, and the detector hybridized to the base nucleic acid may emit a different detectable signal or no detectable signal. Nucleotides in a nucleic acid (e.g., linked probe molecule) may besubstituted with specific nucleotide sequences corresponding to specific nucleotides ("nucleotide representatives"), thereby giving rise to an expanded nucleic acid (e.g., U.S. Patent No. 6,723,513), and the detectors hybridize to the nucleotide representatives in the expanded nucleic acid, which serves as a base nucleic acid. Here, nucleotide representatives may be arranged in a binary or higher order arrangement (e.g., Soni and Meller, Clinical Chemistry 53(11): 1996-2001 (2007)). A nucleic acid not being expanded, may not give rise to an expanded nucleic acid, and may directly serve a base nucleic acid (e.g., a linked probe molecule serves as a non-expanded base nucleic acid), and detectors may be directly contacted with the base nucleic acid. For example, a first detector may hybridize to a first subsequence and a second detector may hybridize to a second subsequence, where the first detector and second detector each have detectable labels that can be distinguished from one another, and where the signals from the first detector and second detector can be distinguished from one another when the detectors are disassociated from the base nucleic acid. Detectors may include a region that hybridizes to the base nucleic acid (e.g., two regions), which can be about 3 to about 100 nucleotides in length (e.g., about 4, 5, 6, 7, 8, 9, 10, 11, 12, 13, 14, 15, 16, 17, 18, 19, 20, 25, 30, 35, 40, 50, 55, 60, 65, 70, 75, 80, 85, 90, or 95 nucleotides in length). A detector also may include one or more regions of nucleotides that do not hybridize to the base nucleic acid. A detector may be a molecular beacon. A detector often comprises one or more detectable labels independently selected from those described herein. Each detectable label can be detected by any convenient detection process capable of detecting a signal generated by each label (e.g., magnetic, electric, chemical, optical and the like). For example, a CD camera can be used to detect signals from one or more distinguishable quantum dots linked to a detector.

In certain sequence analysis methods, reads may be used to construct a larger nucleotide sequence, which can be facilitated by identifying overlapping sequences in different reads and by using identification sequences in the reads. Such sequence analysis methods and software for constructing larger sequences from reads are known to the person of ordinary skill (e.g., Venter et al., Science 291: 1304-1351 (2001)). Specific reads, partial nucleotide sequence constructs, and full nucleotide sequence constructs may be compared between nucleotide sequences within a sample nucleic acid (i.e., internal comparison) or may be compared with a reference sequence (i.e., reference comparison) in certain sequence analysis methods. Internal comparisons sometimes are performed in situations where a sample nucleic acid is prepared from multiple samples or from a single sample source that contains sequence variations. Reference comparisons sometimes are performed when a reference nucleotide sequence is known and an objective is to determine whether a sample nucleic acid contains a nucleotide sequence that is substantially similar or the same, or different, than a reference nucleotide sequence. Sequence analysis is facilitated by sequence analysis apparatus and components known to the person of ordinary skill in the art.

Methods provided herein allow for high-throughput detection of nucleic acid species in a plurality of nucleic acids (e.g., nucleotide sequence species, amplified nucleic acid species and detectable products generated from the foregoing). Multiplexing refers to the simultaneous detection of more than one nucleic acid species. General methods for performing multiplexed reactions in conjunction with mass spectrometry, are known (see, e.g., U.S. Pat. Nos. 6,043,031, 5,547,835 and International PCT application No. WO 97/37041). Multiplexing provides an advantage that a plurality of nucleic acid species (e.g., some having different sequence variations) can be identified in as few as a single mass spectrum, as compared to having to perform a separate mass spectrometry analysis for each individual target nucleic acid species. Methods provided herein may lend themselves to high-throughput, highly-automated processes for analyzing sequence variations with high speed and accuracy. The methods described herein may be multiplexed at high levels in a single reaction.

The number of nucleic acid species multiplexed may include, without limitation, about 1 to about 500 (e.g., about 1-3, 3-5, 5-7, 7-9, 9-11, 11-13, 13-15, 15-17, 17-19, 19-21, 21-23, 23-25, 25-27, 27-29, 29-31, 31-33, 33-35, 35-37, 37-39, 39-41, 41-43, 43-45, 45-47, 47-49, 49-51, 51-53, 53-55, 55-57, 57-59, 59-61, 61-63, 63-65, 65-67, 67-69, 69-71, 71-73, 73-75, 75-77, 77-79, 79-81, 81-83, 83-85, 85-87, 87-89, 89-91, 91-93, 93-95, 95-97, 97-101, 101-103, 103-105, 105-107, 107-109, 109-111, 111-113, 113-115, 115-117, 117-119, 121-123, 123-125, 125-127, 127-129, 129-131, 131-133, 133-135, 135-137, 137-139, 139-141, 141-143, 143-145, 145-147, 147-149, 149-151, 151-153, 153-155, 155-157, 157-159, 159-161, 161-163, 163-165, 165-167, 167-169, 169-171, 171-173, 173-175, 175-177, 177-179, 179-181, 181-183, 183-185, 185-187, 187-189, 189-191, 191-193, 193-195, 195-197, 197-199, 199-201, 201-203, 203-205, 205-207, 207-209, 209-211, 211-213, 213-215, 215-217, 217-219, 219-221, 221-223, 223-225, 225-227, 227-229, 229-231, 231-233, 233-235, 235-237, 237-239, 239-241, 241-243, 243-245, 245-247, 247-249, 249-251, 251-253, 253-255, 255-257, 257-259, 259-261, 261-263, 263-265, 265-267, 267-269, 269-271, 271-273, 273-275, 275-277, 277-279, 279-281, 281-283, 283-285, 285-287, 287-289, 289-291, 291-293, 293-295, 295-297, 297-299, 299-301, 301- 303, 303- 305, 305- 307, 307- 309, 309- 311, 311- 313, 313- 315, 315- 317, 317- 319, 319-321, 321-323, 323-325, 325-327, 327-329, 329-331, 331-333, 333- 335, 335-337, 337-339, 339-341, 341-343, 343-345, 345-347, 347-349, 349-351, 351-353, 353-355, 355-357, 357-359, 359-361, 361-363, 363-365, 365-367, 367-369, 369-371, 371-373, 373-375, 375-377, 377-379, 379-381, 381-383, 383-385, 385-387, 387-389, 389-391, 391-393, 393-395, 395-397, 397-401, 401- 403, 403- 405, 405- 407, 407- 409, 409- 411, 411- 413, 413- 415, 415- 417, 417- 419, 419-421, 421-423, 423-425, 425-427, 427-429, 429-431, 431-433, 433- 435, 435-437, 437-439, 439-441, 441-443, 443-445, 445-447, 447-449, 449-451, 451-453, 453-455, 455-457, 457-459, 459-461, 461-463, 463-465, 465-467, 467-469, 469-471, 471-473, 473-475, 475-477, 477-479, 479-481, 481-483, 483-485, 485-487, 487-489, 489-491, 491-493, 493-495, 495-497, 497-501).

Design methods for achieving resolved mass spectra with multiplexed assays can include primer and oligonucleotide design methods and reaction design methods. See, for example, the multiplex schemes provided in Tables X and Y. For primer and oligonucleotide design in multiplexed assays, the same general guidelines for primer design applies for uniplexed reactions, such as avoiding false priming and primer dimers, only more primers are involved for multiplex reactions. For mass spectrometry applications, analyte peaks in the mass spectra for one assay are sufficiently resolved from a product of any assay with which that assay is multiplexed, including pausing peaks and any other by-product peaks. Also, analyte peaks optimally fall within a user-specified mass window, for example, within a range of 5,000-8,500 Da. Multiplex analysis may be adapted to mass spectrometric detection of chromosome abnormalities, for example. Multiplex analysis may be adapted to various single nucleotide or nanopore based sequencing methods described herein. Commercially produced micro-reaction chambers or devices or arrays or chips may be used to facilitate multiplex analysis, and are commercially available.

### Detection of Fetal Aneuploidy

For the detection of fetal aneuploidies, some methods rely on measuring the ratio between maternally and paternally inherited alleles. However, the ability to quantify chromosomal changes is impaired by the maternal contribution of cell free nucleic acids, which makes it necessary to deplete the sample from maternal DNA prior to measurement. Promising approaches take advantage of the different size distribution of fetal and maternal DNA or measure RNA that is exclusively expressed by the fetus (see for example, US Patent Application No. 11/384128, which published as US20060252071). Assuming fetal DNA makes up only about 5% of all cell free DNA in the maternal plasma, there is a decrease of the ratio difference from 1.6% to only about 1.2% between a trisomy sample and a healthy control. Consequently, reliable detection of allele ratio changes requires enriching the fetal fraction of cell free DNA, for example, using the compositions and methods of the present technology.

Some methods rely on measuring the ratio of maternal to paternally inherited alleles to detect fetal chromosomal aneuploidies from maternal plasma. A diploid set yields a 1:1 ratio while trisomies can be detected as a 2:1 ratio. Detection of this difference is impaired by statistical sampling due to the low abundance of fetal DNA, presence of excess maternal DNA in the plasma sample and variability of the measurement technique. The latter is addressed by using methods with high measurement precision, like digital PCR or mass spectrometry. Enriching the fetal fraction of cell free DNA in a sample is currently achieved by either depleting maternal DNA through size exclusion or focusing on fetal-specific nucleic acids, like fetal-expressed RNA. Another differentiating feature of fetal DNA is its DNA methylation pattern. Thus, provided herein are novel compositions and methods for accurately quantifying fetal nucleic acid based on differential methylation between a fetus and mother. The methods rely on sensitive absolute copy number analysis to quantify the fetal nucleic acid portion of a maternal sample, thereby allowing for the prenatal detection of fetal traits. The methods of the technology have identified approximately 3000 CpG rich regions in the genome that are differentially methylated between maternal and fetal DNA. The selected regions showed highly conserved differential methylation across all measured samples. In addition the set of regions is enriched for genes important in developmental regulation, indicating that epigenetic regulation of these areas is a biologically relevant and consistent process (see Table 3). Enrichment of fetal DNA can now be achieved by using our MBD-FC protein to capture cell free DNA (e.g., substantially all cell free DNA) and then elute the highly methylated DNA fraction with high salt concentrations. Using the low salt eluate fractions, the MBD-FC is equally capable of enriching non-methylated fetal DNA.

The present technology provides 63 confirmed genomic regions on chromosomes 13, 18 and 21 with low maternal and high fetal methylation levels. After capturing these regions, SNPs can be used to determine the aforementioned allele ratios. When high frequency SNPs are used around 10 markers have to be measured to achieve a high confidence of finding at least one SNP where the parents have opposite homozygote genotypes and the child has a heterozygote genotype.

Also described herein is a method for chromosomal abnormality detection that utilizes absolute copy number quantification. A diploid chromosome set will show the same number of copies for differentially methylated regions across all chromosomes, but, for example, a trisomy 21 sample would show 1.5 times more copies for differentially methylated regions on chromosome 21. Normalization of the genomic DNA amounts for a diploid chromosome set can be achieved by using unaltered autosomes as reference (also provided herein - see Table 1). Comparable to other approaches, a single marker is less likely to be sufficient for detection of this difference, because the overall copy numbers are low. Typically there are approximately 100 to 200 copies of fetal DNA from 1 ml of maternal plasma at 10 to 12 weeks of gestation. However, the methods of the present technology offer a redundancy of detectable markers that enables highly reliable discrimination of diploid versus aneuploid chromosome sets.

### Data Processing and Identifying Presence or Absence of a Chromosome Abnormality

The term "detection" of a chromosome abnormality as used herein refers to identification of an imbalance of chromosomes by processing data arising from detecting sets of amplified nucleic acid species, nucleotide sequence species, or a detectable product generated from the foregoing (collectively "detectable product"). Any suitable detection device and method can be used to distinguish one or more sets of detectable products, as addressed herein. An outcome pertaining to the presence or absence of a chromosome abnormality can be expressed in any suitable form, including, without limitation, probability (e.g., odds ratio, p-value), likelihood, percentage, value over a threshold, or risk factor, associated with the presence of a chromosome abnormality for a subject or sample. An outcome may be provided with one or more of sensitivity, specificity, standard deviation, coefficient of variation (CV) and/or confidence level, or combinations of the foregoing.

Detection of a chromosome abnormality based on one or more sets of detectable products may be identified based on one or more calculated variables, including, but not limited to, sensitivity, specificity, standard deviation, coefficient of variation (CV), a threshold, confidence level, score, probability and/or a combination thereof. (i) The number of sets selected for a diagnostic method, and/or (ii) the particular nucleotide sequence species of each set selected for a diagnostic method, may be determined in part or in full according to one or more of such calculated variables.

One or more of sensitivity, specificity and/or confidence level may be expressed as a percentage. The percentage, independently for each variable, may be greater than about 90% (e.g., about 90, 91, 92, 93, 94, 95, 96, 97, 98 or 99%, or greater than 99% (e.g., about 99.5%, or greater, about 99.9% or greater, about 99.95% or greater, about 99.99% or greater)). Coefficient of variation (CV) may be expressed as a percentage, and sometimes the percentage is about 10% or less (e.g., about 10, 9, 8, 7, 6, 5, 4, 3, 2 or 1%, or less than 1% (e.g., about 0.5% or less, about 0.1% or less, about 0.05% or less, about 0.01% or less)). A probability (e.g., that a particular outcome determined by an algorithm is not due to chance) may be expressed as a p-value, and sometimes the p-value is about 0.05 or less (e.g., about 0.05, 0.04, 0.03, 0.02 or 0.01, or less than 0.01 (e.g., about 0.001 or less, about 0.0001 or less, about 0.00001 or less, about 0.000001 or less)).

For example, scoring or a score may refer to calculating the probability that a particular chromosome abnormality is actually present or absent in a subject/sample. The value of a score may be used to determine for example the variation, difference, or ratio of amplified nucleic detectable product that may correspond to the actual chromosome abnormality. For example, calculating a positive score from detectable products can lead to an identification of a chromosome abnormality, which is particularly relevant to analysis of single samples.

In certain methods, simulated (or simulation) data may aid data processing for example by training an algorithm or testing an algorithm. Simulated data may for instance involve hypothetical various samples of different concentrations of fetal and maternal nucleic acid in serum, plasma and the like. Simulated data may be based on what might be expected from a real population or may be skewed to test an algorithm and/or to assign a correct classification based on a simulated data set. Simulated data also is referred to herein as "virtual" data. Fetal/maternal contributions within a sample can be simulated as a table or array of numbers (for example, as a list of peaks corresponding to the mass signals of cleavage products of a reference biomolecule or amplified nucleic acid sequence), as a mass spectrum, as a pattern of bands on a gel, or as a representation of any technique that measures mass distribution. Simulations can be performed in most instances by a computer program. One possible step in using a simulated data set is to evaluate the confidence of the identified results, i.e. how well the selected positives/negatives match the sample and whether there are additional variations. A common approach is to calculate the probability value (p-value) which estimates the probability of a random sample having better score than the selected one. As p-value calculations can be prohibitive in certain circumstances, an empirical model may be assessed, in which it is assumed that at least one sample matches a reference sample (with or without resolved variations). Alternatively other distributions such as Poisson distribution can be used to describe the probability distribution.

An algorithm may assign a confidence value to the true positives, true negatives, false positives and false negatives calculated. The assignment of a likelihood of the occurrence of a chromosome abnormality can also be based on a certain probability model.

Simulated data often is generated in an *in silico* process. As used herein, the term "in silico" refers to research and experiments performed using a computer. In silico methods include, but are not limited to, molecular modeling studies, karyotyping, genetic calculations, biomolecular docking experiments, and virtual representations of molecular structures and/or processes, such as molecular interactions.

As used herein, a "data processing routine" refers to a process, that can be embodied in software, that determines the biological significance of acquired data (i.e., the ultimate results of an assay). For example, a data processing routine can determine the amount of each nucleotide sequence species based upon the data collected. A data processing routine also may control an instrument and/or a data collection routine based upon results determined. A data processing routine and a data collection routine often are integrated and provide feedback to operate data acquisition by the instrument, and hence provide assay-based judging methods provided herein.

As used herein, software refers to computer readable program instructions that, when executed by a computer, perform computer operations. Typically, software is provided on a program product containing program instructions recorded on a computer readable medium, including, but not limited to, magnetic media including floppy disks, hard disks, and magnetic tape; and optical media including CD-ROM discs, DVD discs, magneto-optical discs, and other such media on which the program instructions can be recorded.

Different methods of predicting abnormality or normality can produce different types of results. For any given prediction, there are four possible types of outcomes: true positive, true negative, false positive, or false negative. The term "true positive" as used herein refers to a subject correctly diagnosed as having a chromosome abnormality. The term "false positive" as used herein refers to a subject wrongly identified as having a chromosome abnormality. The term "true negative" as used herein refers to a subject correctly identified as not having a chromosome abnormality. The term "false negative" as used herein refers to a subject wrongly identified as not having a chromosome abnormality. Two measures of performance for any given method can be calculated based on the ratios of these occurrences: (i) a sensitivity value, the fraction of predicted positives that are correctly identified as being positives (e.g., the fraction of nucleotide sequence sets correctly identified by level comparison detection/determination as indicative of chromosome abnormality, relative to all nucleotide sequence sets identified as such, correctly or incorrectly), thereby reflecting the accuracy of the results in detecting the chromosome abnormality; and (ii) a specificity value, the fraction of predicted negatives correctly identified as being negative (the fraction of nucleotide sequence sets correctly identified by level comparison detection/determination as indicative of chromosomal normality, relative to all nucleotide sequence sets identified as such, correctly or incorrectly), thereby reflecting accuracy of the results in detecting the chromosome abnormality.

### EXAMPLES

The following examples are provided by way of illustration only and not by way of limitation. Those of skill in the art will readily recognize a variety of non-critical parameters that could be changed or modified to yield essentially the same or similar results.

In Example 1 below, the Applicants used a new fusion protein that captures methylated DNA in combination with CpG Island array to identify genomic regions that are differentially methylated between fetal placenta tissue and maternal blood. A stringent statistical approach was used to only select regions which show little variation between the samples, and hence suggest an underlying biological mechanism. Eighty-five differentially methylated genomic regions predominantly located on chromosomes 13, 18 and 21 were validated. For this validation, a quantitative mass spectrometry based approach was used that interrogated 261 PCR amplicons covering these 85 regions. The results are in very good concordance (95% confirmation), proving the feasibility of the approach.

Next, the Applicants provide an innovative approach for aneuploidy testing, which relies on the measurement of absolute copy numbers rather than allele ratios.

### Example 1

In the below Example, ten paired maternal and placental DNA samples were used to identify differentially methylated regions. These results were validated using a mass spectrometry-based quantitative methylation assay. First, genomic DNA from maternal buffy coat and corresponding placental tissue was first extracted. Next the MBD-FC was used to capture the methylated fraction of each DNA sample. See Figures 1-3. The two tissue fractions were labeled with different fluorescent dyes and hybridized to an Agilent® CpG Island microarray. See Figure 4. This was done to identify differentially methylated regions that could be utilized for prenatal diagnoses. Therefore, two criteria were employed to select genomic regions as potential enrichment markers: the observed methylation difference had to be present in all tested sample pairs, and the region had to be more than 200 bp in length.

### DNA preparation and fragmentation

Genomic DNA (gDNA) from maternal buffy coat and placental tissue was prepared using the QIAamp DNA Mini Kit™ and QIAamp DNA Blood Mini Kit™, respectively, from Qiagen® (Hilden, Germany). For MCIp, gDNA was quantified using the NanoDrop ND 1000™ spectrophotometer (Thermo Fisher®, Waltham, MA,USA). Ultrasonication of 2.5 µg DNA in 500 µl TE buffer to a mean fragment size of 300-500 bp was carried out with the Branson Digital Sonifier 450™ (Danbury, CT, USA) using the following settings: amplitude 20%, sonication time 110 seconds, pulse on/pulse off time 1.4/0.6 seconds. Fragment range was monitored using gel electrophoresis.

### Methyl-CpG Immunoprecipitation

Per sample, 56 µg purified MBD-Fc protein and 150 µl of Protein A Sepharose 4 Fast Flow beads (Amersham Biosciences®, Piscataway, NJ, USA) were rotated in 15 ml TBS overnight at 4°C. Then, theMBD-Fc beads (150 µl/assay) were transferred and dispersed in to 2 ml Ultrafree-CL centrifugal filter devices (Millipore®, Billerica, MA, USA) and spin-washed three times with Buffer A (20 mM Tris-HCI, pH8.0, 2 mM MgCI2, 0.5 mM EDTA 300 mM NaCl, 0.1% NP-40). Sonicated DNA (2 µg) was added to the washed MBD-Fc beads in 2 ml Buffer A and rotated for 3 hours at 4°C. Beads were centrifuged to recover unbound DNA fragments (300 mM fraction) and subsequently washed twice with 600 µl of buffers containing increasing NaCl concentrations (400, 500, 550, 600, and 1000 mM). The flow through of each wash step was collected in separate tubes and desalted using a MinElute PCR Purification Kit™ (Qiagen®). In parallel, 200 ng sonicated input DNA was processed as a control using the MinElute PCR Purification Kit™ (Qiagen®).

### Microarray handling and analysis

To generate fluorescently labeled DNA for microarray hybridization, the 600 mM and 1M NaCl fractions (enriched methylated DNA) for each sample were combined and labeled with either Alexa Fluor 555-aha-dCTP (maternal) or Alexa Fluor 647-aha-dCTP (placental) using the BioPrime Total Genomic Labeling System™ (Invitrogen®, Carlsbad, CA, USA). The labeling reaction was carried out according to the manufacturer's manual. The differently labeled genomic DNA fragments of matched maternal/placental pairs were combined to a final volume of 80 µl, supplemented with 50 µg Cot-1 DNA (Invitrogen®), 52 µl of Agilent 10X blocking reagent (Agilent Technologies®, Santa Clara, CA, USA), 78 µl of deionized formamide, and 260 µl Agilent 2X hybridization buffer. The samples were heated to 95°C for 3 min, mixed, and subsequently incubated at 37°C for 30 min. Hybridization on Agilent CpG Island Microarray Kit™ was then carried out at 67°C for 40 hours using an Agilent SureHyb™ chamber and an Agilent hybridization oven. Slides were washed in Wash I (6X SSPE, 0.005% N-lauroylsarcosine) at room temperature for 5 min and in Wash II (0.06X SSPE) at 37°C for an additional 5 min. Next, the slides were submerged in acetonitrile and Agilent Ozone Protection Solution™, respectively, for 30 seconds. Images were scanned immediately and analyzed using an Agilent DNA Microarray Scanner™. Microarray images were processed using Feature Extraction Software v9.5 and the standard CGH protocol.

### Bisulfite Treatment

Genomic DNA sodium bisulfite conversion was performed using EZ-96 DNA Methylation Kit™ (ZymoResearch, Orange County, CA). The manufacturer's protocol was followed using 1ug of genomic DNA and the alternative conversion protocol (a two temperature DNA denaturation).

### Quantitative Methylation Analysis

Sequenom's MassARRAY® System was used to perform quantitative methylation analysis. This system utilizes matrix-assisted laser desorption ionization time-of-flight (MALDI-TOF) mass spectrometry in combination with RNA base specific cleavage (Sequenom® MassCLEAVE™). A detectable pattern is then analyzed for methylation status. PCR primers were designed using Sequenom® *EpiDESIGNER™* (www.epidesigner.com). A total of 261 amplicons, covering 85 target regions, were used for validation (median amplification length = 367 bp, min = 108, max = 500; median number of CpG's per amplicon =23, min = 4, max = 65). For each reverse primer, an additional T7 promoter tag for *in-vivo* transcription was added, as well as a 10mer tag on the forward primer to adjust for melting temperature differences. The MassCLEAVE(tm) biochemistry was performed as previously described (Ehrich M, et al. (2005) Quantitative high-throughput analysis of DNA methylation patterns by base specific cleavage and mass spectrometry. Proc Natl Acad Sci U S A 102:15785-15790). Mass spectra were acquired using a MassARRAY™ Compact MALDI-TOF (Sequenom® San Diego) and methylation ratios were generated by the EpiTYPER™ software v1.0 (Sequenom®, San Diego).

### Statistical analysis

All statistical calculations were performed using the:R statistical software package (www.r-project.org). First, the array probes were grouped based on their genomic location. Subsequent probes that were less than 1000 bp apart were grouped together. To identify differentially methylated regions, a control sample was used as reference. In the control sample, the methylated fraction of a blood derived control DNA was hybridized against itself. Ideally this sample should show log ratios of the two color channels around 0. However because of the variability in hybridization behavior, the probes show a mean log ratio of 0.02 and a standard deviation of 0.18. Next the log ratios observed in our samples were compared to the control sample. A two way, paired t-test was used to test the NULL hypothesis that the groups are identical. Groups that contained less than 4 probes were excluded from the analysis. For groups including four or five probes, all probes were used in a paired t-test. For Groups with six or more probes, a sliding window test consisting of five probes at a time was used, whereby the window was moved by one probe increments. Each test sample was compared to the control sample and the p-values were recorded. Genomic regions were selected as being differentially methylated if eight out of ten samples showed a p value < 0.01, or if six out of ten samples showed a p value < 0.001. The genomic regions were classified as being not differentially methylated when the group showed less than eight samples with a p value < 0.01 and less than six samples with a p value < 0.001. Samples that didn't fall in either category were excluded from the analysis. For a subset of genomic regions that have been identified as differentially methylated, the results were confirmed using quantitative methylation analysis.
The Go analysis was performed using the online GOstat tool (http://gostat.wehi.edu.au/cgibin/-goStat.pl). P values were calculated using Fisher's exact test.

### Microarray-based marker discovery results

To identify differentially methylated regions a standard sample was used, in which the methylated DNA fraction of monocytes was hybridized against itself. This standard provided a reference for the variability of fluorescent measurements in a genomic region. Differentially methylated regions were then identified by comparing the log ratios of each of the ten placental/maternal samples against this standard. Because the goal of this study was to identify markers that allow the reliable separation of maternal and fetal DNA, the target selection was limited to genes that showed a stable, consistent methylation difference over a contiguous stretch of genomic DNA. This focused the analysis on genomic regions where multiple probes indicated differential methylation. The selection was also limited to target regions where all samples showed differential methylation, excluding those with strong inter-individual differences. Two of the samples showed generally lower log ratios in the microarray analysis. Because a paired test was used for target selection, this did not negatively impact the results.
Based on these selection criteria, 3043 genomic regions were identified that were differentially methylated between maternal and fetal DNA. 21778 regions did not show a methylation difference. No inter-chromosomal bias in the distribution of differentially methylated regions was observed. The differentially methylated regions were located next to or within 2159 known genes. The majority of differentially methylated regions are located in the promoter area (18%) and inside the coding region (68%), while only few regions are located downstream of the gene (7%) or at the transition from promoter to coding region (7%). Regions that showed no differential methylation showed a similar distribution for promoter (13%) and downstream (5%) locations, but the fraction of regions located in the transition of promoter to coding region was higher (39%) and the fraction inside the coding region was lower (43%).

It has been shown in embryonic stem cells (ES) that genes targeted by the polycomb repressive complex2 (PRC2) are enriched for genes regulating development (Lee TI, et al. (2006) Control of developmental regulators by Polycomb in human embryonic stem cells. Cell 125:301-313). It has also been shown that differentially methylated genes are enriched for genes targeted by PRC2 in many cancer types (Ehrich M, et al. (2008) Cytosine methylation profiling of cancer cell lines. Proc Natl Acad Sci U S A 105:4844-48). The set of genes identified as differentially methylated in this study is also enriched for genes targeted by PRC2 (p-value < 0.001, odds ratio = 3.6, 95% CI for odds ratio= 3.1 - 4.2). A GO analysis of the set of differentially methylated genes reveals that this set is significantly enriched for functions important during development. Six out of the ten most enriched functions include developmental or morphogenic processes [anatomical structure morphogenesis (GO:0009653, p value =0), developmental process (GO:0032502, p value = 0), multicellular organismal development (GO:0007275, p value = 0), developmental of an organ (GO:0048513, p value = 0), system development (GO:0048731, p value = 0) and development of an anatomical structure (GO:0048856, p value = 0)].

### Validation using Sequenom® EpiTYPER™

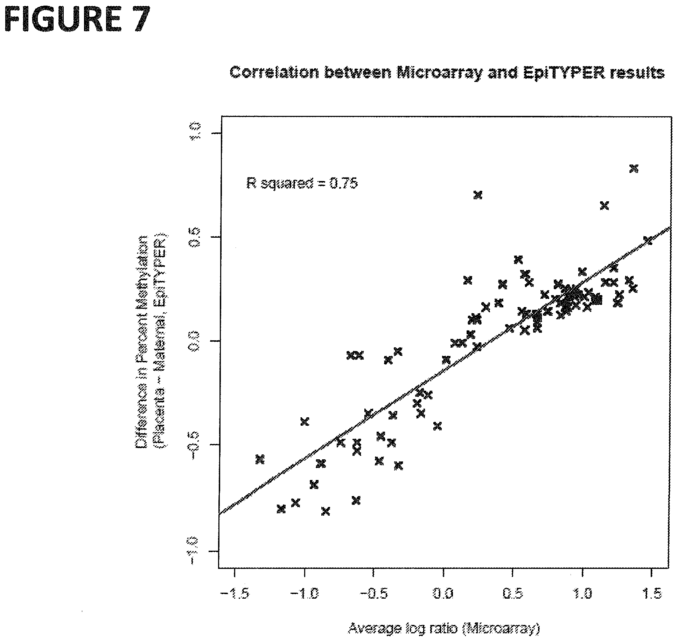

To validate the microarray findings, 63 regions from chromosomes 13, 18 and 21 and an additional 26 regions from other autosomes were selected for confirmation by a different technology. Sequenom EpiTYPER™ technology was used to quantitatively measure DNA methylation in maternal and placental samples. For an explanation of the EpiTYPER™ methods, see Ehrich M, Nelson MR, Stanssens P, Zabeau M, Liloglou T, Xinarianos G, Cantor CR, Field JK, van den Boom D (2005) Quantitative high-throughput analysis of DNA methylation patterns by base specific cleavage and mass spectrometry. Proc Natl Acad Sci U S A 102:15785-15790). For each individual CpG site in a target region the average methylation value across all maternal DNA samples and across all placenta samples was calculated. The difference between average maternal and placenta methylation was then compared to the microarray results. The results from the two technologies were in good concordance (see Figure7). For 85 target regions the quantitative results confirm the microarray results (95% confirmation rate). For 4 target regions, all located on chromosome 18, the results could not be confirmed. The reason for this discrepancy is currently unclear.

In contrast to microarrays, which focus on identification of methylation differences, the quantitative measurement of DNA methylation allowed analysis of absolute methylation values. In the validation set of 85 confirmed differentially methylated regions, a subset of 26 regions is more methylated in the maternal DNA sample and 59 regions are more methylated in the placental sample (see Table 1). Interestingly, genes that are hypomethylated in the placental samples tend to show larger methylation differences than genes that are hypermethylated in the placental sample (median methylation difference for hypomethylated genes = 39%, for hypermethylated genes = 20%).

### Example 2

Example 2 describes a non-invasive approach for detecting the amount of fetal nucleic acid present in a maternal sample (herein referred to as the "Fetal Quantifier Method"), which may be used to detect or confirm fetal traits (e.g., fetal sex of RhD compatibility), or diagnose chromosomal abnormalities such as Trisomy 21 (both of which are herein referred to as the "Methylation-Based Fetal Diagnostic Method"). Figure 10 shows one example of the Fetal Quantifier Method, and Figure 11 shows one example of the Methylation-Based Fetal Diagnostic Method. Both processes use fetal DNA obtained from a maternal sample. The sample comprises maternal and fetal nucleic acid that is differentially methylated. For example, the sample may be maternal plasma or serum. Fetal DNA comprises approximately 2-30% of the total DNA in maternal plasma. The actual amount of fetal contribution to the total nucleic acid present in a sample varies from pregnancy to pregnancy and can change based on a number of factors, including, but not limited to, gestational age, the mother's health and the fetus' health.

As described herein, the technical challenge posed by analysis of fetal DNA in maternal plasma lies in the need to be able to discriminate the fetal DNA from the co-existing background maternal DNA. The methods of the present technology exploit such differences, for example, the differential methylation that is observed between fetal and maternal DNA, as a means to enrich for the relatively small percentage of fetal DNA present in a sample from the mother. The non-invasive nature of the approach provides a major advantage over conventional methods of prenatal diagnosis such as, amniocentesis, chronic villus sampling and cordocentesis, which are associated with a small but finite risk of fetal loss. Also, because the method is not dependent on fetal cells being in any particular cell phase, the method provides a rapid detection means to determine the presence and also the nature of the chromosomal abnormality. Further, the approach is sex-independent (i.e., does not require the presence of a Y-chromosome) and polymorphic-independent (i.e., an allelic ratio is not determined). Thus, the compositions and methods of the technology represent improved universal, noninvasive approaches for accurately determining the amount of fetal nucleic acid present in a maternal sample.

### Assay design and advantages

There is a need for accurate detection and quantification of fetal DNA isolated noninvasively from a maternal sample. The present technology takes advantage of the presence of circulating, cell free fetal nucleic acid (ccfDNA) in maternal plasma or serum. In order to be commercially and clinically practical, the methods of the technology should only consume a small portion of the limited available fetal DNA. For example, less than 50%, 40%, 30%, 25%, 20%, 15%, 10%, 5% or less of the sample. Further, the approach should preferably be developed in a multiplex assay format in which one or more (preferably all) of the following assays are included:
- Assays for the detection of total amount of genomic equivalents present in the sample, i.e., assays recognizing both maternal and fetal DNA species;
- Assays for the detection of fetal DNA isolated from a male pregnancy, i.e., sequences specific for chromosome Y;
- Assays specific for regions identified as differentially methylated between the fetus and mother; or
- Assays specific for regions known to be hypomethylated in all tissues to be investigated, which can serve as a control for restriction efficiency.

Other features of the assay may include one or more of the following:
- For each assay, a target-specific, competitor oligonucleotide that is identical, or substantially identical, to the target sequence apart from a distinguishable feature of the competitor, such as a difference in one or more nucleotides relative to the target sequence. This oligonucleotide when added into the PCR reaction will be co-amplified with the target and a ratio obtained between these two PCR amplicons will indicate the number of target specific DNA sequences (e.g., fetal DNA from a specific locus) present in the maternal sample.
- The amplicon lengths should preferably be of similar length in order not to skew the amplification towards the shorter fragments. However, as long as the amplification efficiency is about equal, different lengths may be used.
- Differentially methylated targets can be selected from Table 1 or from any other targets known to be differentially methylated between mother and fetus. These targets can be hypomethylated in DNA isolated from non-pregnant women and hypermethylated in samples obtained from fetal samples. These assays will serve as controls for the restriction efficiency.
- The results obtained from the different assays can be used to quantify one or more of the following:
   ∘ Total number of amplifiable genomes present in the sample (total amount of genomic equivalents);
   ∘ The fetal fraction of the amplifiable genomes (fetal concentration or percentage); or
   ∘ Differences in copy number between fetally-derived DNA sequences (for example, between fetal chromosome 21 and a reference chromosome such as chromosome 3).

### Examples of assays used in the test

Below is an outline of the reaction steps used to perform a method of the technology, for example, as provided in Figure 10. This outline is not intended to limit the scope of the technology. Rather it provides one example of the technology using the Sequenom® MassARRAY® technology.
1) DNA isolation from plasma samples.
2) Digestion of the DNA targets using methylation sensitive restriction enzymes (for example, Hhal and Hpall).
   For each reaction the available DNA was mixed with water to a final volume of 25 ul.
   10 ul of a reaction mix consisting of 10 units Hhal, 10 units Hpall and a reaction buffer were added. The sample was incubated at an optimal temperature for the restriction enzymes. Hhal and Hpall digest non-methylated DNA (and will not digest hemi- or completely methylated DNA). Following digestion, the enzymes were denatured using a heating step.
3) Genomic Amplification- PCR was performed in a total volume of 50 ul by adding PCR reagents (Buffer, dNTPs, primers and polymerase). Exemplary PCR and extend primers are provided below. In addition, synthetic competitor oligonucleotide was added at known concentrations.
4) Replicates (optional) - Following PCR the 50 ul reaction was split into 5 ul parallel reactions (replicates) in order to minimize variation introduced during the post PCR steps of the test. Post PCR steps include SAP, primer extension (MassEXTEND® technology), resin treatment, dispensing of spectrochip and MassARRAY.
5) Quantification of the Amplifiable Genomes - Sequenom MassARRAY® technology was used to determine the amount of amplification product for each assay. Following PCR, a single base extension assay was used to interrogate the amplified regions (including the competitor oligonucleotides introduced in step 3). Specific extend primers designed to hybridize directly adjacent to the site of interest were introduced. See extend primers provided below. These DNA oligonucleotides are referred to as iPLEX® MassEXTEND® primers. In the extension reaction, the iPLEX primers were hybridized to the complementary DNA templates and extended with a DNA polymerase. Special termination mixtures that contain different combinations of deoxy- and dideoxynucleotide triphosphates along with enzyme and buffer, directed limited extension of the iPLEX primers. Primer extension occurs until a complementary dideoxynucleotide is incorporated.
   The extension reaction generated primer products of varying length, each with a unique molecular weight. As a result, the primer extension products can be simultaneously separated and detected using Matrix Assisted Laser Desorption/Ionization, Time-Of-Flight (MALDI-TOF) mass spectrometry on the MassARRAY® Analyzer Compact. Following this separation and detection, SEQUENOM's proprietary software automatically analyzes the data.
6) Calculating the amount and concentration of fetal nucleic acid - Methods for calculating the total amount of genomic equivalents present in the sample, the amount (and concentration) of fetal nucleic acid isolated from a male pregnancy, and the amount (and concentration) of fetal nucleic based on differentially methylated targets are provided below and in Figures 18 and 19.

The above protocol can be used to perform one or more of the assays described below. In addition to the sequences provided immediately below, a multiplex scheme that interrogates multiple is provided in Table X below.

### 1) Assay for the quantification of the total number of amplifiable genomic equivalents in the sample.

Targets were selected in housekeeping genes not located on the chromosomes 13, 18, 21, X or Y. The targets should be in a single copy gene and not contain any recognition sites for the methylation sensitive restriction enzymes.

Underlined sequences are PCR primer sites, italic is the site for the single base extend primer and bold letter (C) is the nucleotide extended on human DNA
ApoE Chromosome 19:45409835-45409922 DNA target sequence with interrogated nucleotide C in bold. All of the chromosome positions provided in this section are from the February 2009 UCSC Genome Build.
ApoE Forward Primer: 5'-ACGTTGGATG-TTGACAGTTTCTCCTTCCCC (Primer contains a 5' 10 bp MassTag separated by a dash)
ApoE Reverse Primer: 5'-ACGTTGGATG-GAATGTGACCAGCAACGCAG (Primer contains a 5' 10 bp MassTag separated by a dash)
ApoE Extension Primer: 5'-GCAGGAAGATGAAGGTT [C/T] Primer extends C on human DNA targets and T on synthetic DNA targets
ApoE synthetic competitor oligonucleotide: 5'-GATTGACAGTTTCTCCTTCCCCAGACTGGCCAATCACAGGCAGGAAGATGAAGGTTTTGTGGGCTGCGTTGCTGG TCACATTCCTGGC (Bold T at position 57 is different from human DNA)

### 2) Assay for the quantification of the total number of chromosome Y sequences in the sample.

Targets specific for the Y-chromosome were selected, with no similar or paralog sequences elsewhere in the genome. The targets should preferably be in a single copy gene and not contain any recognition sites for the methylation sensitive restriction enzyme(s).

Underlined sequences are PCR primer sites, and italic nucleotide(s) is the site for the single-base extend primer and bold letter (C) is the nucleotide extended on human DNA.
SRY on chrY:2655628-2655717 (reverse complement)
SRY Forward Primer: 5'-ACG-TGGATAGTAAAATAAGTTTCGAACTCTG (Primer contains a 5' 3 bp MassTag separated by a dash)
SRY Reverse Primer: 5'- GAAGCATATGATTGCATTGTCAAAAAC
SRY Extension Primer: 5'-aTTTCAATTTTGTCGCACT [C/T] Primer extends C on human DNA targets and T on synthetic DNA targets. 5' Lower case "a" is a non-complementary nucleotide
SRY synthetic competitor oligonucleotide: 5'-

### 3) Assay for the quantification of fetal methylated DNA sequences present in the sample.

Targets were selected in regions known to be differentially methylated between maternal and fetal DNA. Sequences were selected to contain several restriction sites for methylation sensitive enzymes. For this study the Hhal (GCGC) and Hpall (CCGG) enzymes were used.

Underlined sequences are PCR primer sites, italic is the site for the single base extend primer and bold letter (C) is the nucleotide extended on human DNA, lower case letter are recognition sites for the methylation sensitive restriction enzymes.
TBX3 on chr12:115124905-115125001
TBX3 Forward Primer: 5'- ACGTTGGATG-TCTTTGTCTCTGCGTGCCC (Primer contains a 5' 10 bp MassTag separated by a dash)
TBX3 Reverse Primer: 5'- ACGTTGGATG-TTAATCACCCAGCGCATGGC (Primer contains a 5' 10 bp MassTag separated by a dash)
TBX3 Extension Primer: 5'- CCCCTCCCGGTGGGTGATAAA [C/T] Primer extends C on human DNA targets and T on synthetic DNA targets. 5' Lower case "a" is a non-complementary nucleotide
TBX3 synthetic competitor oligonucleotide: 5'-

### 4) Control Assay for the enzyme restriction efficiency.

Targets were selected in regions known not to be methylated in any tissue to be investigated. Sequences were selected to contain no more than one site for each restriction enzyme to be used.

Underlined sequences are PCR primer sites, italic nucleotide(s) represent the site for the single-base extend primer and bold letter (G) is the reverse nucleotide extended on human DNA, lower case letter are recognition sites for the methylation sensitive restriction enzymes.
CACNA1G chr17:48637892-48637977 (reverse complement)
Hhal Forward Primer: 5'- ACGTTGGATG-CCATTGGCCGTCCGCCGTG (Primer contains a 5' 10 bp MassTag separated by a dash)
Hhal Reverse Primer: 5'- ACGTTGGATG-TCCTGGGTTTTGGGGTGGGAA (Primer contains a 5' 10 bp MassTag separated by a dash)
Hhal Extension Primer: 5'-TTCCAGCTGTGGTTCTCTC
Hhal synthetic competitor oligonucleotide: 5'-

### Validation experiments

The sensitivity and accuracy of the present technology was measured using both a model system and clinical samples. In the different samples, a multiplex assay was run that contains 2 assays for total copy number quantification, 3 assays for methylation quantification, 1 assay specific for chromosome Y and 1 digestion control assay. See Tables X1 and X2. Another multiplex scheme with additional assays is provided in Tables Y1 and Y2.

**TABLE X1: PCR Primers and Extend Primers**

| Gene ID | * | First Primer | Second Primer | Extend Primer |
|---|---|---|---|---|
| SOX14 | M | ACGTTGGATGACATGGTCGGCCCCACGGAAT | ACGTTGGATGCTCCTTCCTAGTGTGAGAACCG | CAGGTTCCGGGGCTTGGG |
| Hhal_CTRL | D | ACGTTGGATGACCCATTGGCCGTCCGCCGT | ACGTTGGATGTTTTGGGGTGGGAATCGGATT | CGCAGGGAGAGAACCACAG |
| TBX3 | M | ACGTTGGATGGAACTCCTCTTTGTCTCTGCG | ACGTTGGATGTGGCATGGCCGGCGCCAGA | CCCCTCCCGGTGGGTGATAAA |
| SRY | Y | ACGTTGGATGCGCAGCAACGGGACCGCTACA | ACGTTGGCATCTAGGTAGGTCTTTGTAGCCAA | AAAGCTGTAGGACAATCGGGT |
| ALB | T | ACGTTGCGTAGCAACCTGTTACATATTAA | ACGTTGGATCTGAGCAAAGGCAATCAACACCC | CATTTTTCTACATCCTTTGTTT |
| EDG6 | M | ACGTTGGATGCATAGAGGCCCATGATGGTGG | ACGTTGGATGACCTTCTGCCCCTCTACTCCAA | agAAGATCACCAGGCAGAAGAGG |
| RNaseP | T | ACGTTGGATGGTGTGGTCAGCTCTTCCCTTCAT | ACGTTGGCCCACATGTAATGTGTTGAAAAAGCA | ACTTGGAGAACAAAGGACACCGTTA |

**TABLE X2: Competitor Oligonucleotide Sequence**

| Gene ID | * | Competitor Oligonucleotide Sequence |
|---|---|---|
| SOX14 | M | GGTCGGCCCCACGGAATCCCGGCTCTGTGTGCGCCCAGGTTCCGGGGCTTGGGTGTTGCCGGTTCTCACACTAGGAAGGAG |
| Hhal_CTRL | D | CCATTGGCCGTCCGCCGTGGCAGTGCGGGCGGGAGCGCAGAGAGAGAACCACAGCTGGAATCCGATTCCCACCCCAAAA |
| TBX3 | M | GAACTCCTCTTTGTCTCTGCGTGCCCGGCGCGCCCCCCTCCCGGTGGGTGATAAATCCACTCTGGCGCCGGCCATGC |
| SRY | Y | GCAGCAACGGGACCGCTACAGCCACTGGACAAAGCCGTAGGACAATCGGGTAACATTGGCTACAAAGACCTACCTAGATGC |
| ALB | T | GCGTAGCAACCTGTTACATATTAAAGTTTTATTATACTACATTTTTCTACATCCTTTGTTTCAGAGTGTTGATTGCCTTTGCTCAGTATCTTCAG |
| EDG6 | M | CCTTCTGCCCCTCTACTCCAAGCGCTACACCCTCTTCTGCCTGGTGATCTTTGCCGGCGTCCTGGCCACCATCATGGGCCTCTATG |
| RNaseP | T | GTGTGGTCAGCTCTTCCCTTCATCACATACTTGGAGAACAAAGGACACCGTTATCCATGCTTTTTCAACACATTACATGTGGG |

**TABLE Y1: PCR Primers and Extend Primers**

| Gene ID | * | First Primer | Second Primer | Extend Primer |
|---|---|---|---|---|
| EDG6 | M | ACGTTGGATGTTCTGCCCCTCTACTCCAAG | ACGTTGGATGCATAGAGGCCCATGATGGTG | TTCTGCCTGGTGATCTT |
| RNAseP | T | ACGTTGGATGTCAGCTCTTCCCTTCATCAC | ACGTTGGATGCCTACCTCCCACATGTAATGT | AACAAAGGACACCGTTA |
| ApoE | T | ACGTTGGATGTTGACAGTTTCTCCTTCCCC | ACGTTGGATGGAATGTGACCAGCAACGCAG | GCAGGAAGATGAAGGTT |
| SOX14 | M | ACGTTGGATGCGGTCGGCCCCACGGAAT | ACGTTGGATGCTCCTTCCTAGTGTGAGAACCG | aAGGTTCCGGGGCTTGGG |
| SRY no2 | Y | ACGTGGATAGTAAAATAAGTTTCGAACTCTG | GAAGCATATGATTGCATTGTCAAAAAC | aTTTCAATTTTGTCGCACT |
| SRY no1 | Y | ACGTTGGATGCACAGCTCACCGCAGCAACG | ACGTTGGATGCTAGGTAGGTCTTTGTAGCCAA | AGCTGTAGGACAATCGGGT |
| TBX3 | M | ACGTTGGATGTCTTTGTCTCTGCGTGCCC | ACGTTGGATGTTAATCACCCAGCGCATGGC | CCCTCCCGGTGGGTGATAAA |
| CACNA1G dig CTRL 1 | D | ACGTTGGATGGACTGAGCCCCAGAACTCG | ACGTTGGATGGTGGGTTTGTGCTTTCCACG | AGGGCCGGGGTCTGCGCGTG |
| DAPK1 dig CTRL 2 | D | ACGTTGGATGAAGCCAAGTTTCCCTCCGC | ACGTTGGATGCTTTTGCTTTCCCAGCCAGG | GAGGCACTGCCCGGACAAACC |
| ALB | T | ACGTTAGCGTAGCAACCTGTTACATATTAA | ACGTTGGATGCTGAGCAAAGGCAATCAACA | CATTTTTCTACATCCTTTGTTT |

**TABLE Y2: Competitor Oligonucleotide Sequence**

| Gene ID | * | Competitor |
|---|---|---|
| EDG6 | M | CCTTCTGCCCCTCTACTCCAAGCGCTACACCCTCTTCTGCCTGGTGATCTTTGCCGGCGTCCTGGCCACCATCATGGGCCTCTATG |
| RNAseP | T | GTGTGGTCAGCTCTTCCCTTCATCACATACTTGGAGAACAAAGGACACCGTTATCCATGCTTTTTCAACACATTACATGTGGGAGGTAGG |
| ApoE | T | GATTGACAGTTTCTCCTTCCCCAGACTGGCCAATCACAGGCAGGAAGATGAAGGTTTTGTGGGCTGCGTTGCTGGTCACATTCCTGGC |
| SOX14 | M | |
| SRY no2 | Y | GAGTTTTGGATAGTAAAATAAGTTTCGAACTCTGGCACCTTTCAATTTTGTCGCACTTTCCTTGTTTTTGACAATGCAATCATATGCTTC |
| SRY no1 | Y | GCAGCCAGCTCACCGCAGCAACGGGACCGCTACAGCCACTGGACAAAGCTGTAGGACAATCGGGTGACATTGGCTACAAAGACCTACCTAGATGC |
| TBX3 | M | |
| CACNA1G dig CTRL 1 | D | GTGGGTTTGTGCTTTCCACGCGTGCACACACACGCGCAGACCCCGGCCCTTGCCCCGCCTACCTCCCCGAGTTCTGGGGCTCAGTC |
| DAPK1 dig CTRL 2 | D | GCGCCAGCTTTTGCTTTCCCAGCCAGGGCGCGGTGAGGTTTGTCCGGGCAGTGCCTCGAGCAACTGGGAAGGCCAAGGCGGAGGGAAAC |
| ALB | T | GCGTAGCAACCTGTTACATATTAAAGTTTTATTATACTACATTTTTCTACATCCTTTGTTTTAGGGTGTTGATTGCCTTTGCTCAGTATCTTCAGC |

| | | |
|---|---|---|
| T=Assay for Total Amount; M=Assay for Methylation quantification; Y= Y-Chromosome Specific Assay; D=Digestion control | | |

### Model system using genomic DNA

In order to determine the sensitivity and accuracy of the method when determining the total number of amplifiable genomic copies in a sample, a subset of different DNA samples isolated from the blood of non-pregnant women was tested. Each sample was diluted to contain approximately 2500, 1250, 625 or 313 copies per reaction. The total number of amplifiable genomic copies was obtained by taking the mean DNA/competitor ratio obtained from the three total copy number assays. The results from the four different samples are shown in Figure 12.

To optimize the reaction, a model system was developed to simulate DNA samples isolated from plasma. These samples contained a constant number of maternal non-methylated DNA and were spiked with different amounts of male placental methylated DNA. The samples were spiked with amounts ranging from approximately 0 to 25% relative to the maternal non-methylated DNA. The results are shown in Figures 13A and B. The fraction of placental DNA was calculated using the ratios obtained from the methylation assays (Figure 13A), the SRY markers (Figure 13B) and the total copy number assays. The primer sequences for the methylation assays (TBX), Y-chromosome assays (SRY) and total copy number (APOE) are provided above. The model system demonstrated that the methylation-based method performed equal to the Y-chromosome method (SRY markers), thus validating the methylation-based method as a sex-independent fetal quantifier.

### Plasma samples

To investigate the sensitivity and accuracy of the methods in clinical samples, 33 plasma samples obtained from women pregnant with a male fetus were investigated using the multiplex scheme from Table X. For each reaction, a quarter of the DNA obtained from a 4ml extraction was used in order to meet the important requirement that only a portion of the total sample is used.

### Total copy number quantification

The results from the total copy number quantification can be seen in Figures 14A and B. In Figure 14A, the copy number for each sample is shown. Two samples (nos. 25 and 26) have a significantly higher total copy number than all the other samples. In general, a mean of approximately 1300 amplifiable copies/ml plasma was obtained (range 766-2055). Figure 14B shows a box-and-whisker plot of the given values, summarizing the results.

### Correlation between results obtained from the methylation markers and the Y-chromosome marker

In Figures 15A and B, the numbers of fetal copies for each sample are plotted. As all samples were from male pregnancies. The copy numbers obtained can be calculated using either the methylation or the Y-chromosome-specific markers. As can be seen in Figure 15B, the box-and-whisker plot of the given values indicated minimal difference between the two different measurements.

The results showing the correlation between results obtained from the methylation markers and the Y-chromosome marker (SRY) is shown in Figure 16. Again, the methylation-based method performed equal to the Y-chromosome method (SRY markers), further validating the methylation-based method as a sex-independent and polymorphism-independent fetal quantifier. The multiplexed assays disclosed in Table X were used to determine the amount fetal nucleic.

Finally, the digestion efficiency was determined by using the ratio of digestion for the control versus the competitor and comparing this value to the mean total copy number assays. See Figure 17. Apart from sample 26 all reactions indicate the efficiency to be above 99%.

### Data Analysis

Mass spectra analysis was done using Typer 4 (a Sequenom software product). The peak height (signal over noise) for each individual DNA analyte and competitor assay was determined and exported for further analysis.

The total number of molecules present for each amplicon was calculated by dividing the DNA specific peak by the competitor specific peak to give a ratio. (The "DNA" Peak in Figures 18 and 19 can be thought of as the analyte peak for a given assay). Since the number of competitor molecules added into the reaction is known, the total number of DNA molecules can be determined by multiplying the ratio by the number of added competitor molecules.

The fetal DNA fraction (or concentration) in each sample was calculated using the Y-chromosome-specific markers for male pregnancies and the mean of the methylated fraction for all pregnancies. In brief, for chromosome Y, the ratio was obtained by dividing the analyte (DNA) peak by the competitor peak and multiplying this ratio by the number of competitor molecules added into the reaction. This value was divided by a similar ratio obtained from the total number of amplifiable genome equivalents determination (using the Assay(s) for Total Amount). See Figure 18. Since the total amount of nucleic acid present in a sample is a sum of maternal and fetal nucleic acid, the fetal contribution can be considered to be a fraction of the larger, background maternal contribution. Therefore, translating this into the equation shown in Figure 18, the fetal fraction (k) of the total nucleic acid present in the sample is equal to the equation: k=2xR/(1-2R), where R is the ratio between the Y-chromosome amount and the total amount. Since the Y-chromosome is haploid and Assays for the Total Amount are determined using diploid targets, this calculation is limited to a fetal fraction smaller than 50% of the maternal fraction.

In Figure 19, a similar calculation for the fetal concentration is shown by using the methylation specific markers (see Assays for Methylation Quantification). In contrast to Y-chromosome specific markers, these markers are from diploid targets, therefore, the limitations stated for the Y-Chromosome Specific Assay can be omitted. Thus, the fetal fraction (k) can be determined using the equation: k=R(1-R), where R is the ratio between the methylation assay and the total assay.

### Simulation

A first simple power calculation was performed that assumes a measurement system that uses 20 markers from chromosome 21, and 20 markers from one or more other autosomes. Starting with 100 copies of fetal DNA, a measurement standard deviation of 25 copies and the probability for a type I error to be lower than 0.001, it was found that the methods of the technology will be able to differentiate a diploid from a triploid chromosome set in 99.5% of all cases. The practical implementation of such an approach could for example be achieved using mass spectrometry, a system that uses a competitive PCR approach for absolute copy number measurements. The method can run 20 assays in a single reaction and has been shown to have a standard deviation in repeated measurements of around 3 to 5%. This method was used in combination with known methods for differentiating methylated and non-methylated nucleic acid, for example, using methyl-binding agents to separate nucleic acid or using methylation-sensitive enzymes to digest maternal nucleic acid. Figure 8 shows the effectiveness of MBD-FC protein (a methyl-binding agent) for capturing and thereby separating methylated DNA in the presence of an excess of unmethylated DNA (see Figure 8).

A second statistical power analysis was performed to assess the predictive power of an example of the Methylation-Based Fetal Diagnostic Method described herein. The simulation was designed to demonstrate the likelihood of differentiating a group of trisomic chromosome 21 specific markers from a group of reference markers (for example, autosomes excluding chromosome 21). Many parameters influence the ability to discriminate the two populations of markers reliably. For the present simulation, values were chosen for each parameter that have been shown to be the most likely to occur based on experimentation. The following parameters and respective values were used:
Copy Numbers
   Maternal copy numbers = 2000
   Fetal copy numbers for chromosomes other than 21, X and Y = 200
   Fetal copy numbers for chromosome 21 in case of euploid fetus = 200
   Fetal copy numbers for chromosome 21 in case of aneuploid T21 fetus = 300
Percent fetal DNA (before methylation-based enrichment) = 10% (see above)
Methylation Frequency
   Average methylation percentage in a target region for maternal DNA = 10%
   Average methylation percentage in a target region for fetal DNA = 80%
Average percentage of non-methylated and non-digested maternal DNA (i.e., a function of restriction efficiency (among other things) = 5%
Number of assays targeting chromosome 21 = 10
Number of assays targeting chromosomes other than 21, X and Y = 10

The results are displayed in Figure 20. Shown is the relationship between the coefficient of variation (CV) on the x-axis and the power to discriminate the assay populations using a simple t-test (y-axis). The data indicates that in 99% of all cases, one can discriminate the two population (euploid vs. aneuploid) on a significance level of 0.001 provided a CV of 5% or less.. Based on this simulation, the method represents a powerful noninvasive diagnostic method for the prenatal detection of fetal aneuploidy that is sex-independent and will work in all ethnicities (i.e., no allelic bias).

**TABLE 1**

| **GENE NAME** | **CHROM** | **START** | **END** | **CpG ISLAND** | **MEAN LOG RATIO MICRO-ARRAY** | **MEAN MATERNAL METHYLATION EPITYPER** | **MEAN PLACENTA METHYLATION EPITYPER** | **METHYLATION DIFFERENCE PLACENTA-MATERNAL** | **RELATIVE METHYLATION PLACENTA TO MATERNAL** |
|---|---|---|---|---|---|---|---|---|---|
| chr13 group00016 | chr13 | 19773745 | 19774050 | chr13:19773518-19774214 | 0.19 | 0.22 | 0.32 | 0.1 | HYPERMETHYLATION |
| chr13 group00005 | chr13 | 19290394 | 19290768 | :- | -0.89 | 0.94 | 0.35 | -0.59 | HYPOMETHYLATION |
| CRYL1 | chr13 | 19887090 | 19887336 | chr13:19887007-19887836 | -0.63 | 0.74 | 0.21 | -0.53 | HYPOMETHYLATION |
| IL17D | chr13 | 20193675 | 20193897 | chr13:20193611-20194438 | -1.01 | 0.53 | 0.13 | -0.39 | HYPOMETHYLATION |
| CENPJ | chr13 | 24404023 | 24404359 | :- | 0.57 | 0.17 | 0.49 | 0.32 | HYPERMETHYLATION |
| ATP8A2 | chr13 | 25484475 | 25484614 | chr13:25484287-25484761 | 0.81 | 0.16 | 0.43 | 0.27 | HYPERMETHYLATION |
| GSH1 | chr13 | 27265542 | 27265834 | chr13:27264549-27266505 | 0.57 | 0.13 | 0.19 | 0.05 | HYPERMETHYLATION |
| PDX1 | chr13 | 27393789 | 27393979 | chr13:27392001-27394099 | 0.55 | 0.06 | 0.2 | 0.14 | HYPERMETHYLATION |
| PDX1 | chr13 | 27400459 | 27401165 | chr13:27400362-27400744; chr13:27401057-27401374 | 0.73 | 0.12 | 0.26 | 0.14 | HYPERMETHYLATION |
| MAB21L1 | chr13 | 34947737 | 34948062 | chr13:34947570-34948159 | 0.66 | 0.11 | 0.17 | 0.06 | HYPERMETHYLATION |
| RB1 | chr13 | 47790983 | 47791646 | chr13:47790636-47791858 | 0.18 | 0.45 | 0.48 | 0.03 | HYPERMETHYLATION |
| PCDH17 | chr13 | 57104856 | 57106841 | chr13:57104527-57106931 | 0.46 | 0.15 | 0.21 | 0.06 | HYPERMETHYLATION |
| KLHL1 | chr13 | 69579933 | 69580146 | chr13:69579733-69580220 | 0.79 | 0.09 | 0.28 | 0.2 | HYPERMETHYLATION |
| POU4F1 | chr13 | 78079515 | 78081073 | chr13:78079328-78079615; chr13:78080860-78081881 | 0.66 | 0.12 | 0.23 | 0.11 | HYPERMETHYLATION |
| GPC6 | chr13 | 92677402 | 92678666 | chr13:92677246-92678878 | 0.66 | 0.06 | 0.19 | 0.13 | HYPERMETHYLATION |
| SOX21 | chr13 | 94152286 | 94153047 | chr13:94152190-94153185 | 0.94 | 0.16 | 0.4 | 0.25 | HYPERMETHYLATION |
| ZIC2 | chr13 | 99439660 | 99440858 | chr13:99439335-99440189; chr13:99440775-99441095 | 0.89 | 0.13 | 0.35 | 0.22 | HYPERMETHYLATION |
| IRS2 | chr13 | 109232856 | 109235065 | chr13:109232467-109238181 | -0.17 | 0.73 | 0.38 | -0.35 | HYPOMETHYLATION |
| chr13 group00350 | chr13 | 109716455 | 109716604 | chr13:109716325-109716726 | -0.37 | 0.77 | 0.41 | -0.36 | HYPOMETHYLATION |
| chr13 group00385 | chr13 | 111595578 | 111595955 | chr13:111595459-111596131 | 0.87 | 0.06 | 0.2 | 0.14 | HYPERMETHYLATION |
| chr13 group00390 | chr13 | 111756337 | 111756593 | chr13:111755805-111756697 | 0.71 | 0.12 | 0.34 | 0.22 | HYPERMETHYLATION |
| chr13 group00391 | chr13 | 111759856 | 111760045 | chr13:111757885-111760666 | 0.86 | 0.11 | 0.36 | 0.25 | HYPERMETHYLATION |
| chr13 group00395 | chr13 | 111808255 | 111808962 | chr13:111806599-111808492; chr13:111808866-111809114 | 0.96 | 0.13 | 0.35 | 0.22 | HYPERMETHYLATION |
| chr13 group00399 | chr13 | 112033503 | 112033685 | chr13:112032967-112033734 | 0.38 | 0.26 | 0.43 | 0.18 | HYPERMETHYLATION |
| MCF2L | chr13 | 112724910 | 112725742 | chr13:112724782-112725121; chr13:112725628-112725837 | -0.47 | 0.91 | 0.33 | -0.58 | HYPOMETHYLATION |
| F7 | chr13 | 112799123 | 112799379 | chr13:112798487-112799566 | -0.05 | 0.97 | 0.55 | -0.41 | HYPOMETHYLATION |
| PROZ | chr13 | 112855566 | 112855745 | chr13:112855289-112855866 | 0.29 | 0.15 | 0.3 | 0.16 | HYPERMETHYLATION |
| chr18 group00039 | chr18 | 6919797 | 6919981 | chr18:6919450-6920088 | -0.38 | 0.88 | 0.39 | -0.49 | HYPOMETHYLATION |
| CIDEA | chr18 | 12244327 | 12244696 | chr18:12244147-12245089 | 0.23 | 0.14 | 0.23 | 0.1 | HYPERMETHYLATION |
| chr18 group00091 | chr18 | 12901467 | 12901643 | chr18:12901024-12902704 | 0.16 | 0.15 | 0.43 | 0.29 | HYPERMETHYLATION |
| chr18 group00094 | chr18 | 13126819 | 13126986 | chr18:13126596-13127564 | 0.41 | 0.07 | 0.34 | 0.27 | HYPERMETHYLATION |
| C18orf1 | chr18 | 13377536 | 13377654 | chr18:13377385-13377686 | -0.12 | 0.95 | 0.69 | -0.26 | HYPOMETHYLA TION |
| KLHL14 | chr18 | 28603978 | 28605183 | chr18:28603688-28606300 | 0.83 | 0.07 | 0.19 | 0.12 | HYPERMETHYLATION |
| CD33L3 | chr18 | 41671477 | 41673011 | chr18:41671386-41673101 | -0.34 | 0.49 | 0.44 | -0.05 | HYPOMETHYLATION |
| ST8SIA3 | chr18 | 53171265 | 53171309 | chr18:53170705-53172603 | 1.02 | 0.09 | 0.25 | 0.16 | HYPERMETHYLATION |
| ONECUT2 | chr18 | 53254808 | 53259810 | chr18:53254152-53259851 | 0.74 | 0.09 | 0.23 | 0.14 | HYPERMETHYLATION |
| RAX | chr18 | 55086286 | 55086436 | chr18:55085813-55087807 | 0.88 | 0.11 | 0.26 | 0.16 | HYPERMETHYLATION |
| chr18 group00277 | chr18 | 57151972 | 57152311 | chr18:57151663-57152672 | 0.58 | 0.08 | 0.21 | 0.13 | HYPERMETHYLATION |
| TNFRSF11A | chr18 | 58203013 | 58203282 | chr18:58202849-58203367 | -0.33 | 0.88 | 0.28 | -0.6 | HYPOMETHYLATION |
| NETO1 | chr18 | 68685099 | 68687060 | chr18:68684945-68687851 | 0.65 | 0.09 | 0.22 | 0.13 | HYPERMETHYLATION |
| chr18 group00304 | chr18 | 70133945 | 70134397 | chr18:70133732-70134724 | 0.12 | 0.93 | 0.92 | -0.01 | NOT CONFIRMED |
| TSHZ1 | chr18 | 71128742 | 71128974 | chr18:71128638-71129076 | 0.23 | 0.95 | 0.92 | -0.03 | NOT CONFIRMED |
| ZNF236 | chr18 | 72664454 | 72664736 | chr18:72662797-72664893 | -0.62 | 0.17 | 0.1 | -0.07 | HYPOMETHYLATION |
| MBP | chr18 | 72953150 | 72953464 | chr18:72953137-72953402 | 0.6 | 0.44 | 0.72 | 0.28 | HYPERMETHYLATION |
| chr18 group00342 | chr18 | 74170347 | 74170489 | chr18:74170210-74170687 | -0.2 | 0.78 | 0.48 | -0.3 | HYPOMETHYLATION |
| NFATC1 | chr18 | 75385424 | 75386008 | chr18:75385279-75386532 | 0.23 | 0.14 | 0.84 | 0.7 | HYPERMETHYLATION |
| CTDP1 | chr18 | 75596358 | 75596579 | chr18:75596009-75596899 | 0.07 | 0.97 | 0.96 | -0.01 | NOT CONFIRMED |
| chr18 group00430 | chr18 | 75653272 | 75653621 | :- | 0.52 | 0.24 | 0.62 | 0.39 | HYPERMETHYLATION |
| KCNG2 | chr18 | 75760343 | 75760820 | chr18:75759900-75760988 | 0.01 | 0.84 | 0.75 | -0.09 | NOT CONFIRMED |
| OLIG2 | chr21 | 33317673 | 33321183 | chr21:33316998-33322115 | 0.66 | 0.11 | 0.2 | 0.09 | HYPERMETHYLATION |
| OLIG2 | chr21 | 33327593 | 33328334 | chr21:33327447-33328408 | -0.75 | 0.77 | 0.28 | -0.49 | HYPOMETHYLATION |
| RUNX1 | chr21 | 35180938 | 35185436 | chr21 :35180822-35181342; chr21:35182320-35185557 | -0.68 | 0.14 | 0.07 | -0.07 | HYPOMETHYLATION |
| SIM2 | chr21 | 36994965 | 36995298 | chr21:36990063-36995761 | 0.83 | 0.08 | 0.26 | 0.18 | HYPERMETHYLATION |
| SIM2 | chr21 | 36999025 | 36999410 | chr21:36998632-36999555 | 0.87 | 0.06 | 0.24 | 0.18 | HYPERMETHYLATION |
| DSCR6 | chr21 | 37300407 | 37300512 | chr21:37299807-37301307 | 0.22 | 0.04 | 0.14 | 0.11 | HYPERMETHYLATION |
| DSCAM | chr21 | 41135559 | 41135706 | chr21:41135380-41135816 | 1.03 | 0.06 | 0.29 | 0.23 | HYPERMETHYLATION |
| chr21 group00165 | chr21 | 43643421 | 43643786 | chr21:43643322-43643874 | 1.14 | 0.16 | 0.81 | 0.65 | HYPERMETHYLATION |
| AIRE | chr21 | 44529935 | 44530388 | chr21:44529856-44530472 | -0.55 | 0.62 | 0.27 | -0.35 | HYPOMETHYLATION |
| SUMO3 | chr21 | 45061293 | 45061853 | chr21:45061154-45063386 | -0.41 | 0.55 | 0.46 | -0.09 | HYPOMETHYLATION |
| C21orf70 | chr21 | 45202815 | 45202972 | chr21:45202706-45203073 | -0.46 | 0.96 | 0.51 | -0.46 | HYPOMETHYLATION |
| C21orf123 | chr21 | 45671984 | 45672098 | chr21:45671933-45672201 | -0.63 | 0.92 | 0.43 | -0.49 | HYPOMETHYLATION |
| COL18A1 | chr21 | 45754383 | 45754487 | chr21:45753653-45754639 | -0.18 | 0.97 | 0.72 | -0.25 | HYPOMETHYLATION |
| PRMT2 | chr21 | 46911967 | 46912385 | chr21:46911628-46912534 | 1.08 | 0.04 | 0.25 | 0.21 | HYPERMETHYLATION |
| SIX2 | chr2 | 45081223 | 45082129 | chr2:45081148-45082287 | 1.15 | 0.08 | 0.36 | 0.28 | HYPERMETHYLATION |
| SIX2 | chr2 | 45084851 | 45085711 | chr2:45084715-45084986; chr2:45085285-45086054 | 1.21 | 0.07 | 0.35 | 0.28 | HYPERMETHYLATION |
| SOX14 | chr3 | 138971870 | 138972322 | chr3:138971738-138972096; chr3:138972281-138973691 | 1.35 | 0.08 | 0.33 | 0.25 | HYPERMETHYLATION |
| TLX3 | chr5 | 170674439 | 170676431 | ch r5:170674208-170675356; chr5:170675783-170676712 | 0.91 | 0.11 | 0.35 | 0.24 | HYPERMETHYLATION |
| FOXP4 | chr6 | 41623666 | 41624114 | chr6:41621630-41624167 | 1.1 | 0.07 | 0.27 | 0.2 | HYPERMETHYLATION |
| FOXP4 | chr6 | 41636384 | 41636779 | chr6:41636244-41636878 | 1.32 | 0.04 | 0.33 | 0.29 | HYPERMETHYLATION |
| chr7 group00267 | chr7 | 12576755 | 12577246 | chr7:12576690-12577359 | 0.94 | 0.08 | 0.26 | 0.17 | HYPERMETHYLATION |
| NPY | chr7 | 24290224 | 24291508 | chr7:24290083-24291605 | 0.93 | 0.09 | 0.3 | 0.21 | HYPERMETHYLATION |
| SHH | chr7 | 155291537 | 155292091 | chr7:155288453-155292175 | 0.98 | 0.19 | 0.52 | 0.33 | HYPERMETHYLATION |
| OSR2 | chr8 | 100029764 | 100030536 | chr8:100029673-100030614 | 1.21 | 0.08 | 0.43 | 0.35 | HYPERMETHYLATION |
| GLIS3 | chr9 | 4288283 | 4289645 | chr9:4287817-4290182 | 1.24 | 0.06 | 0.24 | 0.18 | HYPERMETHYLATION |
| PRMT8 | chr12 | 3472714 | 3473190 | chr12:3470227-3473269 | 0.86 | 0.07 | 0.23 | 0.16 | HYPERMETHYLATION |
| TBX3 | chr12 | 113609153 | 113609453 | chr12:113609112-113609535 | 1.45 | 0.09 | 0.56 | 0.48 | HYPERMETHYLATION |
| chr12 group00801 | chr12 | 118516189 | 118517435 | chr12:118515877-118517595 | 1.1 | 0.06 | 0.25 | 0.19 | HYPERMETHYLATION |
| PAX9 | chr14 | 36201402 | 36202386 | chr14:36200932-36202536 | 0.89 | 0.11 | 0.32 | 0.21 | HYPERMETHYLATION |
| SIX1 | chr14 | 60178801 | 60179346 | chr14:60178707-60179539 | 0.95 | 0.1 | 0.33 | 0.22 | HYPERMETHYLATION |
| ISL2 | chr15 | 74420013 | 74421546 | chr15:74419317-74422570 | 1.08 | 0.08 | 0.27 | 0.19 | HYPERMETHYLATION |
| DLX4 | chr17 | 45397228 | 45397930 | chr17:45396281-45398063 | 1.25 | 0.1 | 0.32 | 0.22 | HYPERMETHYLATION |
| CBX4 | chr17 | 75428613 | 75431793 | chr17:75427586-75433676 | 1 | 0.07 | 0.27 | 0.21 | HYPERMETHYLATION |
| EDG6 | chr19 | 3129836 | 3130874 | chr19:3129741-3130986 | 1.35 | 0.04 | 0.87 | 0.83 | HYPERMETHYLATION |
| PRRT3 | chr3 | 9963364 | 9964023 | chr3:9962895-9964619 | -0.85 | 0.9 | 0.09 | -0.81 | HYPOMETHYLA TION |
| MGC29506 | chr5 | 138757911 | 138758724 | chr5:138755609-138758810 | -0.63 | 0.93 | 0.17 | -0.76 | HYPOMETHYLATION |
| TEAD3 | chr6 | 35561812 | 35562252 | chr6:35561754-35562413 | -1.17 | 0.92 | 0.13 | -0.8 | HYPOMETHYLA TION |
| chr12 group00022 | chr12 | 1642456 | 1642708 | chr12:1642195-1642774 | -1.33 | 0.66 | 0.09 | -0.57 | HYPOMETHYLATION |
| CENTG1 | chr12 | 56406249 | 56407788 | chr12:56406176-56407818 | -1.07 | 0.95 | 0.19 | -0.77 | HYPOMETHYLATION |
| CENTG1 | chr12 | 56416146 | 56418794 | chr12:56416095-56416628; chr12:56418745-56419001 | -0.94 | 0.85 | 0.16 | -0.69 | HYPOMETHYLATION |

| | | | | | | | | | |
|---|---|---|---|---|---|---|---|---|---|
| Information based on the March 2006 human reference sequence (NCBI Build 36.1), which was produced by the International Human Genome Sequencing Consortium. | | | | | | | | | |

**TABLE 2**

| **GENE NAME** | **CHROM** | **START** | **END** | **SNPs** |
|---|---|---|---|---|
| chr13 group00016 | chr13 | 19773745 | 19774050 | rs7996310; rs12870878 |
| chr13 group00005 | chr13 | 19290394 | 19290768 | rs11304938 |
| CENPJ | chr13 | 24404023 | 24404359 | rs7326661 |
| ATP8A2 | chr13 | 25484475 | 25484614 | rs61947088 |
| PDX1 | chr13 | 27400459 | 27401165 | rs58173592; rs55836809; rs61944011 |
| RB1 | chr13 | 47790983 | 47791646 | rs2804094; rs4151432; rs4151433; rs4151434; rs4151435 |
| PCDH17 | chr13 | 57104856 | 57106841 | rs35287822; rs34642962; rs41292834; rs45500496; rs45571031; rs41292836; rs28374395; rs41292838 |
| KLHL1 | chr13 | 69579933 | 69580146 | rs3751429 |
| POU4F1 | chr13 | 78079515 | 78081073 | rs11620410; rs35794447; rs2765065 |
| GPC6 | chr13 | 92677402 | 92678666 | rs35689696; rs11839555; rs55695812; rs35259892 |
| SOX21 | chr13 | 94152286 | 94153047 | rs41277652; rs41277654; rs35276096; rs5805873; rs35109406 |
| ZIC2 | chr13 | 99439660 | 99440858 | rs9585309; rs35501321; rs9585310; rs7991728; rs1368511 |
| IRS2 | chr13 | 109232856 | 109235065 | rs61747993; rs1805097; rs9583424; rs35927012; rs1056077; rs1056078; rs34889228; rs1056080; rs1056081; rs12853546; rs4773092; rs35223808; rs35894564; rs3742210; rs34412495; rs61962699; rs45545638; rs61743905 |
| chr13 group00395 | chr13 | 111808255 | 111808962 | rs930346 |
| MCF2L | chr13 | 112724910 | 112725742 | rs35661110; rs2993304; rs1320519; rs7320418; rs58416100 |
| F7 | chr13 | 112799123 | 112799379 | rs2480951; rs2476320 |
| CIDEA | chr18 | 12244327 | 12244696 | rs60132277 |
| chr18 group00091 | chr18 | 12901467 | 12901643 | rs34568924; rs8094284; rs8094285 |
| C18orf1 | chr18 | 13377536 | 13377654 | rs9957861 |
| KLHL14 | chr18 | 28603978 | 28605183 | rs61737323; rs61737324; rs12960414 |
| CD33L3 | chr18 | 41671477 | 41673011 | rs62095363; rs2919643 |
| ONECUT2 | chr18 | 53254808 | 53259810 | rs35685953; rs61735644; rs8084084; rs35937482; rs35427632; rs7232930; rs3786486; rs34286480; rs3786485; rs28655657; rs4940717; rs4940719; rs3786484; rs34040569; rs35542747; rs33946478; rs35848049; rs7231349; rs7231354; rs34481218; rs12962172; rs3911641 |
| RAX | chr18 | 55086286 | 55086436 | rs58797899; rs45501496 |
| chr18 group00277 | chr18 | 57151972 | 57152311 | rs17062547 |
| TNFRSF11A | chr18 | 58203013 | 58203282 | rs35114461 |
| NETO1 | chr18 | 68685099 | 68687060 | rs4433898; rs34497518; rs35135773; rs6566677; rs57425572; rs36026929; rs34666288; rs10627137; rs35943684; rs9964226; rs4892054; rs9964397; rs4606820; rs12966677; rs8095606 |
| chr18 group00304 | chr18 | 70133945 | 70134397 | rs8086706; rs8086587; rs8090367; rs999332; rs17806420; rs58811193 |
| TSHZ1 | chr18 | 71128742 | 71128974 | rs61732783; rs3744910; rs1802180 |
| chr18 group00342 | chr18 | 74170347 | 74170489 | rs7226678 |
| NFATC1 | chr18 | 75385424 | 75386008 | rs28446281; rs56384153; rs4531815; rs3894049 |
| chr18 group00430 | chr18 | 75653272 | 75653621 | rs34967079; rs35465647 |
| KCNG2 | chr18 | 75760343 | 75760820 | rs3744887; rs3744886 |
| OLIG2 | chr21 | 33317673 | 33321183 | rs2236618; rs11908971; rs9975039; rs6517135; rs2009130; rs1005573; rs1122807; rs10653491; rs10653077; rs35086972; rs28588289; rs7509766; rs62216114; rs35561747; rs7509885; rs11547332 |
| OLIG2 | chr21 | 33327593 | 33328334 | rs7276788; rs7275842; rs7275962; rs7276232; rs16990069; rs13051692; rs56231743; rs35931056 |
| RUNX1 | chr21 | 35180938 | 35185436 | rs2843956; rs55941652; rs56020428; rs56251824; rs13051109; rs13051111; rs3833348; rs7510136; rs743289; rs5843690; rs33915227; rs11402829; rs2843723; rs8128138; rs8131386; rs2843957; rs57537540; rs13048584; rs7281361; rs2843965; rs2843958 |
| SIM2 | chr21 | 36994965 | 36995298 | rs2252821 |
| SIM2 | chr21 | 36999025 | 36999410 | rs58347144; rs737380 |
| DSCAM | chr21 | 41135559 | 41135706 | rs35298822 |
| AIRE | chr21 | 44529935 | 44530388 | rs35110251; rs751032; rs9978641 |
| SUMO3 | chr21 | 45061293 | 45061853 | rs9979741; rs235337; rs7282882 |
| C21orf70 | chr21 | 45202815 | 45202972 | rs61103857; rs9979028; rs881318; rs881317 |
| COL18A1 | chr21 | 45754383 | 45754487 | rs35102708; rs9980939 |
| PRMT2 | chr21 | 46911967 | 46912385 | rs35481242; rs61743122; rs8131044; rs2839379 |
| SIX2 | chr2 | 45081223 | 45082129 | rs62130902 |
| SIX2 | chr2 | 45084851 | 45085711 | rs35417092; rs57340219 |
| SOX14 | chr3 | 138971870 | 138972322 | rs57343003 |
| TLX3 | chr5 | 170674439 | 170676431 | rs11134682; rs35704956; rs2964533; rs35601828 |
| FOXP4 | chr6 | 41623666 | 41624114 | rs12203107; rs1325690 |
| FOXP4 | chr6 | 41636384 | 41636779 | rs56835416 |
| chr7 qroup00267 | chr7 | 12576755 | 12577246 | rs56752985; rs17149965; rs6948573; rs2240572 |
| NPY | chr7 | 24290224 | 24291508 | rs2390965; rs2390966; rs2390967; rs2390968; rs3025123; rs16146; rs16145; rs16144; rs13235842; rs13235935; rs13235938; rs13235940; rs13235944; rs36083509; rs3025122; rs16143; rs16478; rs16142; rs16141; rs16140; rs16139; rs2229966; rs1042552; rs5571; rs5572 |
| SHH | chr7 | 155291537 | 155292091 | rs9333622; rs1233554; rs9333620; rs1233555 |
| GLIS3 | chr9 | 4288283 | 4289645 | rs56728573; rs12340657; rs12350099; rs35338539; rs10974444; rs7852293 |
| PRMT8 | chr12 | 3472714 | 3473190 | rs12172776 |
| TBX3 | chr12 | 113609153 | 113609453 | rs60114979 |
| chr12 proup00801 | chr12 | 118516189 | 118517435 | rs966246; rs17407022; rs970095; rs2711748 |
| PAX9 | chr14 | 36201402 | 36202386 | rs17104893; rs12883298; rs17104895; rs35510737; rs12882923; rs12883049; rs28933970; rs28933972; rs28933971; rs28933373; rs61734510 |
| SIX1 | chr14 | 60178801 | 60179346 | rs761555 |
| ISL2 | chr15 | 74420013 | 74421546 | rs34173230; rs11854453 |
| DLX4 | chr17 | 45397228 | 45397930 | rs62059964; rs57481357; rs56888011; rs17638215; rs59056690; rs34601685; rs17551082 |
| CBX4 | chr17 | 75428613 | 75431793 | rs1285243; rs35035500; rs12949177; rs3764374; rs62075212; rs62075213; rs3764373; rs3764372; rs55973291 |
| EDG6 | chr19 | 3129836 | 3130874 | rs34728133; rs34573539; rs3826936; rs34914134; rs61731111; rs34205484 |
| MGC29506 | chr5 | 138757911 | 138758724 | rs11748963; rs7447765; rs35262202 |
| CENTG1 | chr12 | 56406249 | 56407788 | rs61935742; rs12318065; rs238519; rs238520; rs238521; rs808930; rs2640595; rs2640596; rs2640597; rs2640598; rs34772922 |
| CENTG1 | chr12 | 56416146 | 56418794 | rs11830475; rs34482618; rs2650057; rs2518686; rs12829991 |

**TABLE 3**

| **GENE NAME** | **RELATIVE METHYLATION PLACENTA TO MATERNAL** | **PRC2 TARGET** |
|---|---|---|
| CRYL1 | HYPOMETHYLATION | TRUE |
| IL17D | HYPOMETHYLATION | TRUE |
| GSH1 | HYPERMETHYLATION | TRUE |
| MAB21L1 | HYPERMETHYLA TION | TRUE |
| PCDH17 | HYPERMETHYLATION | TRUE |
| KLHL1 | HYPERMETHYLA TION | TRUE |
| POU4F1 | HYPERMETHYLATION | TRUE |
| SOX21 | HYPERMETHYLA TION | TRUE |
| ZIC2 | HYPERMETHYLA TION | TRUE |
| CIDEA | HYPERMETHYLATION | TRUE |
| KLHL14 | HYPERMETHYLATION | TRUE |
| ONECUT2 | HYPERMETHYLATION | TRUE |
| RAX | HYPERMETHYLA TION | TRUE |
| TNFRSF11A | HYPOMETHYLATION | TRUE |
| OLIG2 | HYPERMETHYLATION | TRUE |
| OLIG2 | HYPOMETHYLATION | TRUE |
| SIM2 | HYPERMETHYLATION | TRUE |
| SIM2 | HYPERMETHYLATION | TRUE |
| SIX2 | HYPERMETHYLATION | TRUE |
| SIX2 | HYPERMETHYLATION | TRUE |
| SOX14 | HYPERMETHYLATION | TRUE |
| TLX3 | HYPERMETHYLATION | TRUE |
| SHH | HYPERMETHYLATION | TRUE |
| OSR2 | HYPERMETHYLATION | TRUE |
| TBX3 | HYPERMETHYLATION | TRUE |
| PAX9 | HYPERMETHYLATION | TRUE |
| SIX1 | HYPERMETHYLATION | TRUE |
| ISL2 | HYPERMETHYLATION | TRUE |
| DLX4 | HYPERMETHYLATION | TRUE |
| CBX4 | HYPERMETHYLATION | TRUE |
| CENTG1 | HYPOMETHYLATION | TRUE |
| CENTG1 | HYPOMETHYLATION | TRUE |

**TABLE 4**

| **SEQ ID NO** | **GENE NAME** | **SEQUENCE** |
|---|---|---|
| **1** | chr13 group-00016 | |
| 2 | CENPJ | |
| | | |
| 3 | ATP8A2 | |
| 4 | GSH1 | |
| 5 | PDX1 | |
| 6 | PDX1 | |
| 7 | MAB21L1 | |
| 8 | RB1 | |
| 9 | PCDH17 | |
| | | |
| 10 | KLHL1 | |
| 11 | POU4F1 | |
| 12 | GPC6 | |
| | | |
| 13 | SOX21 | |
| 14 | ZIC2 | |
| 15 | chr13 group-00385 | |
| | | |
| 16 | chr13 group-00390 | |
| 17 | chr13 group-00391 | |
| 18 | chr13 group-00395 | |
| 19 | chr13 group-00399 | |
| 20 | PROZ | |
| 21 | CIDEA | |
| 22 | chr18 group-00091 | |
| 23 | chr18 group-00094 | |
| 24 | KLHL14 | |
| 25 | ST8SIA3 | CCTCTGTGTTAGTGCCCTCGGGAATTTGGTTGATGGGGTGTTTG |
| 26 | ONECUT2 | |
| | | |
| 27 | RAX | |
| 28 | chr18 group-00277 | |
| 29 | NETO1 | |
| 30 | MBP | |
| 31 | NFATC1 | |
| | | |
| 32 | chr18 group-00430 | |
| 33 | OLIG2 | |
| | | |
| 34 | SIM2 | |
| 35 | SIM2 | |
| 36 | DSCR6 | |
| 37 | DSCAM | |
| 38 | chr21 group-00165 | |
| 39 | PRMT2 | |
| 40 | SIX2 | |
| | | |
| 41 | SIX2 | |
| 42 | SOX14 | |
| 43 | TLX3 | |
| | | |
| 44 | FOXP4 | |
| 45 | FOXP4 | |
| 46 | chr7 group-00267 | |
| 47 | NPY | |
| | | |
| 48 | SHH | |
| 49 | OSR2 | |
| 50 | GLIS3 | |
| 51 | PRMT8 | |
| | | |
| 52 | TBX3 | |
| 53 | chr12 group-00801 | |
| 54 | PAX9 | |
| 55 | SIX1 | |
| | | |
| 56 | ISL2 | |
| 57 | DLX4 | |
| 58 | CBX4 | |
| | | |
| 59 | EDG6 | |
| | | |
| 60 | chr13 group-00005 | |
| 61 | CRYL1 | |
| 62 | IL17D | |
| 63 | IRS2 | |
| | | |
| 64 | chr13 group-00350 | |
| 65 | MCF2L | |
| 66 | F7 | |
| 67 | chr18 group-00039 | |
| 68 | C18orf1 | |
| 69 | CD33L3 | |
| | | |
| 70 | TNFRSF 11A | |
| 71 | ZNF236 | |
| 72 | chr18 group-00342 | |
| 73 | OLIG2 | |
| 74 | RUNX1 | |
| | | |
| 75 | AIRE | |
| 76 | SUMO3 | |
| 77 | C21orf70 | |
| 78 | C21orf123 | |
| 79 | COL18A1 | |
| 80 | PRRT3 | |
| 81 | MGC29506 | |
| 82 | TEAD3 | |
| 83 | chr12 group-00022 | |
| 84 | CENTG1 | |
| 85 | CENTG1 | |
| | | |
| 86 | chr18 group-00304 | |
| 87 | TSHZ1 | |
| 88 | CTDP1 | |
| 89 | KCNG2 | |

Citation of the above patents, patent applications, publications and documents is not an admission that any of the foregoing is pertinent prior art, nor does it constitute any admission as to the contents or date of these publications or documents.

Modifications may be made to the foregoing without departing from the basic aspects of the invention. Although the invention has been described in substantial detail with reference to one or more specific examples, those of ordinary skill in the art will recognize that changes may be made to the examples specifically disclosed in this application.

The invention illustratively described herein suitably may be practiced in the absence of any element(s) not specifically disclosed herein. Thus, for example, in each instance herein any of the terms "comprising," "consisting essentially of," and "consisting of" may be replaced with either of the other two terms. The terms and expressions which have been employed are used as terms of description and not of limitation, and use of such terms and expressions do not exclude any equivalents of the features shown and described or portions thereof, and various modifications are possible within the scope of the invention claimed. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%).

The invention is defined as set forth in the claims that follow.

## Claims

1. A method for determining the amount of fetal nucleic acid in a sample comprising:
a) contacting nucleic acid present in a sample from a pregnant female, which sample comprises differentially methylated fetal nucleic acid and maternal nucleic acid, with an a methyl-CpG binding protein (MBD) or fragment thereof being capable of specifically binding methylated nucleotides; thereby producing bound nucleic acid;
b) isolating the bound nucleic acid of (a) by contact with solutions of increasing salt concentration, thereby enriching the hypermethylated fetal nucleic acid; and
c) determining the amount of fetal nucleic acid by analyzing the amount of fetal nucleic acid of (b) at a plurality of loci selected from loci of SEQ ID NOs:1-59.

2. The method of claim 1, wherein the MBD or fragment thereof is fused to an Fc fragment of an antibody.

3. The method of claim 1 or 2, wherein the process in (c) comprises introducing one or more competitors at known concentrations and determining the absolute amount of fetal nucleic acid in the sample.

4. The method of any one of claims 1 to 3, wherein the plurality of loci comprises a locus of SEQ ID NO: 42.

5. The method of any one of claims 1 to 3, wherein the plurality of loci comprises a locus of SEQ ID NO: 52.

6. The method of any one of claims 1 to 5, wherein the amount of fetal nucleic acid is indicative of a fetal aneuploidy.

7. The method of any one of claims 1 to 6, wherein determining the amount of fetal nucleic acid comprises use of a sequencing method.

8. The method of any one of claims 1 to 6, wherein determining the amount of fetal nucleic acid comprises use of RT-PCR.

9. The method of any one of claims 1 to 6, wherein the process of (c) is an amplification reaction.

## Patentansprüche

1. Verfahren zur Bestimmung der Menge fötaler Nukleinsäure in einer Probe, umfassend:
a) in-Kontakt-bringen von Nukleinsäure, die sich in einer Probe einer schwangeren Frau befindet, wobei die Probe unterschiedlich methylierte fötale Nukleinsäure und maternale Nukleinsäure umfasst, mit einem Methyl-CpG bindenden Protein (MBD) oder einem Fragment davon, das spezifisch methylierte Nukleinsäure binden kann; wodurch gebundene Nukleinsäure hergestellt wird;
b) Isolierung der gebundenen Nukleinsäure aus (a) durch den Kontakt mit Lösungen mit steigender Salzkonzentration, wodurch die hypermethylierte fötale Nukleinsäure angereichert wird; und
c) Bestimmung der Menge an fötaler Nukleinsäure mittels der Analyse der Menge fötaler Nukleinsäure aus (b) an einer Mehrzahl von Loci ausgewählt aus den Loci der SEQ ID Nrn:1-59.

2. Verfahren nach Anspruch 1, wobei das MDB oder das Fragment davon mit einem Fc-Fragment eines Antikörpers verknüpft ist.

3. Verfahren nach Anspruch 1 oder 2, wobei das Verfahren in (c) die Zugabe eines oder mehrerer Kompetitoren in bekannten Konzentrationen und die Bestimmung der absoluten Menge fötaler Nukleinsäure in der Probe umfasst.

4. Verfahren nach einem der Ansprüche 1 bis 3, wobei die Mehrzahl von Loci einen Locus der SEQ ID Nr:42 umfasst.

5. Verfahren nach einem der Ansprüche 1 bis 3, wobei die Mehrzahl von Loci einen Locus der SEQ ID Nr:52 umfasst.

6. Verfahren nach einem der Ansprüche 1 bis 5, wobei die Menge der fötalen Nukleinsäure ein Indiz für fötale Aneuploidie ist.

7. Verfahren nach einem der Ansprüche 1 bis 6, wobei die Bestimmung der Menge der fötalen Nukleinsäure die Verwendung eines Sequenzierungsverfahrens umfasst.

8. Verfahren nach einem der Ansprüche 1 bis 6, wobei die Bestimmung der Menge der fötalen Nukleinsäure die Verwendung von RT-PCR umfasst.

9. Verfahren nach einem der Ansprüche 1 bis 6, wobei das Verfahren nach (c) eine Amplifikationsreaktion ist.

## Revendications

1. Méthode pour déterminer la quantité d'acide nucléique fœtal dans un échantillon, comprenant:
a) la mise en contact d'acide nucléique présent dans un échantillon provenant d'une femelle gravide, lequel échantillon comprend de l'acide nucléique fœtal et de l'acide nucléique maternel méthylés de manière différenciée, avec une protéine se liant au méthyl-CpG (MBD) ou un fragment de celle-ci capable de se lier spécifiquement à des nucléotides méthylés; ce qui produit ainsi un acide nucléique lié;
b) l'isolation de l'acide nucléique lié de (a) par contact avec des solutions de concentration croissante en sel, ce qui enrichit ainsi l'acide nucléique fœtal hyper-méthylé; et
c) la détermination de la quantité d'acide nucléique fœtal par analyse de la quantité d'acide nucléique fœtal de (b) en une pluralité de locus choisis parmi les locus des SEQ ID NO: 1 à 59.

2. Méthode selon la revendication 1, dans laquelle la MBD ou un fragment de celle-ci est fusionnée à un fragment Fc d'un anticorps.

3. Méthode selon la revendication 1 ou 2, dans laquelle le procédé en (c) comprend l'introduction d'un ou plusieurs compétiteurs à des concentrations connues et la détermination de la quantité absolue d'acide nucléique fœtal dans l'échantillon.

4. Méthode selon l'une quelconque des revendications 1 à 3, dans laquelle la pluralité de locus comprend un locus de la SEQ ID NO: 42.

5. Méthode selon l'une quelconque des revendications 1 à 3, dans laquelle la pluralité de locus comprend un locus de la SEQ ID NO: 52.

6. Méthode selon l'une quelconque des revendications 1 à 5, dans laquelle la quantité d'acide nucléique fœtal est indicative d'une aneuploïdie foetale.

7. Méthode selon l'une quelconque des revendications 1 à 6, dans laquelle la détermination de la quantité d'acide nucléique fœtal comprend l'utilisation d'une méthode de séquençage.

8. Méthode selon l'une quelconque des revendications 1 à 6, dans laquelle la détermination de la quantité d'acide nucléique fœtal comprend l'utilisation de RT-PCR.

9. Méthode selon l'une quelconque des revendications 1 à 6, dans laquelle le procédé de (c) est une réaction d'amplification.
